(19) Europäisches Patentamt
European Patent Office
Office européen des brevets

(11) EP 4 663 662 A1

(12) EUROPEAN PATENT APPLICATION
published in accordance with Art. 153(4) EPC

(43) Date of publication:
17.12.2025 Bulletin 2025/51

(51) International Patent Classification (IPC):
C07K 16/30 (2006.01)   C07K 16/28 (2006.01)
A61K 39/395 (2006.01)   A61P 35/00 (2006.01)
A61P 35/02 (2006.01)

(21) Application number: 24752886.2

(22) Date of filing: 07.02.2024

(52) Cooperative Patent Classification (CPC):
A61K 39/395; A61P 35/00; A61P 35/02;
C07K 16/00; C07K 16/28; C07K 16/30; C07K 16/46

(86) International application number:
PCT/CN2024/076521

(87) International publication number:
WO 2024/165031 (15.08.2024 Gazette 2024/33)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 07.02.2023 CN 202310100368

(71) Applicant: Shanghai Qilu Pharmaceutical
Research and
Development Centre Ltd.
Shanghai 201203 (CN)

(72) Inventors:
• YANG, Liuqing
Shanghai 201203 (CN)
• QIAN, Hongliang
Shanghai 201203 (CN)
• LUO, Juan
Shanghai 201203 (CN)

(74) Representative: ABG Intellectual Property Law,
S.L.
Avenida de Burgos, 16D
Edificio Euromor
28036 Madrid (ES)

Remarks:
The complete document including Reference
Table(s) and the Sequence Listing(s) can be
downloaded from the EPO website

(54) TRISPECIFIC ANTIGEN-BINDING MOLECULE AND USE THEREOF

(57) Provided is a configuration of a trispecific antigen-binding molecule. Further provided are a trispecific antigen-binding molecule that is constructed on the basis of the configuration and targets two tumor antigens, i.e. GPRC5D and BCMA, and T cell surface antigen CD3; a specific binding molecule or a fragment thereof that targets GPRC5D; a specific binding molecule or a fragment thereof that targets BCMA; a pharmaceutical composition containing the trispecific antigen-binding molecule or the specific binding molecule or the fragment thereof; and the relevant use thereof in the treatment of tumors.

EP 4 663 662 A1

**Description**

**CROSS REFERECE OF RELATED APPLICATION**

The present application claims priority to Chinese Patent Application No. 2023101003684 filed on February 7, 2023, which is incorporated herein by reference in its entirety.

**TECHNICAL FIELD**

The present disclosure is in the field of immunology and primarily relates to a trispecific antigen-binding molecule. More specifically, the present invention relates to a trispecific antigen-binding molecule that specifically binds to two tumor antigens, GPRC5D and BCMA, and a T cell surface antigen, CD3, as well as a pharmaceutical composition comprising the same, a preparation method, and use thereof.

**BACKGROUND**

Multiple myeloma (MM) is a hematologic malignancy and is the second most common hematologic malignancy after non-Hodgkin's lymphoma. Multiple myeloma can damage bones, immune system, kidneys, and red blood cell count, often leading to extensive bone destruction accompanied by osteolytic lesions, osteopenia, and pathological fractures. The disease is common among the elderly, and the patient population is rapidly increasing with the acceleration of aging and the advancement of diagnostic and therapeutic approaches.

Like treatments for most cancers, the standard treatment for multiple myeloma is chemotherapy, with the commonly used drug melphalan, and the like. The use of this kind of drugs usually leads to bone marrow suppression and shows limited efficacy, with a complete remission rate of only about 5% after treatment. In recent years, with advances in medical field, the development of targeted drugs, such as proteasome inhibitors, immunomodulators, and antibody-based drugs, has revolutionized the treatment for multiple myeloma. Compared to the original treatment regimen, survival rates have greatly improved, and patient prognosis has been significantly improved. However, multiple myeloma remains incurable, and most patients relapsed after multi lines of treatments. The median survival for Chinese patients is 24-36 months, and the five-year survival rate is only about 24%. Therefore, there is an urgent need for new treatment approaches.

B cell maturation antigen (BCMA) is a transmembrane glycoprotein receptor (a non-tyrosine kinase receptor) that belongs to the tumor necrosis factor family. It is primarily expressed on the surface of mature B cells, with a small amount expressed in hematopoietic stem cells or other tissue cells. The ligand molecules for BCMA are BAFF (B cell activating factor) and APRIL (a proliferation-inducing ligand). Under normal physiological state, the BCMA signaling pathway promotes the differentiation of B cells into plasma cells and maintains the homeostasis of long-life-cycle bone marrow plasma cells. BCMA is overexpressed in bone marrow plasma cells of MM patients, and the overexpressed BCMA can stimulate the proliferation of malignant plasma cells and maintain their activity through intracellular AKT, MAPK, and (NF)-κB signaling pathways, thereby promoting disease progression. After blocking the BCMA signaling pathway, the proliferation of malignant plasma cells can be inhibited, and the progression of the disease can be inhibited/alleviated. Thus, BCMA is a good target for clinical treatment of MM. In addition, the BCMA protein on the cell membrane can also be cleaved by γ-secretase *in vivo,* and the cleaved BCMA protein exists in a free state in the serum, referred to as soluble BCMA protein (sBCMA). sBCMA can form complexes with ligands, thereby reducing the concentration of the ligands in the serum, blocking the binding of the ligands to membrane-bound BCMA, and preventing the BCMA signaling pathway.

GPRC5D, a seven-transmembrane protein, is a G protein-coupled receptor C5 family subtype D, and belongs to an orphan receptor. GPRC5D is specifically overexpressed in plasma cells of multiple myeloma, with expression observed only in hair follicles of normal tissues. Both antibodies targeting GPRC5D and CAR-T cell therapy have shown promising effects in preclinical studies, while no hair loss has occurred in animals in the experiments. Moreover, expression of GPRC5D is not limited by the expression of BCMA, and GPRC5D-targeted CAR-T therapy can overcome tumor escape in tumor recurrence models resulting from the loss of BCMA antigens. These results indicate that GPRC5D is an ideal clinical target.

Bispecific antibodies targeting tumor-associated antigen (TAA) and CD3 antigen are capable of rendering T cells in close proximity with tumor cells to form synapses, and achieving specific killing of tumor cells by utilizing T cells. Such antibodies are referred to as T cell-engagers (TCE bispecific antibodies). TCE-based treatment strategies depend on the distribution of TAA on tumor cells to be treated. In addition, studies have also shown that therapeutic forms targeting a single TAA site may lead to recurrence of the disease due to tumor escape mechanisms, thereby limiting the effectiveness of the treatment.

Since the expressions of GPRC5D and BCMA in tumors are not correlated, design of a trispecific antibody targeting both GPRC5D and BCMA can cover both GPRC5D-positive tumor patients and BCMA-positive tumor patients, while preventing recurrence caused by the loss of a single antigen, thereby achieving broader patient coverage and improved therapeutic efficacy.

**SUMMARY OF THE INVENTION**

In order to achieve the above objective, the present disclosure provides a trispecific antigen-binding molecule targeting GPRC5D/BCMA/CD3 and successfully achieves a multi-target combination and avoids the inconvenience of multi-drug combinations via thorough studies on a variety of molecular configurations.

A first aspect of the present disclosure provides a trispecific antigen-binding molecule comprises the following polypeptides:

a first polypeptide comprising: (i) a heavy chain domain of an antigen-binding fragment Fab capable of specifically binding to a first antigen, (ii) a single chain antibody (scFv) domain capable of specifically binding to a second antigen, and (iii) a first Fc domain;

a second polypeptide comprising: a light chain domain of the antigen-binding fragment Fab capable of specifically binding to the first antigen;

a third polypeptide comprising: (i) a heavy chain single-domain antibody (VHH) domain capable of specifically binding to a third antigen, and (ii) a second Fc domain.

The heavy chain domain of the antigen-binding fragment Fab of the first polypeptide forms a first binding site for the first antigen together with the light chain domain of the antigen-binding fragment Fab of the second polypeptide; the single chain antibody (scFv) domain forms a second binding site for the second antigen; the heavy chain single-domain antibody (VHH) domain forms a third binding site for the third antigen; and the first Fc domain and the second Fc domain associate with each other.

Optionally, the trispecific antigen-binding molecule further comprises a fourth polypeptide, which comprises a light chain domain of the antigen-binding fragment Fab capable of specifically binding to the first antigen, in which the light chain domain of the antigen-binding fragment Fab is the same as the light chain domain of the antigen-binding fragment Fab of the second polypeptide, and the third polypeptide further comprises a heavy chain domain of the antigen-binding fragment Fab specifically binds to the first antigen, in which the heavy chain domain of the antigen-binding fragment Fab of the third polypeptide is the same as the heavy chain domain of the antigen-binding fragment Fab of the first polypeptide, with its C-terminus linked to the N-terminus of the VHH domain. The heavy chain domain of the Fab of the third polypeptide form a fourth binding site for the first antigen together with the light chain domain of the Fab of the fourth polypeptide.

In one embodiment, the scFv domain comprises a heavy chain variable region and a light chain variable region; preferably, the heavy chain variable region of the scFv domain is linked to the light chain variable region by a first linker, wherein the C-terminus of the heavy chain variable region is fused to the N-terminus of the first linker, and the C-terminus of the first linker is fused to the N-terminus of the light chain variable region.

More preferably, the first linker comprises an amino acid sequence $(G_4S)_n$, wherein n is any integer from 1 to 10.

In one embodiment, the first Fc domain comprises a first CH2 domain and a first CH3 domain of an immunoglobulin, with the C-terminus of the first CH2 domain fused to the N-terminus of the first CH3 domain; the second Fc domain comprises a second CH2 domain and a second CH3 domain of an immunoglobulin, with the C-terminus of the second CH2 domain fused to the N-terminus of the second CH3 domain.

Preferably, the first CH3 domain comprises a "knob" structure, and the second CH3 domain comprises a "hole" structure; more preferably, the "knob" structure comprises amino acid substitutions S354C and T366W, and the "hole" structure comprises amino acid substitutions Y349C, T366S, L368A, and Y407V.

Preferably, to reduce the ADCC activity of the antibody, the first and/or second Fc domains comprise amino acid substitutions of L234A, L235A, and/or G237A.

Preferably, the second Fc domain of the third polypeptide comprises an amino acid substitution of H435R.

Preferably, the Fc domain is derived from IgG1.

In one embodiment, the N-terminus of the first Fc domain is fused to the C-terminus of the scFv domain, preferably, the N-terminus of the first Fc domain is fused to the C-terminus of the scFv domain via a second linker, more preferably, the second linker comprises an amino acid sequence of EPKSS.

In one embodiment, the heavy chain domain of the antigen-binding fragment Fab comprises a heavy chain variable region and a CH1 domain of an immunoglobulin, with the C-terminus of the heavy chain variable region fused to the N-terminus of the CH1 domain; and the light chain domain of the antigen-binding fragment Fab comprises a light chain variable region and a light chain constant region of an immunoglobulin, with the C-terminus of the light chain variable region fused to the N-terminus of the light chain constant region.

In one embodiment, the heavy chain domain of the antigen-binding fragment Fab of the first polypeptide is linked to the scFv domain via a third linker, wherein the C-terminus of the heavy chain domain of the antigen-binding fragment Fab is fused to the N-terminus of the third linker, and the C-terminus of the third linker is fused to the N-terminus of the scFv domain.

Preferably, the third linker comprises the amino acid sequence $(G_4S)_n$, wherein n is any integer from 1 to 10.

Optionally, the C-terminus of the heavy chain domain of the antigen-binding fragment Fab of the third polypeptide is linked to the N-terminus of the VHH domain via a fifth linker. Preferably, the fifth linker comprises the amino acid sequence $(G_4S)_n$, wherein n is any integer from 1 to 10.

Preferably, the C-terminus of the heavy chain single-domain antibody (VHH) domain is fused to the N-terminus of the second Fc domain, preferably, the C-terminus of the VHH domain is fused to the N-terminus of the second Fc domain via a fourth linker, more preferably, the fourth linker comprises the amino acid sequence EPKSS.

In one embodiment, the first polypeptide comprises the following structure: the heavy chain domain of the Fab - the third linker - the scFv domain -the second linker - the first Fc domain.

Preferably, the first polypeptide comprises the following structure: the heavy chain variable region of the Fab- the Fab CH1 - the third linker - the heavy chain variable region of the scFv - the first linker - the light chain variable region of the scFv - the second linker - the first CH2 - the first CH3.

In one embodiment, the second polypeptide comprises the following structure: the Fab light chain variable region- the light chain constant region.

In one embodiment, the third polypeptide comprises the following structure: VHH domain-the fourth linker- the second Fc domain. Preferably, the third polypeptide comprises the following structure: VHH - the fourth linker - the second CH2 - the second CH3.

Optionally, the fourth polypeptide comprises the following structure: the Fab light chain variable region- the light chain constant region, and the third polypeptide comprises the following structure: the heavy chain domain of the Fab- the fifth linker - the VHH- the fourth linker- the second Fc domain, preferably, the third polypeptide comprises the following structure: the The heavy chain variable region of the Fab - the Fab CH1 - the fifth linker - the VHH- the fourth linker - the second CH2 - the second CH3.

In one embodiment, the second antigen is CD3, preferably CD3$\varepsilon$; preferably, the scFv domain comprises HCDR1 the sequence of which is as set forth in SEQ ID NO: 27, HCDR2 the sequence of which is as set forth in SEQ ID NO: 28, HCDR3 the sequence of which is as set forth in SEQ ID NO: 29, LCDR1 the sequence of which is as set forth in SEQ ID NO: 30, LCDR2 the sequence of which is as set forth in SEQ ID NO: 31, and LCDR3 the sequence of which is as set forth in SEQ ID NO: 32.

More preferably, the scFv domain comprises a heavy chain variable region the sequence of which is as set forth in SEQ ID NO: 25 and a light chain variable region the sequence of which is as set forth in SEQ ID NO: 26.

More preferably, the scFv domain comprises the amino acid sequence as set forth in SEQ ID NO: 13.

In one embodiment, the first Fc domain comprises the amino acid sequence as set forth in SEQ ID NO: 33, and the second Fc domain comprises the amino acid sequence as set forth in SEQ ID NO: 34.

In one embodiment, the first antigen is BCMA; preferably, the antigen-binding fragment Fab comprises HCDR1 the sequence of which is as set forth in SEQ ID NO: 16, HCDR2 the sequence of which is as set forth in SEQ ID NO: 17, and HCDR the sequence of which is as set forth in SEQ ID NO: 18, and/or LCDR1 the sequence of which is as set forth in SEQ ID NO: 19, LCDR2 the sequence of which is as set forth in SEQ ID NO: 20, and LCDR3 the sequence of which is as set forth in SEQ ID NO: 21.

More preferably, the heavy chain domain of the antigen-binding fragment Fab comprises a heavy chain variable region the sequence of which is as set forth in SEQ ID NO: 14, and/or the light chain domain of the antigen-binding fragment Fab comprises a light chain variable region the sequence of which is as set forth in SEQ ID NO: 15.

More preferably, the heavy chain domain of the antigen-binding fragment Fab comprises the amino acid sequence as set forth in SEQ ID NO: 35, and/or the light chain domain of the antigen-binding fragment Fab comprises the amino acid sequence as set forth in SEQ ID NO: 7.

In one embodiment, the third antigen is GPRC5D; preferably, the heavy chain single-domain antibody (VHH) domain, which is capable of specifically binding to the third antigen comprises HCDR1 the sequence of which is as set forth in SEQ ID NO: 22, HCDR2 the sequence of which is as set forth in SEQ ID NO: 23, and HCDR3 the sequence of which is as set forth in SEQ ID NO: 24; more preferably, the VHH domain comprises the sequence as set forth in SEQ ID NO: 10.

In one embodiment, the first polypeptide of the trispecific antigen-binding molecule comprises the amino acid sequence as set forth in SEQ ID NO: 5, the second polypeptide comprises the amino acid sequence as set forth in SEQ ID NO: 7, and the third polypeptide comprises the amino acid sequence as set forth in SEQ ID NO: 6; or optionally, the first polypeptide of the trispecific antigen-binding molecule comprises the sequence as set forth in SEQ ID NO: 5, the second polypeptide and/or the fourth polypeptide comprises the sequence as set forth in SEQ ID NO: 7, and the third polypeptide comprises the sequence as set forth in SEQ ID NO: 8.

There is also provided in the present disclosure a nucleic acid molecule encoding the trispecific antigen-binding molecules described in the present disclosure.

There is also provided in the present disclosure a vector comprising the nucleic acid molecule.

There is also provided in the present disclosure a host cell comprising the nucleic acid molecule or the vector; preferably, the host cell is a prokaryotic cell or an eukaryotic cell; the prokaryotic cell is preferably *Escherichia coli;* the eukaryotic cell is preferably a mammalian cell or yeast; more preferably, the mammal cell is a CHO cell, an Expi293, or a HEK293 cell.

There is also provided in the present disclosure a method for preparing the trispecific antigen-binding molecule, comprising culturing the host cell under suitable conditions.

There is also provided in the present disclosure an antibody-drug conjugate, which is formed by coupling the above trispecific antigen-binding molecule to additional biologically active molecules; preferably, the additional biologically active molecules are small molecule drugs; preferably, the trispecific antigen-binding molecule is linked to the additional biologically active molecules via a linker.

There is also provided in the present disclosure a pharmaceutical composition comprising the trispecific antigen-binding molecule, the nucleic acid molecule, the expression vector, the host cell, and/or the antibody-drug conjugate described above.

In one embodiment, the pharmaceutical composition further comprises a pharmaceutically acceptable carrier.

In one embodiment, the pharmaceutical composition further comprises one or more additional therapeutic agents.

There is also provided in the present disclosure use of the trispecific antigen-binding molecule, the nucleic acid molecule, the vector, the host cell, and/or the antibody-drug conjugate in the preparation of a medicament for the treatment, alleviation, and/or prevention of a tumor. Preferably, the tumor is a GPRC5D-positive and/or BCMA-positive tumor.

There is also provided in the present disclosure a method for inducing the death of a cell expressing GPRC5D and/or BCMA, comprising contacting the cell with the trispecific antigen-binding molecule, the nucleic acid molecule, the vector, the host cell, and/or the pharmaceutical composition, wherein the cell expressing GPRC5D3 and/or BCMA is a tumor cell.

There is also provided in the present disclosure a method for treating a disease associated with the expression of GPRC5D and/or BCMA in a subject, comprising administering to the subject in need thereof the trispecific antigen-binding molecule, the nucleic acid molecule, the vector, the host cell, and/or the pharmaceutical composition. Preferably, the disease is a tumor. Preferably, the subject is relapsed or refractory to treatment with a previous anticancer therapeutic agent. In one embodiment, the method further comprises administering an additional therapeutic agent to the subject.

Preferably, the tumor or tumor cell described in the present disclosure is selected from the group consisting of lymphomas, such as multiple myeloma, and metastatic cancers of the above tumors.

Another aspect of the present disclosure provides an antigen-binding molecule or an antigen-binding fragment thereof that specifically binds to GPRC5D, wherein the antigen-binding molecule or antigen-binding fragment thereof is capable of specifically binding to GPRC5D, but does not specifically bind to GPRC5A, which belongs to the same family as GPRC5D.

Preferably, the antigen-binding molecule or antigen-binding fragment thereof that specifically binds to GPRC5D comprises a heavy chain single-domain antibody (VHH) domain; preferably, the antigen-binding molecule or antigen-binding fragment thereof that specifically binds to GPRC5D comprises HCDR sequences of the heavy chain variable region the amino acid sequence of which is as set forth in SEQ ID NO: 10; preferably, the antigen-binding molecule or antigen-binding fragment thereof that specifically binds to GPRC5D comprises HCDR1 the sequence of which is as set forth in SEQ ID NO: 22, HCDR2 the sequence of which is as set forth in SEQ ID NO: 23, and HCDR3 the sequence of which is as set forth in SEQ ID NO: 24; more preferably, the heavy chain single-domain antibody (VHH) comprises the amino acid sequence as set forth in SEQ ID NO: 10.

Preferably, the antigen-binding molecule or antigen-binding fragment thereof that specifically binds to GPRC5D further comprises the Fc domain of an immunoglobulin selected from IgG1, IgG2, IgG3, or IgG4 isotype, preferably, the antigen-binding molecule or antigen-binding fragment thereof that specifically binds to GPRC5D further comprises the Fc domain of IgG1, more preferably, the Fc domain of IgG1 comprises the amino acid sequence as set forth in SEQ ID NO: 38.

In one embodiment, the antigen-binding molecule or antigen-binding fragment thereof that specifically binds to GPRC5D comprises a heavy chain single-domain antibody (VHH) domain and the Fc domain of IgG1, preferably, the C-terminus of the heavy chain single-domain antibody (VHH) domain is fused to the N-terminus of the Fc domain of IgG1, more preferably, the C-terminus of the heavy chain single-domain antibody (VHH) domain is fused to the N-terminus of the Fc domain of IgG1 via a linker, preferably, the linker comprises the amino acid sequence EPKSS or $(G_4S)_n$, wherein n is any integer from 1 to 10. In a preferred embodiment, the antigen-binding molecule or antigen-binding fragment thereof that specifically binds to GPRC5D comprises, from the N-terminus to the C-terminus, the VHH domain the sequence of which is as set forth in SEQ ID NO: 10, the $G_4S$ linker, and the Fc domain of IgG1 the sequence of which is as set forth in SEQ ID NO: 38.

The antigen-binding molecule or antigen-binding fragment thereof that specifically binds to GPRC5D of the present disclosure may be a monoclonal antibody, a bispecific binding molecule, a multispecific binding molecule, a murine antibody, a humanized antibody, a chimeric antibody, a modified antibody, a fully human antibody, a full-length antibody, a heavy chain antibody, a nanobody, a Fab, an Fv, a scFv, a F(ab')2, a linear antibody, or a heavy chain single-domain antibody.

There is also provided in the present disclosure a conjugate, which is formed by coupling the antigen-binding molecule or antigen-binding fragment thereof that specifically binds to GPRC5D of the present disclosure to a capture label or a detection label; preferably, the detection label comprises a radionuclide, a luminescent substance, a colored substance, or an enzyme.

There is also provided in the present disclosure an antibody-drug conjugate (ADC), which is formed by coupling the antigen-binding molecule or antigen-binding fragment thereof that specifically binds to GPRC5D of the present disclosure to additional biologically active molecules; preferably, the additional biologically active molecules are small molecule drugs; preferably, the antigen-binding molecule or antigen-binding fragment thereof that specifically binds to GPRC5D is linked to the additional biologically active molecules via a linker.

There is also provided in the present disclosure a fusion protein in which one of the fused portions comprises the antigen-binding molecule or antigen-binding fragment thereof that specifically binds to GPRC5D of the present disclosure.

There is also provided in the present disclosure a chimeric antigen receptor (CAR) or a cell comprising the chimeric antigen receptor (for example, CAR-T cell), which comprises the antigen-binding molecule or antigen-binding fragment thereof that specifically binds to GPRC5D of the present disclosure.

There is also provided in the present disclosure a nucleic acid encoding the antigen-binding molecule or antigen-binding fragment thereof that specifically binds to GPRC5D, and a recombinant vector comprising the nucleic acid, as well as a host cell comprising the nucleic acid or the vector described above. Preferably, the host cell is a prokaryotic cell (preferably *E. coli),* or an eukaryotic cell (preferably a mammalian cell or yeast; more preferably, the mammalian cell is a CHO cell or a HEK293 cell).

There is also provided in the present disclosure a method for preparing the antigen-binding molecule or antigen-binding fragment thereof that specifically binds to GPRC5D, comprising culturing the host cell under suitable conditions and purifying the expression product obtained from the cell.

There is also provided in the present disclosure use of the antigen-binding molecule or antigen-binding fragment thereof that specifically binds to GPRC5D in the preparation of a medicament for the treatment or amelioration of tumors.

In one embodiment, the medicament targets tumor cells with abnormal expression of GPRC5D.

In one embodiment, the tumor is selected from the group consisting of lymphomas, such as multiple myeloma, and metastatic cancers of the above tumors.

There is also provided in the present disclosure a method for treating a disease associated with the expression of GPRC5D in a subject, comprising administering to the subject in need thereof the antigen-binding molecule or antigen-binding fragment thereof that specifically binds to GPRC5D.

Preferably, the disease is a tumor; preferably, the tumor disease is selected from the group consisting of lymphomas, such as multiple myeloma, and metastatic cancers of the above tumors.

More preferably, the method further comprises administering an additional therapeutic agent to the subject.

There is also provided in the present disclosure use of the antigen-binding molecule or antigen-binding fragment thereof that specifically binds to GPRC5D in the preparation of a detection reagent or a diagnostic reagent.

In one embodiment, the detection reagent is used for detecting the expression of GPRC5D; the diagnostic reagent is used for diagnosing tumors; preferably, the tumor is selected from the group consisting of lymphomas, such as multiple myeloma, and metastatic cancers of the above tumors.

There is also provided in the present disclosure a method for detecting the expression of GPRC5D in a sample, comprising:

(1) contacting the sample with the antigen-binding molecule or antigen-binding fragment thereof that specifically binds to GPRC5Dof the present disclosure; and
(2) detecting formation of a complex between the antigen-binding molecule or antigen-binding fragment thereof that specifically binds to GPRC5D and GPRC5D; optionally, the antigen-binding molecule or antigen-binding fragment thereof that specifically binds to GPRC5D is detectably labeled.

There is also provided in the present disclosure a method of monitoring GPRC5D-expressing cancer in a subject, comprising exposing a sample obtained from or derived from the subject to one or more of the antigen-binding molecules or antigen-binding fragments thereof specifically binding to GPRC5D described herein; determining the amount of GPRC5D present in the sample bound by the antibody or antigen-binding fragment thereof; comparing the amount of GPRC5D present in the sample with the amount of GPRC5D in a known standard or reference sample, or a similar sample previously obtained from the subject; and determining whether the subject's GPRC5D level is indicative of cancer progression, regression, or stable disease based on the difference in the amount of GPRC5D in the compared sample.

The sample obtained from or derived from the subject is a biological sample, such as urine, blood, serum, plasma, saliva, ascites, a circulating cell, a circulating tumor cell, a non-tissue-associated cell, tissue, a surgically resected tumor tissue, a biopsy, a fine-needle aspiration sample, or a histological preparation.

There is also provided in the present disclosure a pharmaceutical composition comprising an effective amount of the antigen-binding molecule or antigen-binding fragment thereof that specifically binds to GPRC5D of the present disclosure, or comprising an effective amount of the antibody-drug conjugate, the fusion protein, or the CAR-T cells of the present disclosure, or comprising an effective amount of the nucleic acid of the present disclosure, or comprising an effective amount of the recombinant vector of the present disclosure, or comprising an effective amount of the host cells of the present disclosure.

In one embodiment, the pharmaceutical composition further comprises a pharmaceutically acceptable carrier.

Preferably, the pharmaceutical composition further comprises one or more additional therapeutic agents.

Another aspect of the present disclosure provides an antigen-binding molecule or an antigen-binding fragment thereof that specifically binds to BCMA, wherein the antigen-binding molecule or antigen-binding fragment thereof comprises HCDR sequences of a heavy chain variable region the sequence of which is as set forth in SEQ ID NO: 14, and/or LCDR sequences of a light chain variable region the sequence of which is as set forth in SEQ ID NO: 15; preferably, the antigen-binding molecule or antigen-binding fragment thereof comprises HCDR1 the sequence of which is as set forth in SEQ ID NO: 16, HCDR2 the sequence of which is as set forth in SEQ ID NO: 17, and HCDR3 the sequence of which is as set forth in SEQ ID NO: 18, and/or an LCHDR1 the sequence of which is as set forth in SEQ ID NO: 19, LCDR2 the sequence of which is as set forth in SEQ ID NO: 20, and LCDR3 the sequence of which is as set forth in SEQ ID NO: 21.

Preferably, the antigen-binding molecule or antigen-binding fragment thereof that specifically binds to BCMA comprises a heavy chain variable region the sequence of which is as set forth in SEQ ID NO: 14, and/or the antigen-binding molecule or antigen-binding fragment thereof that specifically binds to BCMA comprises a light chain variable region the sequence of which is as set forth in SEQ ID NO: 15.

More preferably, the antigen-binding molecule or antigen-binding fragment thereof that specifically binds to BCMA comprises the heavy chain amino acid sequence as set forth in SEQ ID NO: 9, and/or the antigen-binding molecule or antigen-binding fragment thereof that specifically binds to BCMA comprises the light chain amino acid sequence as set forth in SEQ ID NO: 7.

The antigen-binding molecule or antigen-binding fragment thereof that specifically binds to BCMA of the present disclosure further comprises a heavy chain constant region and/or a light chain constant region; preferably, the heavy chain constant region comprises a Fc; more preferably, the Fc is derived from murine or human; even more preferably, the sequence of the Fc is either naturally occurring or modified.

The antigen-binding molecule or antigen-binding fragment thereof that specifically binds to BCMA of the present disclosure may be a monoclonal antibody, a bispecific binding molecule, a multispecific binding molecule, a murine antibody, a humanized antibody, a chimeric antibody, a modified antibody, a fully human antibody, a full-length antibody, a heavy chain antibody, a nanobody, a Fab, an Fv, a scFv, a F(ab')2, a linear antibody, or a heavy chain single-domain antibody.

The antigen-binding molecule or antigen-binding fragment thereof that specifically binds to BCMA of the present disclosure may be a full-length antibody.

The antigen-binding molecule or antigen-binding fragment thereof that specifically binds to BCMA of the present disclosure may be of the IgG1, IgG2, IgG3, or IgG4 isotype.

There is also provided in the present disclosure a conjugate, which is formed by coupling the antigen-binding molecule or antigen-binding fragment thereof that specifically binds to BCMA of the present disclosure to a capture label or a detection label; preferably, the detection label comprises a radionuclide, a luminescent substance, a colored substance, or an enzyme.

There is also provided in the present disclosure an antibody-drug conjugate (ADC), which is formed by coupling the antigen-binding molecule or antigen-binding fragment thereof that specifically binds to BCMA of the present disclosure to additional biologically active molecules; preferably, the additional biologically active molecules are small molecule drugs; preferably, the antigen-binding molecule or antigen-binding fragment thereof that specifically binds to BCMA is linked to the additional biologically active molecules via a linker.

There is also provided in the present disclosure a fusion protein in which one of the fused portions comprises the antigen-binding molecule or antigen-binding fragment thereof that specifically binds to BCMA of the present disclosure.

There are also provided in the present disclosure a chimeric antigen receptor (CAR) and a cell comprising the chimeric antigen receptor (for example, CAR-T cell), which comprise the antigen-binding molecule or antigen-binding fragment thereof that specifically binds to BCMA of the present disclosure.

There are also provided in the present disclosure a nucleic acid encoding the antigen-binding molecules or antigen-binding fragments thereof that specifically binds to BCMA, and the recombinant vector comprising the nucleic acid, and a host cell comprising the nucleic acid or the vector described above. Preferably, the host cell is a prokaryotic cell (preferably *E. coli*), or an eukaryotic cell (preferably a mammalian cell or yeast; more preferably, the mammalian cell is a CHO cell or a HEK293 cell).

There is also provided in the present disclosure a method for preparing the antigen-binding molecule or antigen-binding fragment thereof that specifically binds to BCMA of the present disclosure, comprising culturing the above host cells under suitable conditions and purifying the expression product obtained from the cells.

There is also provided in the present disclosure use of the antigen-binding molecule or antigen-binding fragment thereof that specifically binds to BCMA in the preparation of a medicament for the treatment or amelioration of tumors.

In one embodiment, the drug targets tumor cells with abnormal expression of BCMA.

In one embodiment, the tumor is selected from the group consisting of lymphomas, such as multiple myeloma, and metastatic cancers of the above tumors.

There is also provided in the present disclosure a method for treating a disease associated with the expression of BCMA in a subject, comprising administering to the subject in need thereof the antigen-binding molecule or antigen-binding fragment thereof that specifically binds to BCMA.

Preferably, the disease is a tumor; preferably, the tumor disease is selected from the group consisting of lymphomas, such as multiple myeloma, and metastatic cancers of the above tumors.

More preferably, the method further comprises administering an additional therapeutic agent to the subject.

There is also provided in the present disclosure use of the antigen-binding molecule or antigen-binding fragment thereof that specifically binds to BCMA in the preparation of a detection reagent or diagnostic reagent.

In one embodiment, the detection reagent is used for detecting the expression of BCMA; the diagnostic reagent is used for diagnosing tumors; preferably, the tumor is selected from the group consisting of lymphomas, such as multiple myeloma, and metastatic cancers of the above tumors.

There is also provided in the present disclosure a method for detecting the expression of BCMA in a sample, comprising:

(1) contacting the sample with the antigen-binding molecule or antigen-binding fragment thereof that specifically binds to BCMA of the present disclosure; and

(2) detecting formation of a complex between the antigen-binding molecule or antigen-binding fragment thereof that specifically binds to BCMA and BCMA; optionally, the antigen-binding molecule or antigen-binding fragment thereof that specifically binds to BCMA is detectably labeled.

There is also provided in the present disclosure a method of monitoring BCMA-expressing cancer in a subject, comprising exposing a sample obtained from or derived from the subject to one or more of the antigen-binding molecules or antigen-binding fragments thereof specifically binding to BCMA described herein; determining the amount of BCMA present in the sample bound by the antigen-binding molecules or antigen-binding fragments thereof; comparing the amount of BCMA present in the sample with the amount of BCMA in a known standard or reference sample, or a similar sample previously obtained from the subject; and determining whether the subject's BCMA level is indicative of cancer progression, regression, or stable disease based on the difference in the amount of BCMA in the compared sample.

The sample obtained from or derived from the subject is a biological sample, such as urine, blood, serum, plasma, saliva, ascites, a circulating cell, a circulating tumor cell, a non-tissue-associated cell, tissue, a surgically resected tumor tissue, a biopsy, a fine-needle aspiration sample, or a histological preparation.

There is also provided in the present disclosure a pharmaceutical composition comprising an effective amount of the antigen-binding molecule or antigen-binding fragment thereof that specifically binds to BCMA of the present disclosure, or comprising an effective amount of the antibody-drug conjugate, the fusion protein, or CAR-T cells of the present disclosure, or comprising an effective amount of the nucleic acid of the present disclosure, or comprising an effective amount of the recombinant vector of the present disclosure, or comprising an effective amount of host cells of the present disclosure.

In one embodiment, the pharmaceutical composition further comprises a pharmaceutically acceptable carrier.

Preferably, the pharmaceutical composition further comprises one or more additional therapeutic agents.

The amino acid positions in the substitutions referred to in the present disclosure are preferably represented by the EU numbering system.

## BRIEF DESCRIPTION OF THE DRAWINGS

The drawings further illustrate the novel features disclosed in the specification. The features and advantages disclosed in the specification will be better understood with reference to the accompanying drawings, which, however, are to be understood as merely illustrating specific embodiments of the principles disclosed herein and are not intended to limit the scope of the appended claims.

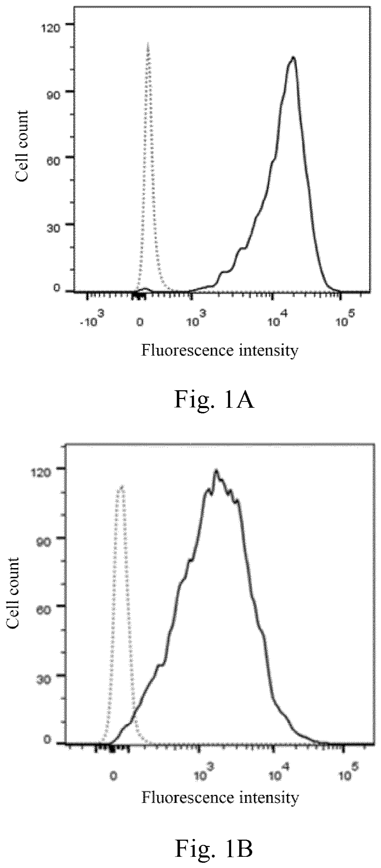

FIG. 1A shows the expression of human GPRC5D on CHOK1 cells verified by anti-GPRC5D-positive control antibodies detected using a flow cytometer; the dashed line in the figure shows the expression of human GPRC5D on CHOK1 parent cells that were not transfected with any plasmids, while the solid line in the figure shows the overexpression of the human GPRC5D protein on CHOK1 parent cells transfected with the human GPRC5D plasmid. FIG. 1B shows the expression of cynomolgus monkey GPRC5D on CHOK1 cells verified by anti-GPRC5D-positive control antibodies detected using a flow cytometer; the dashed line in the figure shows the expression of cynomolgus monkey GPRC5D on the CHOK1 parent cells that were not transfected with any plasmids, while the solid line in the figure shows the overexpression of the cynomolgus monkey GPRC5D protein on CHOK1 parent cells transfected with the cynomolgus monkey GPRC5D plasmid.

FIG. 1C shows the expression of human GPRC5A on CHOK1 cells verified by anti-GPRC5A positive control antibodies detected using a flow cytometer; the dashed line in the figure shows the expression of human GPRC5A on CHOK1 parent cells that were not transfected with any plasmids, while the solid line in the figure shows the overexpression of the human GPRC5A protein on CHOK1 parent cells transfected with the human GPRC5A plasmid.

FIG. 2A shows the expression of human BCMA on CHOK1 cells verified by anti-BCMA-positive control antibodies detected using a flow cytometer; the dashed line in the figure shows the expression of human BCMA on CHOK1 parent cells that were not transfected with any plasmids, while the solid line in the figure shows the overexpression of the human BCMA protein on CHOK1 parent cells transfected with the human BCMA plasmid.

FIG. 2B shows the expression of cynomolgus monkey BCMA on CHOK1 cells verified by anti-BCMA-positive control antibodies detected using a flow cytometer; the dashed line in the figure shows the expression of the cynomolgus monkey BCMA on the CHOK1 parent cells that were not transfected with any plasmids, while the solid line in the figure shows the overexpression of the cynomolgus monkey BCMA protein on CHOK1 parent cells transfected with the cynomolgus monkey BCMA plasmid.

Figure 3 shows the structures of two trispecific antibodies constructed in the Examples, namely the trispecific antibody 1 and the trispecific antibody 2.

FIG. 4A shows the binding of the trispecific antibody 1, the trispecific antibody 2, and Teclistamab to NCI-H929 tumor cells expressing the dual targets BCMA and GPRC5D.

FIG. 4B shows the binding of the trispecific antibody 1, the trispecific antibody 2, and Teclistamab to RPMI8226 tumor cells expressing the dual targets BCMA and GPRC5D.

FIG. 4C shows the binding of the trispecific antibody 1, the trispecific antibody 2, and Teclistamab to the stably transfected cell line expressing human GPRC5D (the human GPRC5D CHOK1 cells).

FIG. 4D shows the binding of the trispecific antibody 1, the trispecific antibody 2, and Teclistamab to the stably transfected cell line expressing human BCMA (the human BCMA CHOK1 cells).

FIG. 4E shows the binding of the trispecific antibody 1, the BGCB491, and the TS-F2-5 to NCI-H929 tumor cells expressing the dual targets BCMA and GPRC5D.

FIG. 4F shows the binding of the trispecific antibody 1, the BGCB491, and the TS-F2-5 to RPMI8226 tumor cells expressing the dual targets BCMA and GPRC5D.

FIG. 4G shows the binding of the trispecific antibody 1, the BGCB491, and the TS-F2-5 to the stably transfected cell line expressing human GPRC5D (the human GPRC5D CHOK1 cells).

FIG. 4H shows the binding of the trispecific antibody 1, the BGCB491, and the TS-F2-5 to the stably transfected cell line expressing human BCMA (the human BCMA CHOK1 cells).

FIG. 5A shows the binding of the trispecific antibody 1, the trispecific antibody 2, and Teclistamab to human CD3-expressing cells (Jurkat cells).

FIG. 5B shows the binding of the trispecific antibody 1, the trispecific antibody 2, and Teclistamab to human PBMC cells.

FIG. 5C shows the binding of the trispecific antibody 1, the BGCB491, and the TS-F2-5 to hCD3-expressing cells (Jurkat cells).

FIG. 6A shows the binding of the trispecific antibody 1, the trispecific antibody 2, and Teclistamab to the stably transfected cell line expressing cynomolgus monkey GPRC5D (the cynomolgus monkey GPRC5D CHOK1 cells).

FIG. 6B shows the binding of the trispecific antibody 1, the trispecific antibody 2, and Teclistamab to the stably transfected cell line expressing cynomolgus monkey BCMA (the cynomolgus monkey BCMA CHOK1 cells).

FIG. 7 shows the binding of the trispecific antibody 1, the trispecific antibody 2, and Teclistamab to cyno PBMC cells.

FIG. 8A shows the T-cell-dependent cellular cytotoxicity (TDCC) experiment, in which the target cells are NCI-H929.

FIG. 8B shows the T-cell-dependent cellular cytotoxicity (TDCC) experiment, in which the target cells are RPMI8226.

FIG. 8C shows the T-cell-dependent cellular cytotoxicity (TDCC) experiment, in which the target cells are human BCMA CHOK1.

FIG. 8D shows the T-cell-dependent cellular cytotoxicity (TDCC) experiment, in which the target cells are human GPRC5D CHOK1.

FIG. 8E shows the T-cell-dependent cellular cytotoxicity (TDCC) experiment, in which the target cells are human BCMA CHOK1 and human GPRC5D CHOK1 cells.

FIG. 9 shows the T-cell-dependent cellular cytotoxicity (TDCC) experiment in the presence of soluble BCMA of 2500 ng/ml (the dashed line represents the condition of 2500 ng/ml of soluble BCMA).

FIG. 10A shows the IL6 release under co-incubation of the trispecific antibody 1, the trispecific antibody 2, and Teclistamab with PBMCs and NCI-H929 tumor cells (PBMC donor No. P122080606F).

FIG. 10B shows the IL6 release under co-incubation of the trispecific antibody 1, the trispecific antibody 2, and Teclistamab with PBMCs and NCI-H929 tumor cells (PBMC donor No. XC11210).

FIG. 11 shows the results of pharmacodynamic experiments of the trispecific antibody 1, the trispecific antibody 2, Teclistamab, Teclistamab, and Talquetamb in a mouse model of human immune cell reconstitution, showing tumor volume changes (tumor cells are NCI-H929).

FIG. 12 shows the results of pharmacodynamic experiments of the trispecific antibody 1, the BGCB491, and the TS-F2-5 in a mouse model of human immune cell reconstitution, showing tumor volume changes (tumor cells are NCI-H929).

FIG. 13 shows the results of pharmacodynamic experiments of the trispecific antibody 1, the BGCB491, and the TS-F2-5 in a mouse model of human immune cell reconstitution, showing tumor weight changes (tumor cells are NCI-H929).

FIG. 14A shows the binding of llama-derived anti-GPRC5D chimeric antibodies of the present disclosure to GPRC5D-expressing cells (the human GPRC5D-CHOK1 cell line).

FIG. 14B shows the binding of llama-derived anti-GPRC5D chimeric antibodies of the present disclosure to GPRC5D-expressing cells (the cynomolgus monkey GPRC5D-CHOK1 cell line).

FIG. 15A shows the binding of llama-derived anti-GPRC5D humanized antibodies of the present disclosure to GPRC5D-expressing cells (NCI-H929 tumor cells).

FIG. 15B shows the binding of llama-derived anti-GPRC5D humanized antibodies of the present disclosure to GPRC5D-expressing cells (the cynomolgus monkey GPRC5D-CHOK1 cell line).

FIG. 16 shows no non-specific binding of the llama-derived anti-GPRC5D humanized antibodies of the present disclosure to GPRC5A, a member of the same family.

FIG. 17A shows the binding of mouse hybridoma-derived anti-BCMA chimeric antibodies of the present disclosure to human BCMA-expressing cells (the human BCMA-CHOK1 cell line).

FIG. 17B shows the binding of mouse hybridoma-derived anti-BCMA chimeric antibodies of the present disclosure to cynomolgus monkey BCMA-expressing cells (the cynomolgus monkey BCMA-CHOK1 cell line).

FIG. 18A shows the binding of mouse hybridoma-derived anti-BCMA humanized antibodies of the present disclosure to human BCMA-expressing cells (the human BCMA-CHOK1 cell line).

FIG. 18B shows the binding of mouse hybridoma-derived anti-BCMA humanized antibodies of the present disclosure to cynomolgus monkey BCMA-expressing cells (the cynomolgus monkey BCMA-CHOK1 cell line).

FIG. 19A shows the binding of mouse hybridoma-derived anti-BCMA humanized antibody variants 19CH-16H2L2-NA and 19CH-16H2L2-QT of the present disclosure to human BCMA-expressing cells (the human BCMA-CHOK1 cell line).

FIG. 19B shows the binding of mouse hybridoma-derived anti-BCMA humanized antibody variants 19CH-16H2L2-NA and 19CH-16H2L2-QT of the present disclosure to cynomolgus monkey BCMA-expressing cells (the cynomolgus monkey BCMA-CHOK1 cell line).

## DETAILED DESCRIPTION OF THE INVENTION

### TERMS

All publications, patents, and patent applications mentioned in the present specification are incorporated herein by reference to the same extent as each individual publication, patent, or patent application is specifically and individually indicated as being incorporated herein by reference.

Before the disclosure is described in detail below, it should be understood that the disclosure is not limited to the specific methodologies, protocols, and reagents described herein, as these may vary. It should also be understood that the terminology used herein is for the purpose of describing particular embodiments only and is not intended to limit the scope of the present disclosure. Unless otherwise defined, all technical and scientific terms used herein have the same meaning as commonly understood by one of ordinary skill in the art to which this disclosure pertains.

Certain embodiments disclosed herein include numerical ranges, and certain aspects of the disclosure may be described in terms of ranges. Unless otherwise indicated, it should be understood that the numerical ranges or the ways of describing in terms of ranges are for the purposes of brevity and convenience only, and are not to be considered as a strict limitation to the scope of the present disclosure. Accordingly, the ways of describing in terms of ranges should be considered to specifically disclose all possible sub-ranges and all possible specific numerical points within that range, as if such sub-ranges and numerical points had been expressly written herein. The above principles apply equally regardless of the breadth of the numerical values. When a range description is employed, the range includes the endpoints of the range.

When referring to measurable values such as amounts, temporary durations, and the like, the term "about" refers to a variation of +20%, or in some cases +10%, or in some cases +5%, or in some cases +1%, or in some cases $\pm 0.1\%$, from a specified value.

The term "antibody" herein may include an intact antibody (for example, a full-length monoclonal antibody) and any antigen-binding fragments thereof (i.e., antigen-binding portion) or single chain thereof, and may also include a product with antigen-specific binding capacity formed by modifying the intact antibody or antigen-binding fragment or single chain thereof (for example, ligation to other peptide segments, functional unit rearrangement, and the like).

In one embodiment, an antibody typically refers to a Y-type tetrameric protein comprising two heavy (H) polypeptide chains and two light (L) polypeptide chains held together by covalent disulfide bonds and non-covalent interactions. Native IgG antibodies have this structure. Each light chain consists of a variable domain (VL) and a constant domain (CL). Each heavy chain contains a variable domain (VH) and a constant region.

Five major classes of antibodies are known in the art: IgA, IgD, IgE, IgG and IgM, with the corresponding heavy chain constant domains being referred to as $\alpha$, $\delta$, $\varepsilon$, $\gamma$, and $\mu$, respectively. IgG and IgA can be further divided into different subclasses, for example, IgG can be subdivided into IgG1, IgG2, IgG3, IgG4, while IgA can be subdivided into IgA1 and IgA2. The light chains of antibodies from any vertebrate species may be assigned to one of two distinct types, named $\kappa$ and $\lambda$, based on the amino acid sequences of constant domains.

In the case of IgG, IgA and IgD antibodies, the constant region comprises three domains referred to as CH1, CH2 and CH3 (IgM and IgE contain a fourth domain, CH4). In the IgG, IgA and IgD classes, the CH1 and CH2 domains are separated by flexible hinge regions, which are proline and cysteine-rich segments with variable lengths. Each class of antibodies further comprises interchain and intrachain disulfide bonds formed from paired cysteine residues.

The term "variable region" or "variable domain" exhibits a significant change in the amino acid composition from one antibody to another and is primarily responsible for antigen recognition and binding. The variable region of each light/heavy chain pair forms an antibody binding site, making the intact IgG antibody has two binding sites (i.e., it is bivalent). The variable region (VH) of the heavy chain and the variable region (VL) of the light chain each comprise three regions with extreme variability, referred to as hyper-variable regions (HVRs), or more commonly as complementarity determining regions (CDRs), and VH and VL each contains four framework regions FRs, designated FR1, FR2, FR3, and FR4, respectively. Therefore, the CDR and FR sequences are typically present in the following sequences of the heavy chain variable domain (or light chain variable domain): FR1 - HCDR1 (LCDR1) - FR2 - HCDR2 (LCDR2) - FR3 - HCDR3 (LCDR3) - FR4.

The term "antibody fragment" includes at least a portion of an intact antibody. As used herein, a "fragment" of an antibody molecule includes the "antigen-binding fragment" of the antibody, and the term "antigen-binding fragment" refers to a polypeptide fragment in an immunoglobulin or antibody that specifically binds to or reacts with a selected antigen or immunogenicity-determining portion thereof, or a fusion protein product further derived from that fragment, such as a single chain antibody, the extracellular binding region in a chimeric antigen receptor, and the like. Exemplary antibody fragments or antigen-binding fragments thereof include, but are not limited to, variable light chain fragments, variable heavy chain fragments, Fab fragments, F(ab')$_2$ fragments, Fd fragments, Fv fragments, single-domain antibodies, linear antibodies, single chain antibodies (scFv), and bispecific or multispecific antibodies formed from the antibody fragments, and the like.

The term "Fab" or "Fab fragment" refers to a monovalent antibody fragment consisting of the VH and CH1 domains of the heavy chain and the VL and CL domains of the light chain. The term "F(ab')$_2$" or "F(ab')$_2$ fragment" comprises two Fab fragments and a hinge region, which is a bivalent antibody fragment.

The term "single chain antibody" or "scFv" refers to a fusion protein comprising at least one antibody fragment comprising the light chain variable region and at least one antibody fragment comprising the heavy chain variable region, where the light and heavy chain variable regions are contiguous (for example, via a synthetic linker, such as a short flexible polypeptide linker), and can be expressed as a single chain polypeptide, and wherein the scFv retains the specificity of the intact antibody from which it is derived. Unless specified, the scFv may have the VL and VH in any order (for example, relative to the N-terminal and C-terminal of the polypeptide), and the scFv may comprise the VL-linker-VH or may comprise the VH-linker-VL.

"VHH domain", also known as heavy chain single-domain antibody, VHH, VHH antibody fragment, VHH antibody, or nanobody, is a variable domain of an antigen-binding immunoglobulin known as "heavy chain antibody" (i.e., "antibody that lacks light chain"). The term "VHH domain" is used to distinguish the variable domain from the heavy chain variable domain (referred to in the present disclosure as the "VH domain") and the light chain variable domain (referred to in the present disclosure as the "VL domain") present in the antibody of the conventional tetrapeptide chain structure. The VHH domain specifically binds to an epitope without the need for other antigen-binding domains (unlike the VH or VL domain in the antibody of the conventional tetrapeptide chain structure, where the epitope is recognized by both the VL and VH domains). The VHH domain is a small, stable, and efficient antigen recognition unit formed from a single immunoglobulin domain. The terms "heavy chain single-domain antibody", "VHH domain", "VHH", "VHH domain", "VHH antibody fragment", "VHH antibody" and "heavy chain antibody variable region" can be used interchangeably. "VHH domain" includes, but is not limited to, natural antibodies produced by camelids, those obtained by humanization of antibodies produced by camelids, or antibodies obtained through phage display technology screening.

The term "amino acid modification" (or "modified amino acid") includes amino acid substitutions, insertions, and/or deletions in a polypeptide sequence. The "amino acid substitution" or "substitution" or "replacement" herein means replacing an amino acid at a particular position in a parent polypeptide sequence with another amino acid. For example, substitution S32A refers to the substitution of serine at position 32 with alanine.

The multi-specific antigen-binding molecules of the present disclosure may also include substitutions or modifications to constant regions (for example, Fc), including but not limited to amino acid residue substitutions, mutations, and/or modifications, which result in compounds having preferred features, including but not limited to altered pharmacokinetics, increased serum half-life, increased binding affinity, decreased immunogenicity, increased yield, altered Fc ligand binding to the Fc receptor (FcR), enhanced or attenuated ADCC or CDC, altered glycosylation and/or disulfide bonds, and modified binding specificity. In certain aspects, antibody variants comprise a Fc region having one or more amino acid replacements (for example, replacements at positions 234 and 235 of the Fc region) that impair Fc$\gamma$R binding. In one aspect, the replacements are L234A and L235A.

The term "Fc" is used to define the C-terminal region of an immunoglobulin heavy chain, comprising at least a portion of the constant region. The term includes the Fc region having a native sequence and the variant Fc region. Although the boundaries of the Fc region of the IgG heavy chain may vary slightly, the human IgG heavy chain Fc region is generally defined as extending from Cys226 or Pro230 to the carboxyl terminus of the heavy chain, for example, the IgG Fc domain contains IgG CH2 and IgG CH3 constant domains. Unless otherwise specified herein, the amino acid residues in the Fc region or the constant region is numbered in accordance with the EU numbering system, also referred to as the EU index.

The term "Knob-into-Hole" refers to a modification for promoting the association of two Fc polypeptide chains, comprising a "knob" modification in one of the two Fc polypeptide chains and a "hole" modification in the other one of the two Fc polypeptide chains. In general, the method involves the introduction of a bump ("knob") at the interface of the first polypeptide chain and of a corresponding cavity ("hole") at the interface of the second polypeptide chain so that the bump can be placed in the cavity to promote heterodimer formation and hinder homodimer formation. The bump is constructed by replacing amino acids having small side chains from the interface of the first polypeptide chain with amino acids having larger side chains, such as tyrosine or tryptophan. Complementary cavity having the same or similar size as the bump is created at the interface of the second polypeptide chain by replacing amino acids having large side chains with amino acids having smaller side chains, such as alanine or threonine.

Therefore, in a particular embodiment, in the CH3 domain of the first polypeptide chain of the Fc domain of the trispecific antigen-binding molecule of the present disclosure, one amino acid residue is replaced with an amino acid residue having a larger side chain, thereby creating a bump in the CH3 domain of the first polypeptide chain, which can be placed in a cavity in the CH3 domain of the second polypeptide chain, and in the CH3 domain of the second polypeptide chain of the Fc domain, one amino acid residue is replaced with an amino acid residue having a smaller side chain, thereby creating a cavity in the CH3 domain of the second polypeptide chain, in which the bump in the CH3 domain of the first polypeptide chain can be placed. Preferably, the amino acid residues having a larger side chain are selected from the group consisting of arginine (R), phenylalanine (F), tyrosine (Y), and tryptophan (W). Preferably, the amino acid residues having a smaller side chain are selected from the group consisting of alanine (A), serine (S), threonine (T), and valine (V).

The term "linker" refers to any tools used to link two different functional units, such as antigen-binding fragments. Types of linkers include, but are not limited to, chemical linkers and polypeptide linkers. The sequence of the polypeptide linker is not limited. The polypeptide linkers are preferably non-immunogenic and flexible, such as those sequences comprising serine and glycine. The linker may be long or short depending on the particular construct.

According to the present disclosure, the linker linking the different functional units preferably comprises a flexible peptide linker, such as a glycine-serine peptide linker. In one embodiment, the linker comprises the amino acid sequence $(G_4S)_n$ or $(G_4S)_nA$, wherein n is any integer from 1 to 10, preferably comprises the amino acid sequence $(G_4S)_3$ or $(G_4S)_3A$. The linker linking the VH and VL domains to form the scFv domain of VH-VL or VL-VH preferably comprises a flexible peptide linker, such as a glycine-serine peptide linker. In one embodiment, the linker comprises the amino acid sequence $(G_4S)_n$ or $(G_4S)_nA$, wherein n is any integer from 1 to 10, preferably comprises the amino acid sequence $(G_4S)_3$ or $(G_4S)$.

"Antibody" herein may be used in the broadest sense and may include, for example, a polyclonal antibody, a monoclonal antibody, a chimeric antibody, a humanized antibody and a primatogenic antibody, a CDR-grafted antibody, a human antibody (including a recombinantly produced human antibody), a recombinantly produced antibody, an intracellular antibody, a multispecific antibody, a bispecific antibody, a monovalent antibody, a multivalent antibody, an anti-individual genotype antibody, a synthetic antibody (including muteins and variants thereof), and the like.

The term "monoclonal antibody" (also referred to as "mAb") refers to an antibody produced by a single cell clone that is substantially homogeneous, targeting only a particular antigen epitope. Monoclonal antibodies can be prepared using a variety of techniques known in the art, including hybridoma techniques, recombinant techniques, phage display techniques, transgenic animals, synthetic techniques, or combinations of the foregoing, and the like.

It should be noted that the CDRs and FRs of the variable regions of the antibodies and the trispecific antigen-binding molecules of the present disclosure are divided according to the Kabat definition. Other naming and numbering systems, such as Chothia, IMGT, or AHo, are known to those skilled in the art. Thus, humanized antibodies comprising one or more CDRs derived from any naming system, based on the antibody sequences of the present disclosure, are expressly maintained within the scope of the present disclosure.

The term "humanized antibody" refers to an antibody in which all or a portion of the amino acids other than CDRs of a non-human antibody (for example, a mouse antibody) are replaced by corresponding amino acids derived from a human immunoglobulin. Minor additions, deletions, insertions, substitutions, or modifications of amino acids are permissible as long as they do not eliminate the ability of the antibody to bind to a particular antigen. "Humanized "antibody retains antigen specificity similar to the original antibody.

The term "chimeric antibody" refers to an antibody in which the variable region is derived from one species and the constant region is derived from another species, for example, an antibody in which the variable region is derived from a mouse antibody and the constant region is derived from a human antibody.

As used herein, "homologous" and "heterologous" are a set of relative concepts that may refer either to the same or different sources of different elements in a construct, or to the fact that, after the construct has been constructed, several elements of the same original source, i.e., "homologous", have been modified to become "heterologous" in comparison to other original elements that have not been modified.

The term "antigen" refers to a substance that is recognized by and specifically bound by an antibody or antibody binding fragment. Broadly, an antigen can include any immunogenic fragments or determinants of a selected target, including a single epitope, multiple epitopes, a single domain, multiple domains, an intact extracellular domain (ECD), or a protein. Peptides, proteins, glycoproteins, polysaccharides, and lipids, portions thereof, and combinations thereof, may all serve as antigens. Non-limiting exemplary antigens include tumor antigens or pathogen antigens, and the like. "An antigen" may also refer to a molecule that triggers the immune response. Any forms of an antigen, or cells or preparations containing the antigen, can be used to generate antibodies specific to the antigenic determinants.

The terms "epitope" and "antigenic determinant" refer to a site on an antigen that specifically binds to an immunoglobulin or an antibody. Epitopes may be formed from adjacent amino acids or from non-adjacent amino acids that are juxtaposed through the tertiary folding of the protein. Epitopes formed from adjacent amino acids are generally retained after exposure to denaturing solvents, whereas epitopes formed by tertiary folding are generally lost after treatment with denaturing solvents. Epitopes generally exist in unique spatial conformations and include at least 3-15 amino acids.

The term "multispecific" refers to the ability of an antigen-binding molecule to specifically bind to multiple different antigenic determinants. The term "antigen-binding molecule" in its broadest sense refers to a molecule that specifically binds to an antigenic determinant. Examples of the antigen-binding molecules are immunoglobulins and derivatives thereof, such as fragments. The term "trispecific antigen-binding molecule" or "trispecific binding molecule" refers to a binding molecule (for example, an antibody or a molecule comprising an antibody fragment), particularly a trispecific antibody, that is specific for three different antigens (or epitopes).

The term "specific binding" means selective binding to an antigen and can be distinguished from undesirable or non-specific interactions. The ability of an antibody to bind to a specific antigenic determinant can be determined by an enzyme-linked immunosorbent assay (ELISA) or other techniques known to those skilled in the art.

When an antibody, a binding molecule, a bispecific binding molecule, or a multispecific binding molecule is made from the variable regions in the present disclosure, the constant region is not specifically limited. A constant region known to those skilled in the art or one obtained independently may be used, and amino acid mutations (such as a mutation that increase or decrease the binding of Fc to a receptor or FcRn) may also be introduced into the constant region.

There is no particular limitation to the methods for obtaining the binding molecules, antigen-binding fragments, antibodies, bispecific binding molecules or multispecific binding molecules of the present disclosure, and these molecules can be obtained by any methods. The binding molecules, antigen-binding fragments, antibodies, bispecific binding molecules or multispecific binding molecules of the invention can be prepared and purified using conventional methods. For example, cDNA sequences encoding the heavy and light chains can be cloned and recombined into expression vectors. Recombinant immunoglobulin expression vectors can be stably transfected into CHO cells. As a more preferred technique in the prior art, the mammalian expression system results in glycosylation of antibodies, particularly at the highly conserved N-terminus of the Fc region. Stable clones are obtained by expressing antibodies that specifically bind to human antigens. Positive clones were expanded in serum-free medium in a bioreactor to produce antibodies. The culture medium in which the antibody is secreted can be purified and collected using conventional techniques. The antibody can be concentrated by filtration using conventional methods. Soluble mixtures and multimers may also be removed by conventional methods, such as molecular sieves or ion exchange.

As used herein, the term "transfection" refers to the introduction of an exogenous nucleic acid into an eukaryotic cell. Transfection can be accomplished by a variety of means known in the art, including calcium phosphate-DNA co-precipitation, DEAE-dextran-mediated transfection, polybrene-mediated transfection, electroporation, microinjection, liposome fusion, lipid transfection, protoplast fusion, retroviral infection, and biolistics.

The term "stable transfection" refers to the introduction and integration of an exogenous nucleic acid, DNA, or RNA into the genome of a transfected cell. The term "stable transfectant" refers to a cell in which foreign DNA is stably integrated into genomic DNA.

The term "antibody-drug conjugate" (ADC) refers to an antibody that has been covalently coupled to a therapeutically active substance or an active pharmaceutical ingredient (API) so that the therapeutically active substance or the active pharmaceutical ingredient (API) can be targeted to a binding target of the antibody to exhibit its pharmacological function. The therapeutically active substance or the active pharmaceutical ingredient may be a cytotoxin capable of killing ADC targeted cells, preferably malignant or cancerous cells. The covalent attachment of the therapeutically active substance, the active pharmaceutical ingredient or the cytotoxin can be carried out in a non-site-specific manner using a standard chemical linker with an effective payload coupled to a lysine or cysteine residue, or preferably, the conjugation is carried out in a site-specific manner that allows complete control of the conjugation site and the drug-to-antibody ratio of the resulting ADC.

The term "amino acid substitution", "substitution", or "replacement" means that an amino acid at a particular position in a parent polypeptide sequence is replaced with another amino acid.

The term "affinity" or "binding affinity" refers to the strength of the sum of all non-covalent interactions between a single binding site of a molecule (for example, an antibody) and its binding partner (for example, an antigen). The term "$K_d$" refers to the dissociation constant of a particular antibody-antigen interaction. Various techniques known in the art can be used to determine binding affinity, such as surface plasmon resonance, biolayer interferometry, bipolarized interferometry, static light scattering, dynamic light scattering, isothermal titration calorimetry, ELISA, analytical ultracentrifugation, flow cytometry, and the like.

The term "biological activity" refers to the ability of an antibody to bind to an antigen and induce a measurable biological response that can be measured *in vitro* or *in vivo.*

The term "pharmaceutical composition" refers to a formulation or combination of formulations comprising one, two or more active ingredients that allows the active ingredients contained therein to exist in a biologically active effective form and does not include additional ingredients that are unacceptable in toxicity to the subject to whom the formulation is administered. When the "pharmaceutical composition" is in the form of a combination of separate formulations containing two or more different active ingredients, it may be administered simultaneously, sequentially, separately, or at intervals, with the aim of exerting the biological activity of the various active ingredients together for the treatment of diseases.

The binding molecules or antigen-binding fragments of the present disclosure may be used in combination with other drugs, where the active ingredients may be mixed together to form a single administration unit, or may be separately made as administration units and used individually.

The term "effective amount" refers to the dose of a pharmaceutical formulation of the antibody or fragment thereof of the present disclosure that, upon administration to a patient in a single or multiple doses, produces the expected effect in the patient being treated. An effective amount can be easily determined by the attending physician, who is skilled in the art, by considering a variety of factors such as racial differences; weight, age, and health status; the specific disease involved; the severity of the disease; the individual patient's response; the specific antibody administered; the mode of administration; the bioavailability profile of the administered formulation; the chosen dosing protocol; and the use of any concomitant therapy.

As used herein, the term "individual" or "subject" refers to any animals, such as mammals or marsupials. The individual of the present disclosure includes, but is not limited to, human, non-human primates (such as cynomolgus monkey, rhesus monkey, or other types of macaques), mouse, pig, horse, donkey, cattle, sheep, rat, and any kind of poultry.

As used herein, the term "disease", "condition", "disorder", or the like refers to any alteration or imbalance that impairs or interferes with the normal function of a cell, tissue, or organ. For example, the "disease" includes, but is not limited to, tumors, pathogen infections, autoimmune diseases, T cell dysfunction diseases, or immunotolerance deficiency (such as transplant rejection).

As used herein, the term "tumor" refers to a disease characterized by the pathological proliferation of cells or tissues, and their subsequent migration or invasion into other tissues or organs. Tumor growth is generally uncontrolled and progressive, and it does not induce or inhibit normal cell proliferation.

As used herein, the term "treat" or "treatment" refers to clinical intervention aimed at altering a disease process caused by a person or treating a cell, either prophylactically or during a clinical pathological process. Therapeutic effects include, but are not limited to, prevention of the onset or recurrence of a disease, alleviation of symptoms, reduction of any direct or indirect pathological consequences of the disease, prevention of metastasis, slowing of the rate of progression of a disease, amelioration or alleviation of the disease state, alleviation or improvement of prognosis, and the like.

The terms "G protein-coupled receptor family C5 subtype D" and "GPRC5D" specifically include human GPRC5D proteins, as well as variants, subtypes, homologous species, and analogs that share at least one epitope with GPRC5D (for example, human GPRC5D). Exemplary human GPRC5D sequences can be found in GenBank Accession Number BC069341, NCBI Reference Sequence: NP_061124.1, and UniProtKB/Swiss-Prot Accession Number Q9NZD1 (see also Brauner-Osborne, H et al., 2001, Biochim.Biophys.Acta 1518, 237-248).

The term "BCMA" refers to the tumor-associated antigen B cell maturation antigen, also known as TNFRSF17. The exemplary human BCMA sequences include the human BCMA protein under Accession Number UniProt Q02223.

## DETAILED DESCRIPTION

The present disclosure is further illustrated below in connection with specific examples. It should be understood that these examples are for illustrative purposes only and are not intended to limit the scope of the disclosure. Experimental methods not specifically stated in the following examples are typically carried out under conventional conditions known in the art or according to conditions recommended by the manufacturers.

### Example 1. Antigen information of human GPRC5D, human GPRC5A, and cynomolgus monkey GPRC5D

The full-length amino acid sequence (SEQ ID NO: 1) (Uniprot ID: Q9NZD1) of human GPRC5D (human-GPRC5D) used in the examples is as follows:

MYKDCIESTGDYFLLCDAEGPWGIILE*SLAILGIVVTILLLLAFLFLM*RKIQDCSQWNV
LPTQ*LLFLLSVLGLFGLAFAFIIEL*NQQTAPVRY*FLFGVLFALCFSCLLAHASNL*VKLVRGCV
S*FSWTTILCIAIGCSLLQIIIA*TEYVTLIMTRGMMFVNMTPCQLN*VDFVVLLVYVLFLMALTF
FVS*KATFCGPCENWKQHGR*LIFITVLFSIIIWVVWISMLL*RGNPQFQRQPQWDD*PVVCIALV
TNA*WVFLLLYIVP*ELCILYRSCRQECPLQGNACPVTAYQHSFQVENQELSRARDSDGAEE
DVALTSYGTPIQPQTVDPTQECFIPQAKLSPQQDAGGV

Note: This protein is a seven transmembrane protein. The double underlined portion represents the extracellular region (1-27; 85-93; 145-167; 226-239); the underlined portion represents the intracellular region (49-63; 115-123; 189-204; 261-345); and the italic portion represents the transmembrane region (28-48; 64-84; 94-114; 124-144; 168-188; 205-225; 240-260).

The full-length amino acid sequence (SEQ ID NO: 2) (Uniprot ID: A0A2K5W6I2) of cynomolgus monkey GPRC5D (cyno-GPRC5D) used in the examples is as follows:

MYKDCIESTGDYFLPCDSEGPWGIVLE*SLAILGIVVTILLLLAFLFLM*RKIQDCSQWNV
LPTQ*LLFLLSVLGLFGLAFAFIIQL*NQQTAPVRY*FLFGVLFALCFSCLLAHASNL*VKLVRGRV
S*FSWTTILCIAIGCSLLQVIIA*IEYVTLIMTRGMMFVHMTPYQLN*VDFVVLLVYVLFLMALTF
FVS*KATFCGPCENWKQHGR*LIFITVLFSIIIWVVWISMLL*RGNPQFQRQPQWDD*PVVCIALV
TNA*WVFLLLYIVP*ELCILYRSCRQECPSQGHACPVTAYQRSFQVENQELSRARDSDGAEE
DVALTSFGTPIQPQTVDPTQECFIPRAKLSPQQDAGV

Note: This protein is a seven transmembrane protein. The double underlined portion represents the extracellular domain (1-27; 85-93; 145-167; 226-239); the underlined portion represents the intracellular region (49-63; 115-123; 189-204; 261-344); and the italic portion represents the transmembrane region (28-48; 64-84; 94-114; 124-144; 168-188; 205-225; 240-260).

The full-length amino acid sequence (SEQ ID NO: 36) (Uniprot ID: Q8NFJ5) of human GPRC5A used in the examples is as follows:

MATTVPDGCRNGLKSKYYRLCDKAEAWGIVLET*VATAGVVTSVAFMLTLPILVC*KV
QDSNRRKMLPTQ*FLFLLGVLGIFGLTFAFIIGL*DGSTGPTR*FFLFGILFSICFSCLLAHAVS*LT
KLVRGRKPL*SLLVILGLAVGFSLVQDVIAI*EYIVLTMNRTNVNVFSELSAPRRNED*FVLLLTY

*VLFLMALTFLMSSF*<u>TFCGSFTGWKRHGAH</u>*IYLTMLLSIAIWVAWITLLML*<u>PDFDRRWDDTIL</u>

<u>SS</u>*ALAANGWVFLLAYVSPEFWLL*<u>TKQRNPMDYPVEDAFCKPQLVKKSYGVENRAYSQEEI</u>

<u>TQGFEETGDTLYAPYSTHFQLQNQPPQKEFSIPRAHAWPSPYKDYEVKKEGS</u>

Note: This protein is a seven transmembrane protein. The double underlined portion represents the extracellular region (1-33; 90-97; 151-176; 234-247); the underlined portion represents the intracellular region (55-68; 119-129; 198-212; 269-357); and the italic portion represents the transmembrane region (34-54; 69-89; 98-118; 130-150; 177-197; 213-233; 248-268).

### Example 2. Preparation of cell lines expressing human GPRCSD, human GPRCSA, and cynomolgus monkey GPRCSD

The nucleotide sequence encoding the amino acids of the human GPRC5D as set forth in SEQ ID NO: 1 was cloned into the pCMV3 (SinoBiological, Cat. No.: CV011) vector to obtain a plasmid for the construction of a human GPRC5D cell line. The obtained plasmid was transfected into CHOK1 cells (ATCC, Accession Number CCL-61) to obtain a CHOK1 cell line expressing the human GPRC5D (abbreviated as: Human GPRC5D-CHOK1 cell line).

The nucleotide sequence encoding the amino acids of the cynomolgus monkey GPRC5D as set forth in SEQ ID NO: 2 was cloned into the pCMV3 vector to obtain a plasmid for the construction of a cynomolgus monkey GPRC5D cell line. The obtained plasmid was transfected into CHOK1 cells to obtain a CHOK1 cell line expressing the cynomolgus monkey GPRC5D (abbreviated as: Cynomolgus monkey GPRC5D-CHOK1 cell line).

The nucleotide sequence encoding the amino acids of the human GPRC5A as set forth in SEQ ID NO: 36 was cloned into the pCMV3 vector to obtain a vector for the construction of a human GPRC5A cell line. The obtained vector was transfected into CHOK1 cells to obtain a CHOK1 cell line expressing the human GPRC5A (abbreviated as: Human GPRC5A-CHOK1 cell line).

The expression of the human GPRC5D in the human GPRC5D-CHOK1 cell line obtained as described above was detected using the FACS assay, and a negative control, hIgG1 LALA isotype (Biointron, Cat. No.: B109802), was included in the experiment.

The expression of the cynomolgus monkey GPRC5D in the cynomolgus monkey GPRC5D-CHOK1 cell line obtained as described above was detected using the FACS assay, and a negative control, hIgG1 LALA isotype (Biointron, Catalog: B109802), was included in the experiment.

The expression of the human GPRC5A in the human GPRC5A-CHOK1 cell line obtained as described above was detected using the FACS assay, and a negative control, hIgG1 LALA isotype (Biointron, Catalog: B109802), was included in the experiment.

The results showed that:
The expression of the human GPRC5D in the human GPRC5D-CHOK1 cell line is shown in FIG. 1A. The results indicate that the human GPRC5D is well overexpressed in CHOK1 cells and can be used in subsequent experiments to verify the binding of the GPRC5D monoclonal antibody to the human GPRC5D at the cellular level.

The expression of the cynomolgus monkey GPRC5D in the cynomolgus monkey GPRC5D-CHOK1 cell line is shown in FIG. 1B. The results indicate that the cynomolgus monkey GPRC5D is well overexpressed in CHOK1 cells and can be used in subsequent experiments to verify the binding of the GPRC5D monoclonal antibody to the cynomolgus monkey GPRC5D at the cellular level.

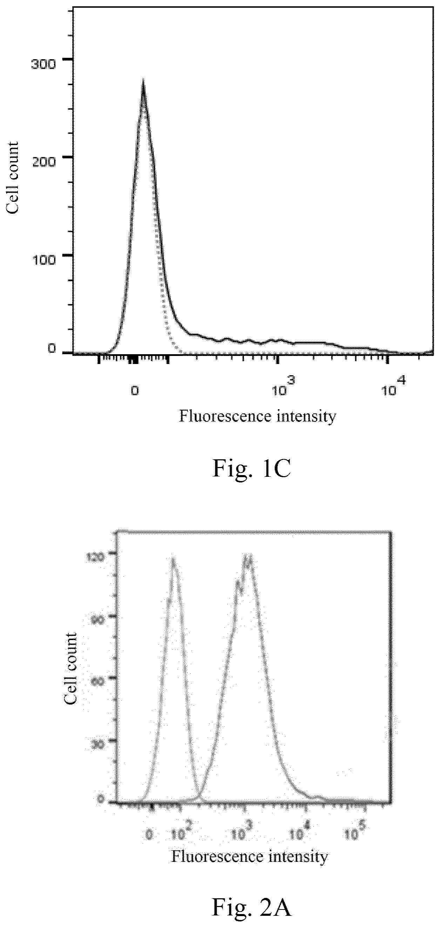

The expression of the human GPRC5A in the human GPRC5A-CHOK1 cell line is shown in FIG. 1C. The results indicate that the human GPRC5A is well overexpressed in CHOK1 cells and can be used in subsequent experiments to verify that no non-specific binding of the GPRC5D monoclonal antibody to the human GPRC5A at the cellular level.

### Example 3. Antigen information of human BCMA and cynomolgus monkey BCMA

The full-length amino acid sequence (SEQ ID NO: 3) (Uniprot ID: Q02223) of the human BCMA (human-BCMA) used in the examples is as follows.

MLQMAGQCSQNEYFDSLLHACIPCQLRCSSNTPPLTCQRYCNASVTNSVKGTNAIL

WTCLGLSLIISLAVFVLMFLLRKINSEPLKDEFKNTGSGLLGMANIDLEKSRTGDEIILPRG

LEYTVEECTCEDCIKSKPKVDSDHCFPLPAMEEGATILVTTKTNDYCKSLPAALSATEIEK

SISAR

Note: This protein is a single transmembrane protein. The double underlined portion represents the extracellular region (1-54); the wavy underlined portion represents the transmembrane region (55-77); and the underlined portion represents the intracellular region (78-184).

The full-length amino acid sequence (SEQ ID NO: 4) (Uniprot ID: G7Q0I4) of the cynomolgus monkey BCMA (cyno-BCMA) used in the examples is as follows.

MLQMARQCSQNEYFDSLLHDCKPCQLRCSSTPPLTCQRYCNASMTNSVKGMNAIL

WTCLGLSLIISLAVFVLTFLLRKMSSEPLKDEFKNTGSGLLGMANIDLEKGRTGDEIVLPR

GLEYTVEECTCEDCIKNKPKVDSDHCFPLPAMEEGATILVTTKTNDYCNSLSAALSVTEIE

KSISAR

Note: This protein is a single transmembrane protein. The double underlined portion represents the extracellular region (1-53); the wavy underlined portion represents the transmembrane region (54-76); and the underlined portion represents the intracellular region (77-183).

## Example 4. Preparation of human BCMA and cynomolgus monkey BCMA cell lines

The nucleotide sequence encoding the amino acids of the human BCMA as set forth in SEQ ID NO: 3 was cloned into a pCMV3 (SinoBiological, Cat.: CV011) vector to obtain a vector for construction of a human BCMA cell line. The obtained vector was transfected into CHOK1 cells (ATCC, Accession Number CCL-61) to obtain a CHOK1 cell line expressing the human BCMA (abbreviated as: Human BCMA-CHOK1 cell line).

The nucleotide sequence encoding the amino acids of the cynomolgus monkey BCMA as set forth in SEQ ID NO: 4 was cloned into the pCDH-CMV-MCS-EF1-Hygro (Honorgene, Cat. No.: CD515B-1) vector to obtain a vector for construction of a cynomolgus monkey BCMA cell line. The obtained vector was constructed into a virus and then transfected into CHOK1 cells to obtain a CHOK1 cell line expressing the cynomolgus monkey BCMA (abbreviated as: Cynomolgus monkey BCMA-CHOK1 cell line).

The expression of the human BCMA in the human BCMA-CHOK1 cell line obtained as described above was detected using the FACS assay, and a negative control, hIgG1 LALA isotype (Biointron, Catalog: B109802), was included in the experiment.

The expression of the cynomolgus monkey BCMA in the cynomolgus monkey BCMA-CHOK1 cell line obtained as described above was detected using the FACS assay, and a negative control, hIgG1 LALA isotype (Biointron, Catalog: B109802), was included in the experiment.

The results showed that:

The expression of the human BCMA in the human BCMA-CHOK1 cell line is shown in FIG. 2A. The results indicate that the human BCMA is well overexpressed in CHOK1 cells and can be used in subsequent experiments to verify the binding of the BCMA monoclonal antibody to the human BCMA at the cellular level.

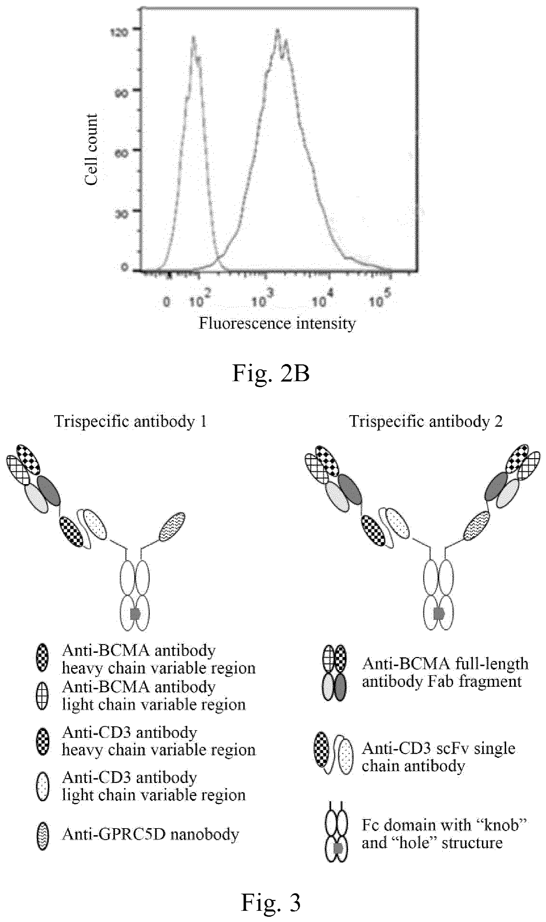

The expression of the cynomolgus monkey BCMA in the cynomolgus monkey BCMA-CHOK1 cell line is shown in FIG. 2B. The results indicate that the cynomolgus monkey BCMA is well overexpressed in CHOK1 cells and can be used in subsequent experiments to verify the binding of the BCMA monoclonal antibody to the cynomolgus monkey BCMA at the cellular level.

## Example 5. Design and sequence of anti-GPRC5D-BCMA-CD3 trispecific antibodies

An anti-BCMA full-length antibody (BCMA mAb) was obtained by hybridoma screening, with the heavy chain sequence as set forth in SEQ ID NO: 9 and the light chain sequence as set forth in SEQ ID NO: 7.

Anti-GPRC5D nanobody (GPRC5D VHH), whose variable region sequence is as set forth in SEQ ID NO: 10, was obtained by screening using llama (Lama glama) immunization.

The CD3ε binding domain was derived from the full-length antibody CD3 mAb, with the heavy chain sequence as set forth in SEQ ID NO: 11 and the light chain sequence as set forth in SEQ ID NO: 12. The heavy chain variable region and the light chain variable region of the CD3 mAb were linked via a flexible linker to form a single chain antibody scFv with the structure VH - $(G_4S)_3$ - VL, the sequence of which is set forth in SEQ ID NO: 13. The scFv was then fused via a flexible linker to the C-terminus of the heavy chain of the BCMA full-length antibody Fab fragment.

The CD3 scFv was fused to the C-terminus of the Fab fragment heavy chain CH1 of the anti-BCMA antibody to form one arm of the trispecific antibody, while another complete GPRC5D nanobody binding domain and Fc region with "knob" and "hole" structures were introduced. The resulting trispecific antibody was named the trispecific antibody 1. The sequences of the three fused heterologous peptide chains are set forth in SEQ ID NO: 5, SEQ ID NO: 6, and SEQ ID NO: 7, and the schematic diagram is shown in FIG. 3.

The CD3 scFv was fused to the C-terminus of the Fab fragment heavy chain of the BCMA full-length antibody to form one arm of the antibody, and the GPRC5D nanobody was fused to the C-terminus of the other Fab fragment heavy chain of the BCMA full-length antibody to form the other arm of the antibody, while the Fc region with "knob" and "hole" structures was introduced. The resulting trispecific antibody is named the trispecific antibody 2. The sequences of the three fused heterologous peptide chains are set forth in SEQ ID NO: 5, SEQ ID NO: 7, and SEQ ID NO: 8, and the schematic diagram is shown in FIG. 3.

To reduce the ADCC activity of the antibody, the Fc fragments of the final trispecific antibodies were substituted with amino acids of L234A, L235A, and G237A. The Fc domain in the scFv-containing peptide chain was designed as a "knob" structure, including amino acid substitutions at both positions S354C and T366W. The Fc domain in the peptide chain without scFv was designed as a "hole" structure, including amino acid substitutions at four positions Y349C, T366S, L368A, and Y407V. Additionally, to facilitate purification of the trispecific antibodies, the heavy chain having the "hole" structure was further substituted with H435R.

The structures and associated molecular sequences of the trispecific antibody 1 and the trispecific antibody 2 are summarized in Table 1 and Table 2, respectively.

Table 1. Description of the structures of trispecific antibodies

| Name | Description of moleculer structure | SEQ ID NO: |
|---|---|---|
| Trispecific antibody 1 | BCMA VH - BCMA CH1 - G$_4$S - CD3VH - (G$_4$S)$_3$ - CD3VL - EPKSS - IgG1 Fc (knob) | 5 |
| | GPRC5D VHH - EPKSS - IgG1 Fc (hole) | 6 |
| | BCMA VL - BCMA CL (i.e., BCMA LC) | 7 |
| Trispecific antibody 2 | BCMA VH - BCMA CH1 - G$_4$S - CD3VH - (G$_4$S)$_3$ - CD3VL - IgG1 Fc (knob) | 5 |
| | BCMA VH - BCMA CH1 - G$_4$S - GPRC5D VHH - EPKSS - IgG1 Fc (hole) | 8 |
| | BCMA VL - BCMA CL (i.e., BCMA LC) x2 | 7 |
| BCMA mAb | BCMA HC | 9 |
| | BCMA LC | 7 |
| GPRC5D VHH | GPRC5D VHH | 10 |
| CD3 mAb | CD3 HC | 11 |
| | CD3 LC | 12 |
| CD3 scFv | CD3VH - (G$_4$S)$_3$ - CD3VL | 13 |

Table 2. Amino acid sequences of trispecific antibodies

| Name | SEQ ID NO: | Amino acid sequence |
|---|---|---|
| The heavy chain with the "knob" structure of trispecific antibody 1 | 5 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCGGGGSEVKLVESGGGLV QPGGSLKLSCAASGFTFNTYAMNWVRQAPGKGLEWVARI RSKYNNYATYYADSVKDRFTISRDDAKNTLYLQMNNLRT EDTAVYYCVRHGNFGNSYVSWFAYWGQGTLVTVSSGGG GSGGGGSGGGGSQAVVTQEPSLTVSPGGTVTLTCRSSTGA VTTSNYANWVQQKPGQAPRGLIGGTNKRAPGTPARFSGSL LGGKAALTLSGAQPEDEAEYYCALWYSNLWVFGGGTKLT VLEPKSSDKTHTCPPCPAPEAAGAPSVFLFPPKPKDTLMISR TPEVTCVVVDVSHEDPEVKFNWYVDGVEVHNAKTKPREE QYNSTYRVVSVLTVLHQDWLNGKEYKCKVSNKALPAPIE KTISKAKGQPREPQVYTLPPCRDELTKNQVSLWCLVKGFY PSDIAVEWESNGQPENNYKTTPPVLDSDGSFFLYSKLTVDK SRWQQGNVFSCSVMHEALHNHYTQKSLSLSPGK |
| The heavy chain with the "hole" structure of trispecific antibody 1 | 6 | QVQLVESGGGVVQPGGSLRLSCAASGLFFASSDMSWFRQ APGKGRELVAEITSVGNNIKYADSVEGRFTISRDNSKNTVY LQMNSLRAEDTAVYYCSARRRVRYWGQGTLVTVSSEPKS SDKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVCTLPPSRDELTKNQVSLSCAVKGFYPSDIAVE WESNGQPENNYKTTPPVLDSDGSFFLVSKLTVDKSRWQQ GNVFSCSVMHEALHNRYTQKSLSLSPGK |
| The light chain of trispecific antibody 1 | 7 | DIQLTQSPSSLSASVGDRVTITCSASSGVSHMHWYQQKPG KAPKRWIYDTSKLTSGVPSRFSGSGSGTDFTLTISSLQPEDF ATYYCQQWSRNPWTFGQGTKVEIKRTVAAPSVFIFPPSDE QLKSGTASVVCLLNNFYPREAKVQWKVDNALQSGNSQES VTEQDSKDSTYSLSSTLTLSKADYEKHKVYACEVTHQGLS SPVTKSFNRGEC |
| The heavy chain with the "knob" structure of trispecific antibody 2 | 5 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCGGGGSEVKLVESGGGLV QPGGSLKLSCAASGFTFNTYAMNWVRQAPGKGLEWVARI RSKYNNYATYYADSVKDRFTISRDDAKNTLYLQMNNLRT EDTAVYYCVRHGNFGNSYVSWFAYWGQGTLVTVSSGGG GSGGGGSGGGGSQAVVTQEPSLTVSPGGTVTLTCRSSTGA VTTSNYANWVQQKPGQAPRGLIGGTNKRAPGTPARFSGSL LGGKAALTLSGAQPEDEAEYYCALWYSNLWVFGGGTKLT VLEPKSSDKTHTCPPCPAPEAAGAPSVFLFPPKPKDTLMISR TPEVTCVVVDVSHEDPEVKFNWYVDGVEVHNAKTKPREE |

(continued)

| Name | SEQ ID NO: | Amino acid sequence |
|------|------------|---------------------|
| | | QYNSTYRVVSVLTVLHQDWLNGKEYKCKVSNKALPAPIE KTISKAKGQPREPQVYTLPPCRDELTKNQVSLWCLVKGFY PSDIAVEWESNGQPENNYKTTPPVLDSDGSFFLYSKLTVDK SRWQQGNVFSCSVMHEALHNHYTQKSLSLSPGK |
| The heavy chain with the "hole" structure of trispecific antibody 2 | 8 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCGGGGSQVQLVESGGGVV QPGGSLRLSCAASGLFFASSDMSWFRQAPGKGRELVAEITS VGNNIKYADSVEGRFTISRDNSKNTVYLQMNSLRAEDTAV YYCSARRRVRYWGQGTLVTVSSEPKSSDKTHTCPPCPAPE AAGAPSVFLFPPKPKDTLMISRTPEVTCVVVDVSHEDPEVK FNWYVDGVEVHNAKTKPREEQYNSTYRVVSVLTVLHQD WLNGKEYKCKVSNKALPAPIEKTISKAKGQPREPQVCTLP PSRDELTKNQVSLSCAVKGFYPSDIAVEWESNGQPENNYK TTPPVLDSDGSFFLVSKLTVDKSRWQQGNVFSCSVMHEAL HNRYTQKSLSLSPGK |
| The light chain of trispecific antibody 2 | 7 | DIQLTQSPSSLSASVGDRVTITCSASSGVSHMHWYQQKPG KAPKRWIYDTSKLTSGVPSRFSGSGSGTDFTLTISSLQPEDF ATYYCQQWSRNPWTFGQGTKVEIKRTVAAPSVFIFPPSDE QLKSGTASVVCLLNNFYPREAKVQWKVDNALQSGNSQES VTEQDSKDSTYSLSSTLTLSKADYEKHKVYACEVTHQGLS SPVTKSFNRGEC |
| BCMA heavy chain | 9 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCDKTHTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL TKNQVSLTCLVKGFYPSDIAVEWESNGQPENNYKTTPPVL DSDGSFFLYSKLTVDKSRWQQGNVFSCSVMHEALHNHYT QKSLSLSPGK |
| BCMA light chain | 7 | DIQLTQSPSSLSASVGDRVTITCSASSGVSHMHWYQQKPG KAPKRWIYDTSKLTSGVPSRFSGSGSGTDFTLTISSLQPEDF ATYYCQQWSRNPWTFGQGTKVEIKRTVAAPSVFIFPPSDE QLKSGTASVVCLLNNFYPREAKVQWKVDNALQSGNSQES VTEQDSKDSTYSLSSTLTLSKADYEKHKVYACEVTHQGLS SPVTKSFNRGEC |
| BCMA heavy chain variable region VH | 14 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS S |

(continued)

| Name | SEQ ID NO: | Amino acid sequence |
|---|---|---|
| BCMA light chain variable region VL | 15 | DIQLTQSPSSLSASVGDRVTITCSASSGVSHMHWYQQKPG KAPKRWIYDTSKLTSGVPSRFSGSGSGTDFTLTISSLQPEDF ATYYCQQWSRNPWTFGQGTKVEIK |
| BCMA heavy chain HCDR1 | 16 | SSYIS |
| BCMA heavy chain HCDR2 | 17 | WIYAGSGNADYNQKFTG |
| BCMA heavy chain HCDR3 | 18 | HYDPPHYFDY |
| BCMA light chain LCDR1 | 19 | SASSGVSHMH |
| BCMA light chain LCDR2 | 20 | DTSKLTS |
| BCMA light chain LCDR3 | 21 | QQWSRNPWT |
| GPRC5D heavy chain variable region VHH | 10 | QVQLVESGGGVVQPGGSLRLSCAASGLFFASSDMSWFRQ APGKGRELVAEITSVGNNIKYADSVEGRFTISRDNSKNTVY LQMNSLRAEDTAVYYCSARRRVRYWGQGTLVTVSS |
| GPRC5D heavy chain HCDR1 | 22 | SSDMS |
| GPRC5D heavy chain HCDR2 | 23 | EITSVGNNIKYADSVEG |
| GPRC5D heavy chain HCDR3 | 24 | RRRVRY |
| CD3 heavy chain | 11 | EVKLVESGGGLVQPGGSLKLSCAASGFTFNTYAMNWVRQ APGKGLEWVARIRSKYNNYATYYADSVKDRFTISRDDAK NTLYLQMNNLRTEDTAVYYCVRHGNFGNSYVSWFAYWG QGTLVTVSSASTKGPSVFPLAPSSKSTSGGTAALGCLVKDY FPEPVTVSWNSGALTSGVHTFPAVLQSSGLYSLSSVVTVPSS SLGTQTYICNVNHKPSNTKVDKKVEPKSCDKTHTCPPCPA PEAAGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTL PPSRDELTKNQVSLTCLVKGFYPSDIAVEWESNGQPENNYK TTPPVLDSDGSFFLYSKLTVDKSRWQQGNVFSCSVMHEAL HNHYTQKSLSLSPGK |
| CD3 light chain | 12 | QAVVTQEPSLTVSPGGTVTLTCRSSTGAVTTSNYANWVQQ KPGQAPRGLIGGTNKRAPGTPARFSGSLLGGKAALTLSGA QPEDEAEYYCALWYSNLWVFGGGTKLTVLGQPKANPTVT LFPPSSEELQANKATLVCLISDFYPGAVTVAWKADSSPVKA GVETTTPSKQSNNKYAASSYLSLTPEQWKSHRSYSCQVTH EGSTVEKTVAPTEC |
| CD3 heavy chain variable region VH | 25 | EVKLVESGGGLVQPGGSLKLSCAASGFTFNTYAMNWVRQ APGKGLEWVARIRSKYNNYATYYADSVKDRFTISRDDAK NTLYLQMNNLRTEDTAVYYCVRHGNFGNSYVSWFAYWG QGTLVTVSS |
| CD3 light chain variable region VL | 26 | QAVVTQEPSLTVSPGGTVTLTCRSSTGAVTTSNYANWVQQ KPGQAPRGLIGGTNKRAPGTPARFSGSLLGGKAALTLSGA QPEDEAEYYCALWYSNLWVFGGGTKLTVL |

(continued)

| Name | SEQ ID NO: | Amino acid sequence |
|---|---|---|
| The scFv of CD3 | 13 | EVKLVESGGGLVQPGGSLKLSCAASGFTFNTYAMNWVRQ APGKGLEWVARIRSKYNNYATYYADSVKDRFTISRDDAK NTLYLQMNNLRTEDTAVYYCVRHGNFGNSYVSWFAYWG QGTLVTVSSGGGGSGGGGSGGGGSQAVVTQEPSLTVSPGG TVTLTCRSSTGAVTTSNYANWVQQKPGQAPRGLIGGTNK RAPGTPARFSGSLLGGKAALTLSGAQPEDEAEYYCALWYS NLWVFGGGTKLTVL TYAMN |
| CD3 heavy chain HCDR1 | 27 | TYAMN |
| CD3 heavy chain HCDR1 | 28 | RIRSKYNNYATYYADSVKD |
| CD3 heavy chain HCDR1 | 29 | HGNFGNSYVSWFAY |
| CD3 light chain LCDR1 | 30 | RSSTGAVTTSNYAN |
| CD3 light chain LCDR2 | 31 | GTNKRAP |
| CD3 light chain LCDR3 | 32 | ALWYSNLWV |
| First Fc domain sequence | 33 | DKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVYTLPPCRDELTKNQVSLWCLVKGFYPSDIAV EWESNGQPENNYKTTPPVLDSDGSFFLYSKLTVDKSRWQQ GNVFSCSVMHEALHNHYTQKSLSLSPGK |
| Second Fc domain sequence | 34 | DKTHTCPPCPAPEAAGAPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISKA KGQPREPQVCTLPPSRDELTKNQVSLSCAVKGFYPSDIAVE WESNGQPENNYKTTPPVLDSDGSFFLVSKLTVDKSRWQQ GNVFSCSVMHEALHNRYTQKSLSLSPGK |
| BCMA Fab fragment heavy chain | 35 | QVQLVQSGAEVKKPGASVKVSCKASGFTFRSSYISWVRQA PGQGLEWIAWIYAGSGNADYNQKFTGRVTMTVDTSTSTV YMELSSLRSEDTAVYYCARHYDPPHYFDYWGQGTLVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKV |

## Example 6. Construction of the anti-GPRC5D-BCMA-CD3 trispecific antibodies and their transient expression by transfection in eukaryotic cells

The gene coding fragments of the trispecific antibodies described above were separately cloned into the PTT5 expression vector to prepare transfection-grade expression plasmids.

Expi293F™ cells (Thermo Fisher Scientific) were incubated in a serum-free medium, seeded into shake flasks (Corning Inc.), and incubated on a shaker at 37°C, 8% $CO_2$. The cell density was adjusted, and the recombinant expression vector containing the gene fragment of interest was mixed with the transfection reagent PEI at an appropriate ratio and added to the shake flasks for cell culture. After 6 days of cell culture, the expression supernatant was collected, the cell debris was removed by high-speed centrifugation, and the antibodies were affinity purified using the Protein A column. The column was washed with PBS until the A280 fell to the baseline. The target proteins were eluted with an acidic eluent (pH 3.0-3.5) and neutralized with 1 M Tris-HCl (pH 8.0-9.0). After the eluted samples were appropriately concentrated, they were further purified using a PBS-equilibrated gel filtration column Superdex200 (GE) to remove aggregates. The monomer peak was collected and buffer-exchanged into PBS for aliquoting. The final purified antibodies were subjected to SDS-PAGE and HPLC purity analysis, and A280 concentration determination.

At the same time, the GPRC5DxCD3 bispecific antibody Talquetamab from Janssen (derived from WO2018017786A2, the antibody consists of the sequences as set forth in SEQ ID NOs: 25, 26, 55, and 58, and contains a GPRC5D binding site and a CD3 binding site) was also expressed and purified.

At the same time, the BCMAxCD3 bispecific antibody Teclistamab from Janssen (derived from WO2019220368A1, the antibody consists of the sequences as set forth in SEQ ID NOs: 31, 32, 41, and 42, and contains a BCMA binding site and a CD3 binding site) was also expressed and purified.

At the same time, the BGCB491 trispecific antibody from Janssen (derived from WO2022175255A2, the antibody consists of the sequences as set forth in SEQ ID NOs: 29, 30, and 31, and contains a GPRC5D binding site, a BCMA binding site, and a CD3 binding site) was also expressed and purified.

At the same time, the TS-F2-5 trispecific antibody from Innovent Biologics (derived from WO2022174813A1, the antibody consists of the sequences as set forth in SEQ ID NOs: 68, 75, 76, and 78, and contains a GPRC5D binding site, a BCMA binding site, and a CD3 binding site) was also expressed and purified.

## Example 7. Affinity test of the anti-GPRC5D-BCMA-CD3 trispecific antibodies at cell-level

The FACS was used to detect the binding of the anti-GPRC5D-BCMA-CD3 trispecific antibodies to NCI-H929 cells and RPMI8226 cells expressing the dual-target human GPRC5D cells and human BCMA, human GPRC5D-expressing human GPRC5D-CHOK1 cells, human BCMA-expressing human BCMA-CHOK1 cells, and naturally hCD3-expressing T lymphocytes (Jurkat) and Human PBMCs.

NCI-H929 cells (ATCC, Accession Number CRL-9068), RPMI8226 cells (ATCC, Accession Number CCL-155), Human GPRC5D-CHOK1 cells (Example 2), Human BCMA-CHOK1 cells (Example 4), and Jurkat cells (ATCC, Accession Number TIB-152) were cultured. The medium for NCI-H929 cells was RPMI1640 + 10% FBS + 0.05 mM mercaptoethanol, the medium for RPMI8226 and Jurkat cells was RPMI1640 + 10% FBS, and the medium for Human GPRC5D-CHOK1 and Human BCMA-CHOK1 cells was F12K + 10% FBS + 400 $\mu$g/ml Hygromycin B, and they were incubated in a 37°C, 5% $CO_2$ incubator using T75 cell culture flasks. When the cells were needed, NCI-H929 cells, RPMI8226 cells, and Jurkat cells were placed directly into 50 mL centrifuge tubes without digestion, respectively. Human GPRC5D-CHOK1 and Human BCMA-CHOK1 were digested with 0.25% Trypsin-EDTA, and digestion was terminated with F12K + 10% FBS + 400 $\mu$g/ml Hygromycin B.

The resulting cells were centrifuged at 1000 rpm for 5 minutes at room temperature, the supernatant was discarded, and the cells were then resuspended in 1% BSA (in PBS). The purchased Human PBMCs were centrifuged at 1500 rpm for 10 minutes at room temperature, the supernatant was discarded, and the cells were then resuspended in 1% BSA (in PBS). The cells were counted, and the cell density was adjusted to 1E6/mL. The cells were then plated into 96-well round bottom culture plates (corning, Product Number 3799) at 100 $\mu$L/well. The cells were centrifuged at 1500 rpm for 5 minutes at 4°C, the supernatant was discarded, and the cells were then resuspended in 200 $\mu$L of 1% BSA (in PBS). The cells were centrifuged again at 1500 rpm for 5 minutes at 4°C, the supernatant was discarded, and the cells were kept at 4°C for future use. The antibodies to be tested and the negative control hIgG1 LALA isotype (Biointron, Catalog: B109802) were diluted in 1% BSA (in PBS) with a starting concentration of 100 nM, and then serially diluted 5-fold to obtain 8 concentrations. The cells were resuspended in diluted antibodies (100 $\mu$L/well) and incubated at 4°C for 1 hour. The cells were centrifuged at 1500 rpm for 5 minutes at 4°C, and the supernatant was discarded. The cells were resuspended and washed in 160 $\mu$L of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at a 1:400 ratio according to the instructions, and the cells were resuspended in the diluted secondary antibody (100 $\mu$L/well) and incubated at 4°C for 0.5 hour. The cells were centrifuged at 1500 rpm for 5 minutes at 4°C, and the supernatant was discarded. The cells were resuspended and washed in 200 $\mu$L of 1% BSA (in PBS), centrifuged at 1500 rpm for 5 minutes at 4°C, and the supernatant was discarded. The cells were resuspended in 100 $\mu$L of 1% BSA (in PBS), filtered through 300-mesh gauze, and the mean fluorescence intensity in the PE channel was measured by a flow cytometer.

FCS files were exported from the flow cytometer. The mean fluorescence intensity (referred to as MFI below) of the PE channels of each sample was analyzed using FlowJo software. The mean fluorescence intensities as obtained were then imported into GraphPad to determine the half binding concentration (referred to as EC$_{50}$ below) of the antibodies to the cells and the top mean fluorescence intensity (Top MFI).

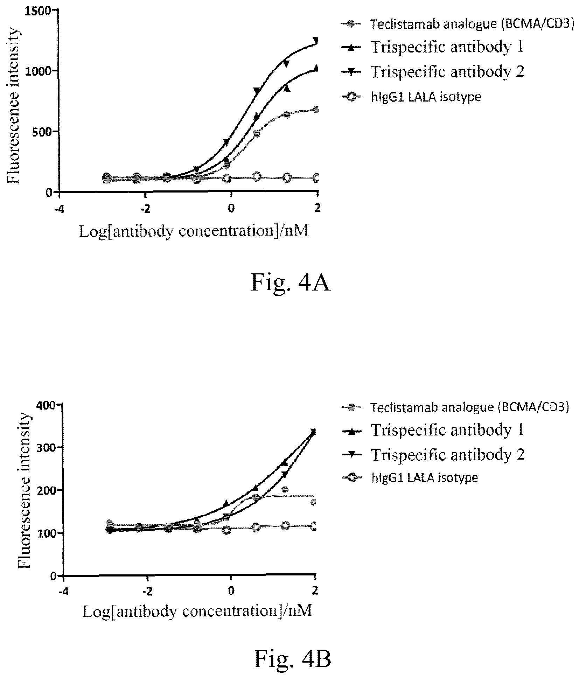

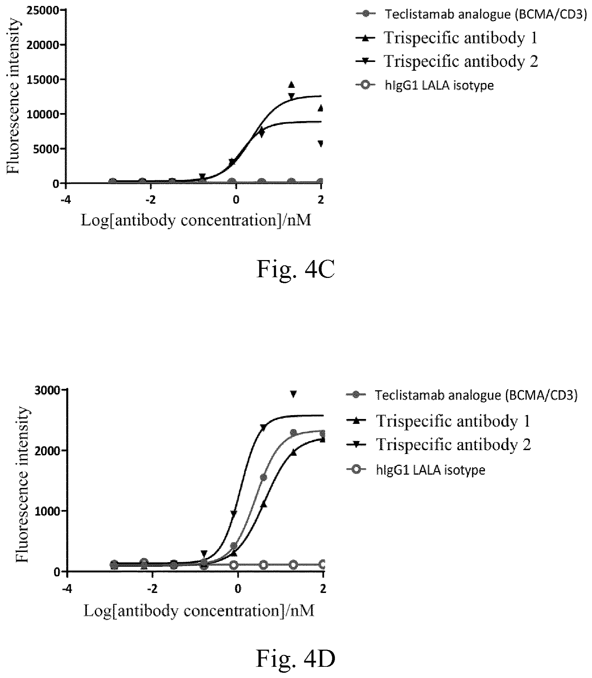

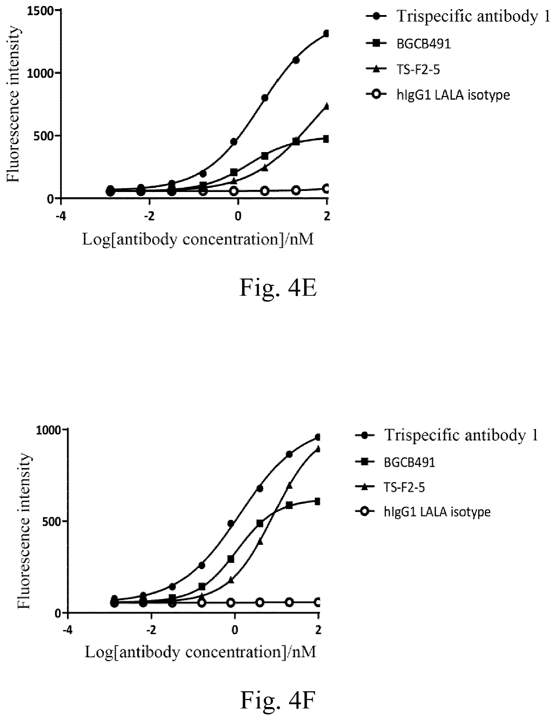

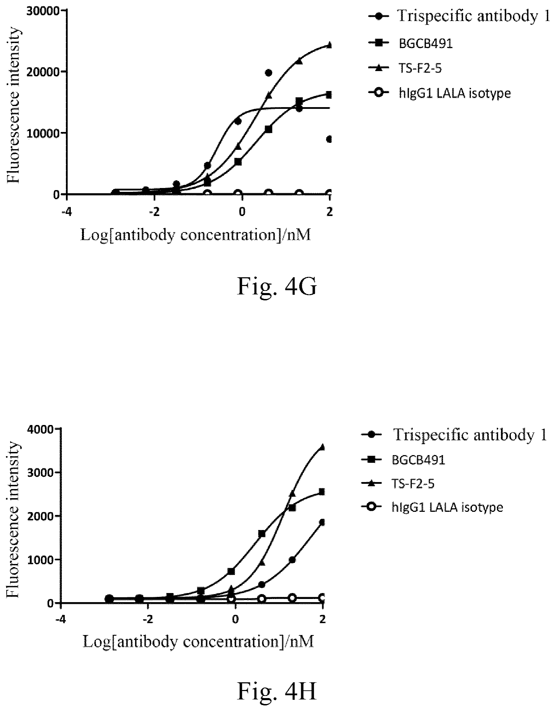

The results of the binding of the test molecules to tumor antigen cells are shown in FIGs. 4A-4H and Tables 3 and 4. In tumor cell lines NCI-H929 and RPMI8226 with dual-target expression, the binding of the trispecific antibodies was stronger than that of the single-target bispecific antibody, and it was positively correlated with the level of tumor antigen expression (the dual-target expression levels in NCI-H929 cells were higher than those in RPMI8226 cells). In cell lines with single-target expression, the binding of the trispecific antibodies was weaker than or slightly stronger than that of the corresponding bispecific antibody. This result is presumed to be caused by the synergistic binding of the two targets. In addition, in tumor cell lines with dual-target expression, the binding of the trispecific antibody 1 was stronger than that of the BGCB491 (Janssen) and the TS-F2-5 (Innovent) molecules.

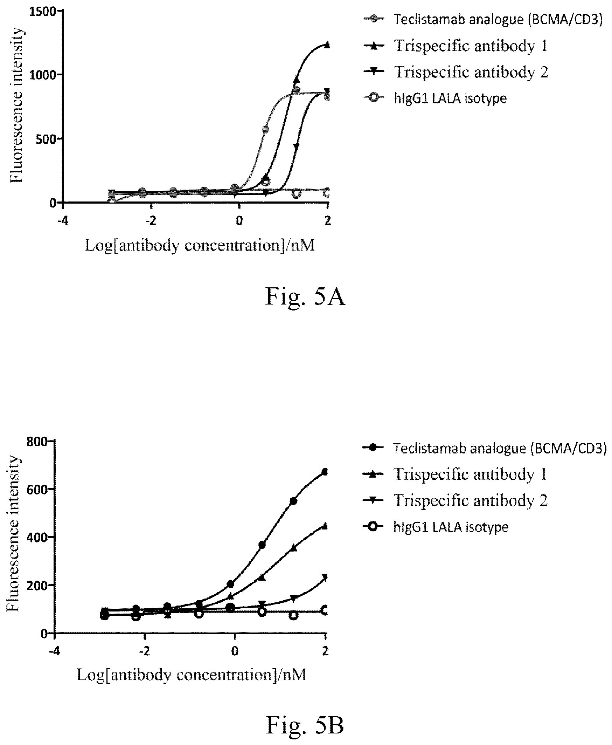

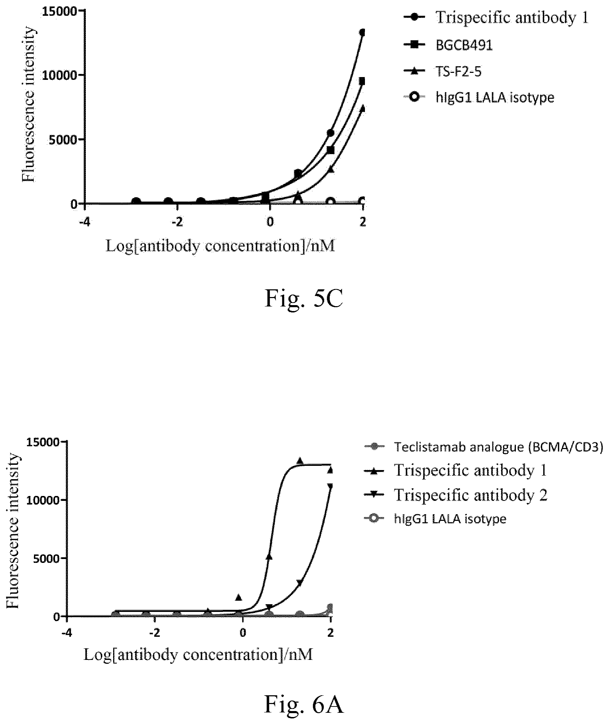

The binding results of the test molecules to Jurkat cells and human PBMCs are shown in FIGs. 5A-5C and Tables 5 and 6. The binding of the trispecific antibodies on Jurkat cells was weaker than that of Teclistamab and comparable to that of BGCB491 (Janssen) and TS-F2-5 (Innovent) molecules.

Table 3. Results of the binding of the trispecific antibodies to tumor antigen-expressing cells

| Molecule | NCI-H929 | | RPMI8226 | | Human BCMA CHOK1 | | Human GPRC5D CHOK1 | |
|---|---|---|---|---|---|---|---|---|
| | $EC_{50}$ (nM) | Top MFI | $EC_{50}$ (nM) | Top MFI | $EC_{50}$ (nM) | Top MFI | $EC_{50}$ (nM) | Top MFI |
| Teclistamab analogue (BCMA/CD3) | 2.43 | 669 | 1.17 | 169 | 2.63 | 2277 | / | 160 |
| Trispecific antibody 1 | 3.53 | 1011 | 53.51 | 334 | 4.25 | 2191 | 2.32 | 10900 |
| Trispecific antibody 2 | 2.47 | 1233 | 199.9 | 332 | 1.14 | 2231 | 1.33 | 5670 |
| hIgG1 LALA isotype | / | 104 | / | 112 | / | 119 | / | 100 |

Table 4. Results of the binding of the trispecific antibodies to tumor antigen-expressing cells

| Molecule | NCI-H929 | | RPMI8226 | | Human GPRC5D CHOK1 | | Human BCMA CHOK1 | |
|---|---|---|---|---|---|---|---|---|
| | $EC_{50}$ (nM) | Top MFI | $EC_{50}$ (nM) | Top MFI | $EC_{50}$ (nM) | Top MFI | $EC_{50}$ (nM) | Top MFI |
| Trispecific antibody 1 | 3.27 | 1314 | 1.34 | 959 | 0.26 | 19800 | 52.56 | 1855 |
| BGCB491 | 1.79 | 473 | 1.12 | 608 | 2.09 | 16200 | 2.84 | 2559 |
| TS-F2-5 | 44.95 | 737 | 8.29 | 896 | 2.08 | 24400 | 12.61 | 3592 |

Table 5. Results of the binding of the trispecific antibodies to Jurkat cells and human PBMCs

| Molecule | Jurkat | | Huamn PBMC | |
|---|---|---|---|---|
| | $EC_{50}$ (nM) | Top MFI | $EC_{50}$ (nM) | Top MFI |
| Teclistamab analogue (BCMA/CD3) | 3.216 | 825 | 6.056 | 672 |
| Trispecific antibody 1 | 11.44 | 1238 | 10.07 | 449 |
| Trispecific antibody 2 | 21.18 | 862 | / | 231 |
| hIgG1 LALA isotype | / | 77.6 | / | 96.1 |

Table 6. Results of the binding of the trispecific antibodies to Jurkat cells

| Molecule | Jurkat | |
|---|---|---|
| | $EC_{50}$ (nM) | Top MFI |
| Trispecific antibody 1 | / | 13300 |
| BGCB491 | / | 9516 |
| TS-F2-5 | / | 7438 |
| hIgG1 LALA isotype | / | / |

## Example 8. Species cross-reactivity test of the anti-GPRC5D-BCMA-CD3 trispecific antibodies

The FACS was used to detect the binding of anti-GPRC5D-BCMA-CD3 trispecific antibodies to Cyno GPRC5D-expressing cynomolgus monkey GPRC5D-CHOK1 cells, Cyno BCMA-expressing cynomolgus monkey BCMA-CHOK1 cells, and naturally Cyno CD3-expressing Cyno PBMCs.

The cynomolgus monkey GPRC5D-CHOK1 (Example 2) and cynomolgus monkey BCMA-CHOK1 cells (Example 4) were cultured in the medium of F12K + 10% FBS + 400 $\mu$g/ml Hygromycin B in a 37°C, 5% $CO_2$ incubator using T75 cell culture flasks. When cells were needed, the cynomolgus monkey GPRC5D-CHOK1 and cynomolgus monkey BCMA-CHOK1 cells were digested with 0.25% Trypsin-EDTA, and digestion was terminated with F12K + 10% FBS + 400 $\mu$g/ml Hygromycin B.

The resulting cells were centrifuged at 1000 rpm for 5 minutes at room temperature, the supernatant was discarded, and the cells were then resuspended in 1% BSA (in PBS). The purchased Cyno PBMCs were centrifuged at 1500 rpm for 10 minutes at room temperature, the supernatant was discarded, and the cells were then resuspended in 1% BSA (in PBS). The cells were counted, and the cell density was adjusted to 1E6/mL. The cells were then plated into 96-well round bottom culture plates (corning, Product Number 3799) at 100 $\mu$L/well. The cells were centrifuged at 1500 rpm for 5 minutes at 4°C, the supernatant was discarded, and the cells were then resuspended in 200 $\mu$L of 1% BSA (in PBS). The cells were centrifuged again at 1500 rpm for 5 minutes at 4°C, the supernatant was discarded, and the cells were kept at 4°C for future use. The antibodies to be tested and the negative control hIgG1 LALA isotype (Biointron, Catalog: B109802) were diluted in 1% BSA (in PBS) with a starting concentration of 100 nM, and then serially diluted 5-fold to obtain 8 concentrations. The cells were resuspended in diluted antibodies (100 $\mu$L/well) and incubated at 4°C for 1 hour. The cells were centrifuged at 1500 rpm for 5 minutes at 4°C, and the supernatant was discarded. The cells were resuspended and washed in 160 $\mu$L of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at a 1:400 ratio according to the instructions, and the cells were resuspended in the diluted secondary antibody (100 $\mu$L/well) and incubated at 4°C for 0.5 hour. The cells were centrifuged at 1500 rpm for 5 minutes at 4°C, and the supernatant was discarded. The cells were resuspended and washed in 200 $\mu$L of 1% BSA (in PBS), centrifuged at 1500 rpm for 5 minutes at 4°C, and the supernatant was discarded. The cells were resuspended in 100 $\mu$L of 1% BSA (in PBS), filtered through 300-mesh gauze, and the mean fluorescence intensity in the PE channel was measured by a flow cytometer.

FCS files were exported from the flow cytometer. The mean fluorescence intensity (referred to as MFI below) of the PE channels of each sample was analyzed using FlowJo software. The mean fluorescence intensities as obtained were then imported into GraphPad to determine the half binding concentration (referred to as $EC_{50}$ below) of the antibodies to the cells and the top mean fluorescence intensity (Top MFI).

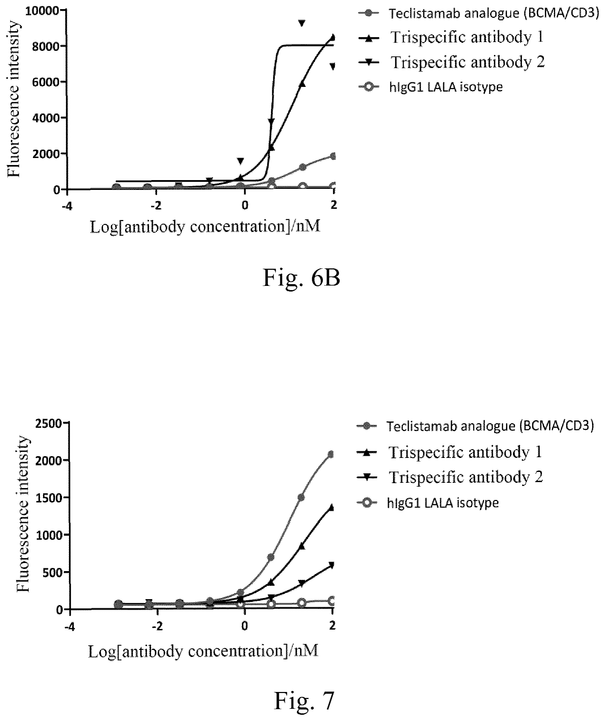

The results of the binding of the test molecules to the corresponding tumor antigen cells of cynomolgus monkeys are shown in FIGs. 6A-6B and Table 7. The trispecific antibody molecules showed good binding to the corresponding tumor antigen of the cynomolgus monkey.

The results of the binding of the test molecules to cynomolgus monkey PBMCs are shown in FIG. 7 and Table 8. The trispecific antibody molecules showed good binding to the cynomolgus monkey PBMCs.

Table 7. Results of the binding of the trispecific antibodies to the cells expressing cynomolgus monkey tumor antigens

| Molecule | cyno BCMA CHOK1 | | cyno GPRC5D CHOK1 | |
|---|---|---|---|---|
| | $EC_{50}$ (nM) | Top MFI | $EC_{50}$ (nM) | Top MFI |
| Teclistamab analogue (BCMA/CD3) | 15.34 | 1767 | / | / |
| Trispecific antibody 1 | 12.49 | 8413 | 4.59 | 13400 |
| Trispecific antibody 2 | 4.13 | 9160 | / | 11100 |
| hIgG1 LALA isotype | / | / | / | / |

Table 8. Results of the binding of the trispecific antibodies to cyno PBMCs

| Molecule | cyno PBMC | |
|---|---|---|
| | $EC_{50}$ (nM) | Top MFI |
| Teclistamab analogue (BCMA/CD3) | 55.82 | 2059 |
| Trispecific antibody 1 | 117.8 | 1352 |
| Trispecific antibody 2 | 184.5 | 568 |
| hIgG1 LALA isotype | / | 93.3 |

### Example 9. Binding affinity and kinetics of the anti-GPRC5D-BCMA-CD3 trispecific antibodies to recombinant proteins *in vitro*

To determine the affinity and kinetic properties of the antibodies to human CD3 (purchased from Acro, Cat. No. CDD-H52W1)/cynomolgus monkey CD3 (purchased from Acro, Cat. No. CDD-C52W4), a method of direct immobilization of human/cynomolgus monkey CD3 molecules on CM5 chips was employed. The CM5 chips were first activated with EDC and NHS. The chips were immobilized with Human/cynomolgus CD3 molecules diluted to 1 $\mu$g/mL with the acetate solution at pH 5 at a flow rate of 10 $\mu$L/min for 60 s, and then blocked with ethanolamine. The anti-GPRC5D-BCMA-CD3 trispecific antibody molecules were diluted in HBS-EP+ (10 mM HEPES, pH 7.4, 150 mM NaCl, 3 mM EDTA, 0.05% P20) buffer to a series of concentrations (100 nM-0.39 nM) using two-fold dilutions, bound at a flow rate of 50 $\mu$L/min for 90 s, followed by 360 s of dissociation.

At the end of each run, the chips were regenerated by rinsing with a 3 M $MgCl_2$ solution at a flow rate of 30 $\mu$L/min for 30 s to remove the anti-GPRC5D-BCMA-CD3 trispecific antibody molecules. The raw data were analyzed using Biacore Insight Evaluation Software (3.0.12.15655) and fitted to a (1:1) Langmuir model. The resulting affinity and kinetic experimental data of the trispecific antibodies are shown in Table 9.

Table 9. Binding affinity and kinetics of the anti-GPRC5D-BCMA-CD3 trispecific antibodies to human/cynomolgus monkey CD3 proteins

| Molecule | Antigen | Association rate $k_a$(1/M*s) | Dissociation rate $k_d$ (1/s) | Affinity $K_D$(M) |
|---|---|---|---|---|
| Talquetamab analogue (GPRC5D/CD3) | Human CD3 | 1.12E+05 | 2.01E-03 | 1.80E-08 |
| Teclistamab analogue (BCMA/CD3) | | 2.48E+05 | 5.22E-03 | 2.10E-08 |
| Trispecific antibody 1 | | 1.81E+05 | 3.95E-03 | 2.18E-08 |
| Trispecific antibody 2 | | 2.12E+05 | 4.28E-03 | 2.02E-08 |
| Talquetamab analogue (GPRC5D/CD3) | Cynomolgus monkey CD3 | 1.70E+05 | 3.79E-03 | 2.22E-08 |
| Teclistamab analogue (BCMA/CD3) | | 1.59E+05 | 3.36E-03 | 2.11E-08 |
| Trispecific antibody 1 | | 1.57E+05 | 3.29E-03 | 2.10E-08 |
| Trispecific antibody 2 | | 1.49E+05 | 2.90E-03 | 1.95E-08 |

The experimental results showed that both trispecific antibody molecules bind to the human/cynomolgus monkey CD3 with comparable affinity.

To determine the affinity and kinetic properties of the antibodies to human BCMA (purchased from Acro, Cat. No. BCA-H522y)/cynomolgus monkey BCMA (purchased from Acro, Cat. No. BCA-C52H7), the affinity and kinetic properties of antibody molecules and human/cynomolgus monkey BCMA were measured using an indirect capture method with CM5 chips. The CM5 chips were first activated with EDC and NHS. The chips were immobilized with anti-human IgG (Fc) antibody (purchased from cytiva, Cat. No. 10325009) diluted to 1 $\mu$g/mL with sodium acetate solution at pH 5 10 nM at a flow rate of 5 $\mu$L/min for 420 s, and then blocked with ethanolamine. The anti-GPRC5D-BCMA-CD3 trispecific antibody molecules were diluted in HBS-EP+ (10 mM HEPES, pH 7.4, 150 mM NaCl, 3 mM EDTA, 0.05% P20) buffer to 4 $\mu$g/mL and captured at a flow rate of 10 $\mu$L/min for 60 s. Human/cynomolgus monkey BCMA were diluted in HBS-EP+ (10 mM HEPES, pH 7.4, 150 mM NaCl, 3 mM EDTA, 0.05% P20) buffer to a series of concentrations (50 nM-0.195 nM) using two-fold dilutions, bound at a flow rate of 30 $\mu$L/min for 90 s, followed by 180 s of dissociation.

At the end of each run, the chips were regenerated by rinsing with a 3 M $MgCl_2$ solution at a flow rate of 30 $\mu$L/min for 30 s to remove the anti-GPRC5D-BCMA-CD3 trispecific antibody molecules. The raw data were analyzed using Biacore Insight Evaluation Software (3.0.12.15655) and fitted to a (1:1) Langmuir model. The resulting affinity and kinetic experimental data of the trispecific antibodies are shown in Table 10.

Table 10. Binding affinity and kinetics of the anti-GPRC5D-BCMA-CD3 trispecific antibodies to human/cynomolgus monkey BCMA proteins

| Molecule | Antigen | Association rate $k_a$ (1/M*s) | Dissociation rate $k_d$ (1/s) | Affinity $K_D$(M) |
|---|---|---|---|---|
| Teclistamab analogue (BCMA/CD3) | Human BCMA | 3.55E+06 | 1.35E-03 | 3.79E-10 |
| Trispecific antibody 1 | | 2.17E+06 | 5.01E-03 | 2.31E-09 |
| Trispecific antibody 2 | | 2.08E+06 | 5.17E-03 | 2.48E-09 |
| Teclistamab analogue (BCMA/CD3) | Cynomolgus monkey BCMA | 2.77E+06 | 2.35E-02 | 8.49E-09 |
| Trispecific antibody 1 | | 1.23E+06 | 5.08E-03 | 4.14E-09 |
| Trispecific antibody 2 | | 1.26E+06 | 5.43E-03 | 4.30E-09 |

The experimental results showed that both trispecific antibody molecules bind to human/cynomolgus monkey BCMA with comparable affinity.

## Example 10. *In vitro* functional assays of the anti-GPRC5D-BCMA-CD3 trispecific antibodies

A. T-cell-dependent cellular cytotoxicity (TDCC) assay using NCI-H929 as target **cells**

NCI-H929 cells (ATCC, Accession Number CRL-9068) were cultured in the medium of RPMI1640 + 10% FBS + 0.05 mM mercaptoethanol in a 37°C, 5% $CO_2$ incubator using T75 cell culture flasks. When cells were needed, NCI-H929 cells were placed directly in a 50 mL centrifuge tube without digestion. The cells were centrifuged at 1000 rpm for 5 minutes, and the supernatant was discarded. The cells were then resuspended in RPMI1640 + 2% FBS medium and counted, and the cell density was adjusted to 1E5/mL.

The target cells were plated into flat bottom 96-well plates (corning, Product Number 3599) at 100 $\mu$L/well and incubated overnight at 37°C, 5% $CO_2$. CD3+ T cells were sorted from fresh PBMCs using a T-cell negative isolation kit (StemCell, Cat. No. 17951). The cells were counted, and the cell density was adjusted to 2E6/mL using RPMI1640 + 2% FBS. Effector cells were plated into 96-well plates at 50 $\mu$L/well and incubated at 37°C, 5% $CO_2$.

The antibodies were diluted in RPMI1640 + 2% FBS medium with a starting concentration of 100 nM and then serially diluted 14-fold to obtain 10 concentrations. The diluted antibodies were added to the cell culture plates at 50 $\mu$L/well, and the cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH the detection reagent (Romos, Cat. No. 4744934001) was added to each well and mixed. The plate was centrifuged at 1000 rpm for 5 minutes, followed by a 10-minute dark incubation, and OD492 values were read using the Envision.

The percentage of cell killing caused by the TDCC effect was calculated using the following equation:

Cell killing %= [(sample wells - wells of spontaneous release of T cells - wells of spontaneous release of target cells)/ (wells of maximum lysis of target cells - wells of spontaneous release of target cells)]*100%.

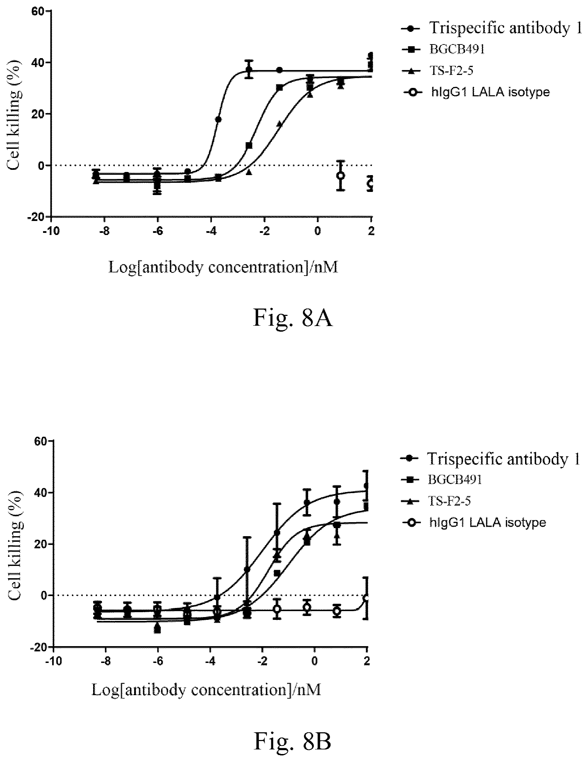

Cell killing data were imported into GraphPad Prism, and cell killing/concentration curves were plotted to calculate $EC_{50}$ values. The results are shown in Table 11 and FIG. 8A. The cytotoxicity of the trispecific antibody 1 against NCI-H929 cells was stronger than that of BGCB491 and TS-F2-5.

B. T-cell-dependent cellular cytotoxicity (TDCC) assay using RPMI8226 as target **cells**

RPMI8226 cells (ATCC, Accession Number CCL-155) were cultured in RPMI1640 + 10% FBS medium in a 37°C, 5% $CO_2$ incubator using T75 cell culture flasks. When cells were needed, RPMI8226 cells were placed directly in a 50 mL centrifuge tube without digestion. The cells were centrifuged at 1000 rpm for 5 minutes, and the supernatant was discarded. The cells were then resuspended in RPMI1640 + 2% FBS medium and counted, and the cell density was adjusted to 1E5/mL.

Target cells were plated into flat bottom 96-well plates (corning, Product Number 3599) at 100 $\mu$L/well and incubated overnight at 37°C, 5% $CO_2$. CD3+ T cells were sorted from fresh PBMCs using a T-cell negative isolation kit (StemCell, Cat. No.17951). The cells were counted, and the cell density was adjusted to 2E6/mL using RPMI1640 + 2% FBS. Effector cells were plated into 96-well plates at 50 $\mu$L/well and incubated at 37°C, 5% $CO_2$.

The antibodies were diluted in RPMI1640 + 2% FBS medium with a starting concentration of 100 nM and then serially diluted 14-fold to obtain 10 concentrations. The diluted antibodies were added to the cell culture plates at 50 $\mu$L/well, and the cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH detection reagent (Romos, Cat. No. 4744934001) was added to each well and mixed. The plate was centrifuged at 1000 rpm for 5 minutes, followed by a 10-minute dark incubation, and OD492 values were read using the Envision.

The percentage of cell killing caused by the TDCC effect was calculated using the following equation:

Cell killing % = [(sample wells - wells of spontaneous release of T cells - wells of spontaneous release of target cells)/ (wells of maximum lysis of target cells - wells of spontaneous release of target cells)]*100%.

Cell killing data were introduced into GraphPad Prism, and cell killing/concentration curves were plotted to calculate $EC_{50}$ values. The results are shown in Table 11 and FIG. 8B. The cytotoxicity of the trispecific antibody1 against RPMI8226 cells was stronger than that of BGCB491 and TS-F2-5.

C. T-cell-dependent cellular cytotoxicity (TDCC) assay using human BCMA-CHOK1 as target cells

Human BCMA-CHOK1 (Example 4) cells were cultured in the medium of F12K + 10% FBS + 400 $\mu$g/ml Hygromycin B in a 37°C, 5% $CO_2$ incubator using T75 cell culture flasks. When cells were needed, Human BCMA-CHOK1 cells were digested with 0.25% Trypsin-EDTA, and the digestion was terminated with the medium of F12K + 10% FBS + 400 $\mu$g/ml Hygromycin B. The cells were centrifuged at 1000 rpm for 5 minutes, and the supernatant was discarded. The cells were then resuspended in RPMI1640 + 2% FBS medium and counted, and the cell density was adjusted to 1E5/mL.
Target cells were plated into flat bottom 96-well plates (corning, Product Number 3599) at 100 $\mu$L/well and incubated overnight at 37°C, 5% $CO_2$. CD3+ T cells were sorted from fresh PBMCs using a T-cell negative isolation kit (StemCell, Cat. No.17951). The cells were counted, and the cell density was adjusted to 2E6/mL using RPMI1640 + 2% FBS. Effector cells were plated into 96-well plates at 50 $\mu$L/well and incubated at 37°C, 5% $CO_2$.
The antibodies were diluted in RPMI1640 + 2% FBS medium with a starting concentration of 400 nM and then serially diluted 10-fold. The diluted antibodies were added to the cell culture plates at 50 $\mu$L/well, resulting in a final starting concentration of 100 nM. The cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH detection reagent (Romos, Cat. No. 4744934001) was added to each well and mixed. The plate was centrifuged at 1000 rpm for 5 minutes, followed by a 10-minute dark incubation, and OD492 values were read using the Envision.
The percentage of cell killing caused by the TDCC effect was calculated using the following equation:

Cell killing %= [(sample wells - wells of spontaneous release of T cells - wells of spontaneous release of target cells)/ (wells of maximum lysis of target cells - wells of spontaneous release of target cells)]*100%.

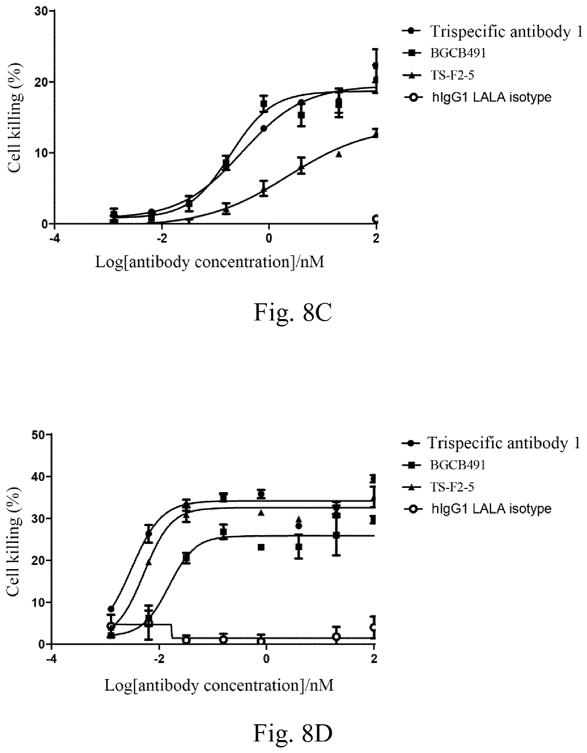

Cell killing data were imported into GraphPad Prism, and cell killing/concentration curves were plotted to calculate $EC_{50}$ values. The results are shown in Table 11 and FIG. 8C. The cytotoxicity of the trispecific antibody 1 against Human BCMA-CHOK1 cells was comparable to that of BGCB491 and stronger than that of TS-F2-5.

D. T-cell-dependent cellular cytotoxicity (TDCC) assay using human GPRC5D-CHOK1 as target cells

Human GPRC5D-CHOK1 (Example 2) cells were cultured in the medium of F12K + 10% FBS + 400 $\mu$g/ml Hygromycin B in a 37°C, 5% $CO_2$ incubator using T75 cell culture flasks. When cells were needed, Human GPRC5D-CHOK1 cells were digested with 0.25% Trypsin-EDTA, and the digestion was terminated with the medium of F12K + 10% FBS + 400 $\mu$g/ml Hygromycin B. The cells were centrifuged at 1000 rpm for 5 minutes, and the supernatant was discarded. The cells were then resuspended in RPMI1640 + 2% FBS medium and counted, and the cell density was adjusted to 1E5/mL.
Target cells were plated into flat bottom 96-well plates (corning, Product Number 3599) at 100 $\mu$L/well and incubated overnight at 37°C, 5% $CO_2$. CD3+ T cells were sorted from fresh PBMCs using a T-cell negative isolation kit (StemCell, Cat. No.17951). The cells were counted, and the cell density was adjusted to 2E6/mL using RPMI1640 + 2% FBS. Effector cells were plated into 96-well plates at 50 $\mu$L/well and incubated at 37°C, 5% $CO_2$.
The antibodies were diluted in RPMI1640 + 2% FBS medium with a starting concentration of 100 nM and then serially diluted 5-fold to obtain 8 concentrations. The diluted antibodies were added to the cell culture plates at 50 $\mu$L/well, and the cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH detection reagent (Romos, Cat. No. 4744934001) was added to each well and mixed. The plate was centrifuged at 1000 rpm for 5 minutes, followed by a 10-minute dark incubation, and OD492 values were read using the Envision.

The percentage of cell killing caused by the TDCC effect was calculated using the following equation:

Cell killing %= [(sample wells -wells of spontaneous release of T cells - wells of spontaneous release of target cells)/ (wells of maximum lysis of target cells - wells of spontaneous release of target cells)]*100%.

Cell killing data were imported into GraphPad Prism, and cell killing/concentration curves were plotted to calculate $EC_{50}$ values. The results are shown in Table 11 and FIG. 8D. The cytotoxicity of the trispecific antibody 1 against Human GPRC5D-CHOK1 cells was comparable to that of TS-F2-5 and stronger than that of BGCB491.

E. T-cell-dependent cellular cytotoxicity (TDCC) assay using a mixture of human BCMA-CHOK1 and human GPRC5D-CHOK1 as target **cells**

Human GPRC5D-CHOK1 and Human BCMA-CHOK1 cells were cultured in the medium of F12K + 10% FBS + 400 $\mu$g/ml Hygromycin B in a 37°C, 5% $CO_2$ incubator using T75 cell culture flasks. When the cells were needed, Human GPRC5D-CHOK1 and Human BCMA-CHOK1 cells were digested with 0.25% Trypsin-EDTA, and the digestion was terminated with the medium of F12K + 10% FBS + 400 $\mu$g/ml Hygromycin B. The cells were centrifuged at 1000 rpm for 5 minutes, and the supernatant was discarded. The cells were then resuspended in RPMI1640 + 2% FBS medium and counted, and the cell density was adjusted to 1E5/mL. The two cells were then mixed in equal.

The target cell mixture were plated into flat bottom 96-well plates (corning, Product Number 3599) at 100 $\mu$L/well and incubated overnight at 37°C, 5% $CO_2$. CD3+ T cells were sorted from fresh PBMCs using a T-cell negative isolation kit (StemCell, Cat. No.17951). The cells were counted, and the cell density was adjusted to 1.6E6/mL using RPMI1640 + 2% FBS. Effector cells were plated into 96-well plates at 50 $\mu$L/well and incubated at 37°C, 5% $CO_2$.

The antibodies were diluted in RPMI1640 + 2% FBS medium with a starting concentration of 100 nM and then serially diluted 14-fold to obtain 10 concentrations. The diluted antibodies were added to the cell culture plates at 50 $\mu$L/well, and the cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH detection reagent (Romos, Cat. No. 4744934001) was added to each well and mixed. The plate was centrifuged at 1000 rpm for 5 minutes, followed by a 10-minute dark incubation, and OD492 values were read using the Envision.

The percentage of cell killing caused by the TDCC effect was calculated using the following equation:

Cell killing %= [(sample wells -wells of spontaneous release of T cells - wells of spontaneous release of target cells)/ (wells of maximum lysis of target cells - wells of spontaneous release of target cells)]*100%.

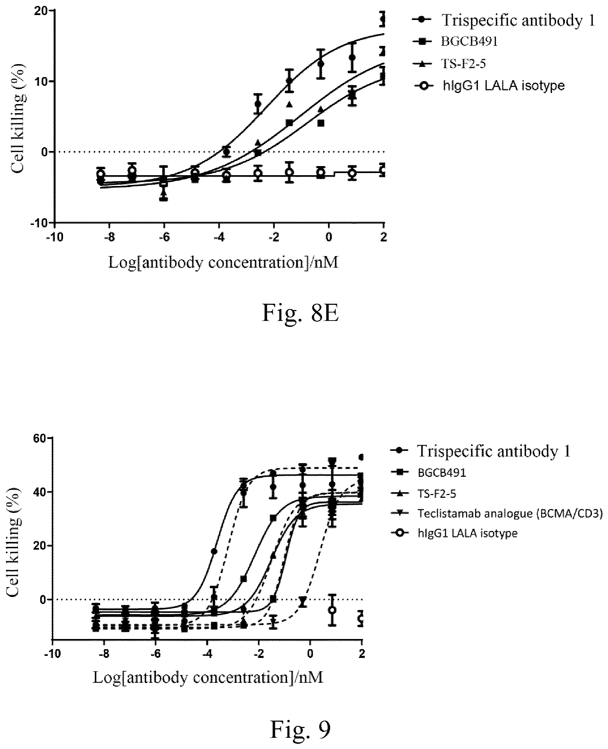

Cell killing data were imported into GraphPad Prism, and cell killing/concentration curves were plotted to calculate $EC_{50}$ values. The results are shown in Table 11 and FIG. 8E. The cytotoxicity of the trispecific antibody 1 against the mixture was stronger than that of BGCB491 and TS-F2-5.

Table 11. T-cell-dependent cellular cytotoxicity (TDCC) assay using NCI-H929, RPMI8226, human BCMA-CHOK1, human GPRC5D-CHOK1, and a mixture of human BCMA-CHOK1 and human GPRC5D-CHOK1 as target cells

| Molecule | NCI-H929 | | RPMI8226 | | BCMA-CHOK1 | | GPRC5D-CHOK1 | | BCMA-CHOK1 + GPRC5D-CHOK1 mixture | |
|---|---|---|---|---|---|---|---|---|---|---|
| | $EC_{50}$ (nM) | Cytotoxicity (max) | $EC_{50}$ (nM) | Cytotoxicity (max) | $EC_{50}$ (nM) | Cytotoxicity (max) | $EC_{50}$ (nM) | Cytotoxicity (max) | $EC_{50}$ (nM) | Cytotoxicity (max) |
| Trispecific antibody 1 | 0.0002 | 49.321 | 0.010 | 42.720 | 0.316 | 22.348 | 0.003 | 40.369 | 0.005 | 18.800 |
| BGCB491 | 0.005 | 45.098 | 0.101 | 34.961 | 0.192 | 24.621 | 0.015 | 30.905 | 0.163 | 10.759 |
| TS-F2-5 | 0.035 | 43.137 | 0.017 | 34.089 | 2.267 | 13.384 | 0.005 | 37.605 | 0.091 | 14.270 |

EP 4 663 662 A1

F. T-cell-dependent cellular cytotoxicity (TDCC) assay using NCI-H929 as target cells in the presence of 2500 ng/mL soluble BCMA

NCI-H929 cells (ATCC, Accession Number CRL-9068) were cultured in the medium of RPMI1640 + 10% FBS + 0.05 mM mercaptoethanol in a 37°C, 5% $CO_2$ incubator using T75 cell culture flasks. When cells were needed, NCI-H929 cells were placed directly in a 50 mL centrifuge tube without digestion. The cells were centrifuged at 1000 rpm for 5 minutes, and the supernatant was discarded. The cells were then resuspended in RPMI1640 + 2% FBS medium and counted, and the cell density was adjusted to 1E5/mL. Soluble BCMA was added to the cell suspension to a concentration of 2500 ng/ml.

Target cells containing soluble BCMA were plated into flat bottom 96-well plates (corning, Product Number 3599) at 100 $\mu$L/well, and incubated overnight at 37°C, 5% $CO_2$. CD3+ T cells were sorted from fresh PBMCs using a T-cell negative isolation kit (StemCell, Cat. No.17951). The cells were counted, and the cell density was adjusted to 2E6/mL using RPMI1640 + 2% FBS. Effector cells were plated into 96-well plates at 50 $\mu$L/well and incubated at 37°C, 5% $CO_2$.

The antibodies were diluted in RPMI1640 + 2% FBS medium with a starting concentration of 100 nM and then serially diluted 14-fold. The diluted antibodies were added to the cell culture plates at 50 $\mu$L/well, resulting in a final starting concentration of 100 nM. The cells were incubated at 37°C, 5% $CO_2$ for 24 hours. The culture plates were centrifuged at 1000 rpm for 5 minutes. 50 $\mu$L of the supernatant was transferred into another flat bottom 96-well plate, and 50 $\mu$L of LDH detection reagent (Romos, Cat. No. 4744934001) was added to each well and mixed. The plate was centrifuged at 1000 rpm for 5 minutes, followed by a 10-minute dark incubation, and OD492 values were read using the Envision.

The percentage of cell killing caused by the TDCC effect was calculated using the following equation:

Cell killing %= [(sample wells -wells of spontaneous release of T cells - wells of spontaneous release of target cells)/ (wells of maximum lysis of target cells - wells of spontaneous release of target cells)]*100%.

Cell killing data were imported into GraphPad Prism, and cell killing/concentration curves were plotted to calculate EC50 values. The results are shown in FIG. 9 (dashed lines indicate the condition of 2500 ng/mL soluble BCMA) and Table 12. In the presence of 2500 ng/ml soluble BCMA, the $EC_{50}$ value of BGCB491 cytotoxicity was reduced by about 23-fold, the $EC_{50}$ value of TS-F2-5 cytotoxicity was generally maintained, and the $EC_{50}$ value of the trispecific antibody 1 cytotoxicity was reduced by about 3-fold. (The EC50 value of the cytotoxicity of the BCMA bispecific antibody Teclistamab was decreased by about 25-fold.)

Table 12. T-cell-dependent cellular cytotoxicity assay in the presence of 2500 ng/ml soluble BCMA

| Molecule | 0 ug/ml BCMA | | 2.5 ug/ml BCMA | | Ratio ($EC_{50}$) |
|---|---|---|---|---|---|
| | $EC_{50}$ (nM) | Cytotoxicity (max) | $EC_{50}$ (nM) | Cytotoxicity (max) | |
| Trispecific antibody 1 | 0.0002 | 49.95 | 0.0007 | 58.61 | ~3 |
| BGCB491 | 0.007 | 44.25 | 0.16 | 50.05 | ~23 |
| TS-F2-5 | 0.031 | 41.62 | 0.03 | 50.75 | ~1 |
| Teclistamab (BCMA/CD3) | 0.115 | 40.86 | 2.36 | 53.85 | ~21 |
| hIgG1 LALA isotype | / | / | / | / | / |

G. IL 6 release levels under the co-incubation condition of PBMCs, NCI-H929 tumor cells, and the trispecific antibodies

NCI-H929 cells (ATCC, Accession Number CRL-9068) were incubated in the medium of RPMI1640 + 10% FBS + 0.05 mM mercaptoethanol in a 37°C, 5% $CO_2$ incubator using T75 cell culture flasks. When the cells were needed, NCI-H929 cells were placed directly in a 50 mL centrifuge tube without digestion. The cells were centrifuged at 1000 rpm for 5 minutes, and the supernatant was discarded. The cells were then resuspended in RPMI1640 + 2% FBS medium and counted, and the cell density was adjusted to 1E5/mL. NCI-H929 cells were plated into 96-well plates (corning, Product Number 3799) at 100 $\mu$L/well, and incubated in 37°C, 5%$CO_2$.

Fresh PBMCs were purchased and counted, and the cell density was adjusted to 4E6/mL with RPMI1640 + 2% FBS. Effector cells were plated into 96-well plates at 50 $\mu$L/well and incubated at 37°C, 5% $CO_2$.

The antibodies were diluted in RPMI1640 + 2% FBS medium with a starting concentration of 100 nM and then serially diluted 13-fold. The diluted antibodies were added to the cell culture plates at 50 $\mu$L/well, resulting in a final starting concentration of 100 nM. The cells were incubated at 37°C, 5% $CO_2$ for 24 hours.

The plates were centrifuged at 400 g for 10 minutes. 100 $\mu$L of the supernatant was frozen at -80°C for later use.

The standard was diluted two-fold according to the instructions of the cytokine assay kit (Biolegend, 430504 (ELISA MAX Deluxe Set Human IL-6)) with a maximum concentration of 8000 pg/mL and a minimum concentration of 0 pg/mL for a total of 12 concentration points. The prepared standards or thawed samples were added to 96-well plates at 100 μL/well (with additional dilution was required for each sample), and the OD450 values were read on Envision following the kit protocol

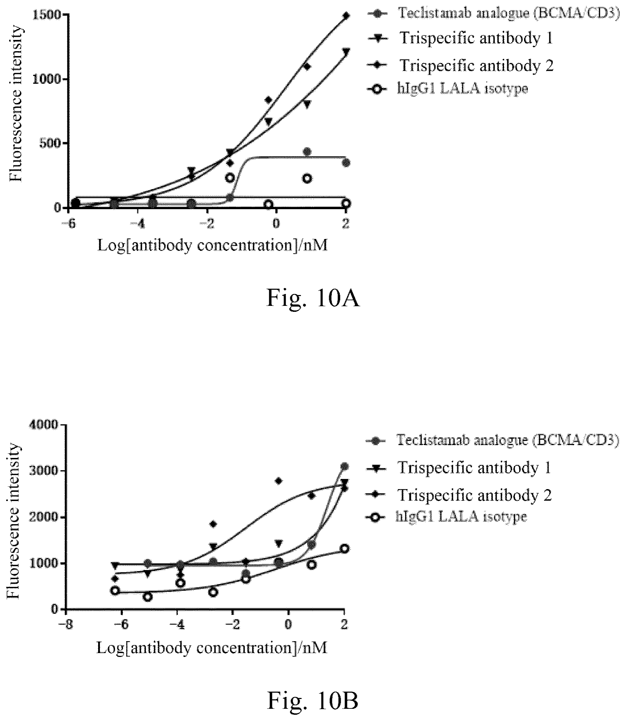

The results are shown in FIGs. 10A-10B. In the co-incubation condition of PBMCs and tumor cells, the levels of IL6 cytokine release induced by the trispecific antibody 1 were weaker than those induced by the trispecific antibody 2.

## Example 11. Stability test of the anti-GPRC5D-BCMA-CD3 trispecific antibodies

The thermal stability of the trispecific antibodies in a PBS buffer at pH 7.4 was detected by differential fluorescence scanning technology. The sample concentration was about 1 mg/mL and the detection was performed using Prometheus NT.Plex (nano DSF). Before the detection, each sample was centrifuged at 10000 g for 10 minutes. 40 μL of the sample was added to each well of the sample plate (the loading amount was 10 μL, with each sample having a replicate well). The scanning temperature started at 30°C and ended at 95°C, with a scanning rate of 0.5°C/min. The experimental results are shown in Table 13. Both trispecific antibody molecules demonstrated good thermal stability.

Table 13. NanoDSF detection results of anti-GPRC5D-BCMA-CD3 trispecific antibodies

| Molecule | Tm Onset | Tml | Tagg |
|---|---|---|---|
| Trispecific antibody 1 | 57.8°C | 61.4°C | 65.2°C |
| Trispecific antibody 2 | 57.3°C | 61.9°C | 65.6°C |

## Example 12. Pharmacokinetics of the anti-GPRC5D-BCMA-CD3 trispecific antibodies

Two naive cynomolgus monkeys were used in the experiment with free access to water. The trispecific antibodies were administered at a dose of 1 mg/kg via intravenous injection to the hind limbs. Blood samples were collected at the following time points: pre-dose, 5 min, 2 hr, 6 hr, 24 hr, 72 hr, 168 hr, 264 hr, 336 hr, 504 hr, 672 hr, and 840 hr. Whole blood samples were collected in polyethylene tubes without anticoagulants, left at room temperature for about 1 hour, centrifuged at 6000 g, 25°C, immediately placed on dry ice, and transferred to a -80°C refrigerator for long-term storage.

The concentration of intact anti-GPRC5D-BCMA-CD3 trispecific antibody molecules in serum was determined by ELISA. 96-well plates were coated with human GPRC5D protein at a concentration of 2 μg/mL, 100 μL per well, and placed overnight at 4°C; 300 μL/well PBST was used to wash the plates 3 times, followed by the addition of 300 μL of blocking reagent containing 5% milk powder, and the plates were incubated at 37°C for 1 h; 300 μL/well PBST was used to wash the plates 3 times, followed by the addition of 100 μL of the samples to be tested, and the plates were incubated at 37°C for 1 h; 300 μL/well PBST was used to wash the plates 6 times, followed by the addition of 100 μL of Biotin-BCMA reagent, and the plates were incubated at 37°C for 1 h; 300 μL/well PBST was used to wash the plates 6 times, followed by the addition of 100 μL of the SA-HRP reagent, and the plates were incubated at 37°C for 1 h; 300 μL/well PBST was used to wash the plates 6 times, followed by the addition of 100 μL of TMB, and after 10 min of standing in the dark, 50 μL of the stop solution was added to terminate the color reaction. The concentration of the intact anti-GPRC5D-BCMA-CD3 trispecific antibody molecules was determined quantitatively based on the color reaction.

Absorbance at the 450 nm wavelength was detected using an Envision plate reader from PE Corporation and the data were processed using softmax software.

Serum concentrations of the trispecific antibodies in monkeys were calculated using Phoenix Winnolin 8.2 software.

The results showed that the trispecific antibody 1 exhibited favorable pharmacokinetic properties in monkeys, consistent with the typical metabolic characteristics of macromolecules.

## Example 13. *In vivo* pharmacodynamic study of the anti-GPRC5D-BCMA-CD3 trispecific antibodies

A. Human immune cell-reconstituted mouse model with NCI-H929 as tumor cells

All experimental animals were housed in individually ventilated cages at a constant temperature and humidity, with the temperature of 20.0-26.0°C, humidity of 40-70%, and a light/dark cycle of 12h/12h.

NCI-H929 cells (ATCC, Accession Number CRL-9068) were cultured in RPMI 1640 medium containing 10% fetal bovine serum and 0.05 mM β-ME. NCI-H929 cells in the exponential growth phase were harvested and resuspended in 0.1 mL of a suspension of PBS and Matrigel (1:1). $5\times10^6$ NCI-H929 cells were inoculated into the right upper flank of the back of each mouse, and tumor growth was monitored regularly. When tumors reached an average volume of 83 mm$^3$, mice were randomly grouped based on tumor size and body weight.

Donor PBMCs were purchased from AllCells, LLC. Each mouse was intraperitoneally injected with $1\times10^7$ PBMCs in 0.1 mL PBS on the same day as NCI-H929 cell inoculation, thereby establishing a human immune cell-reconstituted mouse model.

Before dosing, all the animals were weighted, and tumor volumes were measured using a vernier caliper. Since tumor volume may influence treatment efficacy, the mice were grouped based on tumor volume using a random grouping design to ensure similar tumor volumes across groups. Mice were grouped using the Matched Distribution method in StudyDirectorTM (version 3.1.399.19, Studylog System, Inc., South San Francisco, CA, USA). The day when mice were grouped was defined as day 0, and the mice were intraperitoneally administered on D0, once every three days for a total of 5 doses. The experimental groupings and dosing regimens are shown in Table 14.

Table 14. Experimental groupings and dosing regimens

| Group numberings | Number of mice per group | Molecule | Dose (mg/kg) | Administration route | Actual dosing frequency and cycle |
|---|---|---|---|---|---|
| 1 | 6 | hIgG1 LALA isotype | 0.004 | Intraperitoneal | Once every three days for a total of 5 doses |
| 2 | 6 | Teclistamab | 0.004 | Intraperitoneal | Once every three days for a total of 5 doses |
| 3 | 6 | Trispecific antibody 1 | 0.004 | Intraperitoneal | Once every three days for a total of 5 doses |
| 4 | 6 | Trispecific antibody 2 | 0.005 | Intraperitoneal | Once every three days for a total of 5 doses |
| 5 | 6 | Talquetamab and Teclistamab combination | 0.004 + 0.004 | Intraperitoneal | Once every three days for a total of 5 doses |

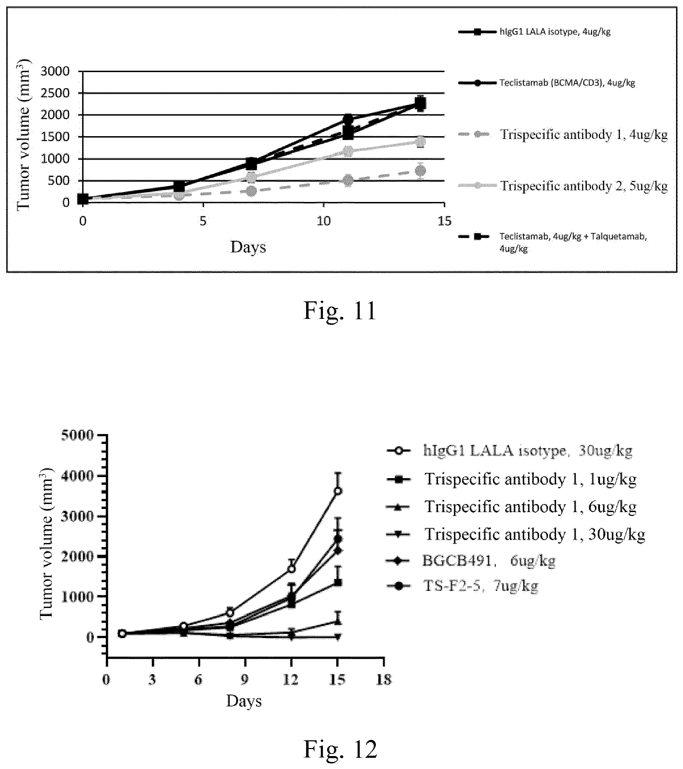

Daily behavioral performance of animals was monitored every day after dosing for a total of 14 days. Throughout the experiment, the length and width of tumors were measured twice a week using a vernier caliper, and tumor volume was calculated as: tumor volume (mm$^3$) = $0.5 \times$ (tumor length $\times$ tumor width$^2$). Relative tumor growth inhibition rate (TGI) (%): TGI% = (1 - T/C) $\times$ 100%. T/C% represents the relative tumor growth rate, i.e., the percentage of reletive tumor volumes or tumor weights of the treatment groups and the hIgG1 LALA isotype control group at a specific time point. T and C refer to the tumor volume (TV) or tumor weight (TW) at a specific time point in the treatment groups and the hIgG1 LALA isotype control group, respectively. The experimental results such as tumor volume, mouse body weight and tumor weight for each group were expressed as mean + standard error (Mean + SEM). An independent sample T-test was used to compare whether there were significant differences between the different treatment groups and the control group. Data were analyzed using SPSS. A P value of < 0.05 was considered statistically significant. The experimental results are shown in Table 15 and FIG. 11.

The treatment group with 0.004 mg/kg of the trispecific antibody 1 exhibited a highly significant tumor inhibitory effect in the Xenograft model of NPG female mice which were implanted subcutaneously with human-derived myeloma NCI-H929 (p value less than 0.001), and was significantly superior to the equimolar doses of the bispecific antibody Teclistamab and its combination with Talquetamab. Mice tolerated the trispecific antibody molecules as the test drugs well, with no significant body weight loss or toxicity observed.

Table 15. Tumor volume and tumor growth inhibition rate of each treatment group

| Group numberings | Molecule | Dose (mg/kg) | Average tumor volume (mm$^3$) | | T/C (%) | Tumor growth inhibition rate (TGI) (%) | P value |
|---|---|---|---|---|---|---|---|
| | | | Day 0 | Day 14 | | | |
| 1 | hIgG1 LALA isotype | 0.004 | 83.80 | 2256.68 | / | / | / |
| 2 | Teclistamab | 0.004 | 83.70 | 2262.02 | 102 | -2 | 1 |

(continued)

| Group numberings | Molecule | Dose (mg/kg) | Average tumor volume (mm$^3$) | | T/C (%) | Tumor growth inhibition rate (TGI) (%) | P value |
|---|---|---|---|---|---|---|---|
| | | | Day 0 | Day 14 | | | |
| 3 | Trispecific antibody 1 | 0.004 | 83.62 | 727.54 | 30 | 70 | <0.001 *** |
| 4 | Trispecific antibody 2 | 0.005 | 83.71 | 1387.11 | 60 | 40 | <0.001 *** |
| 5 | Talquetamab and Teclistamab combination | 0.004 | 83.75 | 2277.89 | 104 | -4 | 1 |

B. Human immune cell-reconstituted mouse model with NCI-H929 as tumor cells

All experimental animals were housed in individually ventilated cages at a constant temperature and humidity, with the temperature of 20.0-26.0°C, humidity of 40-70%, and a light/dark cycle of 12h/12h.

NCI-H929 cells (ATCC, Accession Number CRL-9068) were cultured in RPMI 1640 medium containing 10% fetal bovine serum and 0.05 mM β-ME. NCI-H929 cells in the exponential growth phase were harvested and resuspended in 0.1 mL of a suspension of PBS and Matrigel (1:1). $5 \times 10^6$ NCI-H929 cells were inoculated into the right upper flank of the back of each mouse, and tumor growth was monitored regularly. When tumors reached an average volume of 100 mm$^3$, mice were randomly grouped based on tumor size and body weight.

Donor PBMCs were purchased from AllCells, LLC. Each mouse was intraperitoneally injected with $1 \times 10^7$ PBMCs in 0.2 mL PBS on the same day as NCI-H929 cell inoculation, thereby establishing a human immune cell-reconstituted mouse model.

Before dosing, all the animals were weighted, and tumor volumes were measured using a vernier caliper. Since tumor volume may influence treatment efficacy, the mice were grouped based on tumor volume using a random grouping design to ensure similar tumor volumes across groups. The day when mice were grouped is defined as day 1, and the mice were intraperitoneally administered on D1, twice a week for a total of 4 doses. The experimental groupings and dosing regimens are shown in Table 16.

Table 16. Experimental groupings and dosing regimens

| Group numberings | Number of mice per group | Molecule | Dose (mg/kg) | Administration route | Actual dosing frequency and cycle |
|---|---|---|---|---|---|
| 1 | 7 | hIgG1 LALA isotype | 0.03 | Intraperitoneal | Twice a week for a total of 4 doses |
| 2 | 7 | Trispecific antibody 1 | 0.001 | Intraperitoneal | Twice a week for a total of 4 doses |
| 3 | 7 | Trispecific antibody 1 | 0.006 | Intraperitoneal | Twice a week for a total of 4 doses |
| 4 | 7 | Trispecific antibody 1 | 0.03 | Intraperitoneal | Twice a week for a total of 4 doses |
| 5 | 7 | BCGB491 | 0.006 | Intraperitoneal | Twice a week for a total of 4 doses |
| 6 | 7 | TS-F2-5 | 0.007 | Intraperitoneal | Twice a week for a total of 4 doses |

Daily behavioral performance of animals was monitored every day after dosing for a total of 15 days. Throughout the experiment, the length and width of tumors were measured twice a week using a vernier caliper, and tumor volume was calculated as: tumor volume (mm$^3$) = 0.5 × (tumor length × tumor width$^2$). Relative tumor growth inhibition rate (TGI) (%): TGI (%) = [1 - (Vti - Vt0) / (Vci - Vc0)] 100%. T/C% represents the relative tumor growth rate, i.e., the percentage of relative tumor volumes or tumor weights of the treatment groups and the hIgG1 LALA isotype control group at a specific time point.

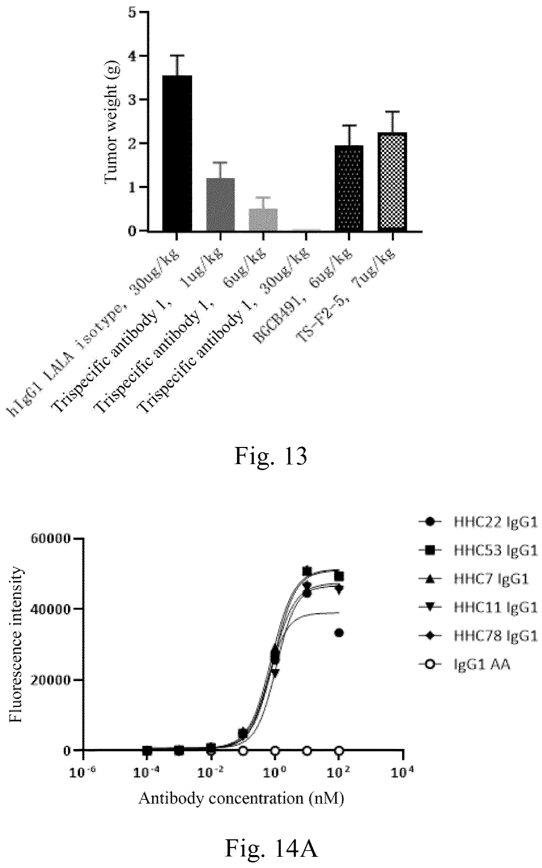

T and C refer to the tumor volume (TV) or tumor weight (TW) at a specific time point in the treatment groups and the hIgG1 LALA isotype control group, respectively. The experimental results such as tumor volume, mouse body weight and tumor weight for each group were expressed as mean $\pm$ standard error (Mean $\pm$ SEM). An independent sample T-test was used to compare whether there were significant differences between the different treatment groups and the control group. Data were analyzed using GraphPad prism 8.0.2. $P < 0.05$ was considered statistically significant. The experimental results are shown in Table 17 and FIGs. 12 and 13.

The treatment groups with different doses of the trispecific antibody 1 exhibited a highly significant tumor inhibitory effect in the Xenograft model of NPG female mice which were implanted subcutaneously with human-derived myeloma NCI-H929 (all the p values were less than 0.05). The 0.006 mg/kg of the trispecific antibody 1 was significantly superior to equimolar doses of BCGB491 and TS-F2-5. Mice tolerated the trispecific antibody molecules as the test drugs well, with no significant body weight loss or toxicity observed.

Table 17. Tumor volume and tumor growth inhibition rate of each treatment group

| Group numberings | Molecule | Dose (mg/kg) | Average tumor volume (mm³) | | T/C (%) | Tumor growth inhibition rate (TGI) (%) | P value |
|---|---|---|---|---|---|---|---|
| | | | Day 1 | Day 15 | | | |
| 1 | hIgG1 LALA isotype | 0.03 | 100 | 3623 | / | / | / |
| 2 | Trispecific antibody 1 | 0.001 | 100 | 1348 | 37 | 65 | 0.003** |
| 3 | Trispecific antibody 1 | 0.006 | 100 | 400 | 11 | 91 | <0.0001 *** |
| 4 | Trispecific antibody 1 | 0.03 | 100 | 5 | 0 | 103 | <0.0001 *** |
| 5 | BCGB491 | 0.006 | 100 | 2146 | 59 | 42 | 0.054 |
| 6 | TS-F2-5 | 0.007 | 100 | 2438 | 67 | 34 | 0.114 |

## Example 14. Llama immunization and construction of the heavy chain-only antibody immune library

The llamas were immunized with CHO-K1 cells expressing high levels of the human GPRC5D. The immunization were performed on day 0, day 21, day 35, day 49, and day 70, for a total of 5 rounds of immunization. Blood samples were drawn on day 28, day 42, and day 63 to isolate serum, and immune responses in the serum were detected by FACS at the cellular level. When the serum titer plateaued, immunization was terminated, and a 50 mL blood sample was drawn from the immunized llamas. The llama PBMCs were isolated using Solarbio lymphocyte separation medium, and the extracted total RNA (OMEGA total cellular RNA extraction kit) was reverse transcribed into cDNA as a template using the Takara PrimeScript™ II reverse transcription kit according to the manufacturer's instructions. The VHH fragments was amplified by nested PCR using the designed specific primers. The VHH fragments were recovered and ligated into the pADL-23c phagemid vector using Sfi I digestion. TG1 electrocompetent cells were electroporated to construct the GPRC5D llama immune library, with a library size of 4.07E9.

## Example 15. Screening of positive clones of the llama-derived anti-GPRC5D antibodies

To obtain positive antibodies that can cross-bind to the human GPRC5D and the cynomolgus monkey GPRC5D, the library described above was amplified and added to the M13K07 helper phage to assemble the phages. The human GPRC5D-CHOK1 cell line, the cynomolgus monkey GPRC5D-CHOK1 cell line, and the CHOK1 cells were cultured to confluence, washed twice with PBS, and then 100 μL of 4% paraformaldehyde was added to fix the cells, followed by treatment at 25°C for 20-30 min. After washed twice with PBS, 300 μL of 5% skim milk was added to each well and the cells were incubated at 37°C for 1 h for blocking. Then the plates were washed three times with PBST, followed by the addition of 50 μL of 5% skim milk and 50 μL of phage supernatant per well, and the cells were incubated at 37°C for 1 h. The plates were washed 5 times with 0.1% PBST, then the horseradish peroxidase-labeled anti-M13 antibody (diluted 1:10000 with PBS) was added to the wells at 100 μL/well and the cells were incubated at 37°C for 1 h. The plates were washed 6 times with 0.1% PBST. 100 μL of TMB chromogenic solution was added to each well and the cells were incubated at 37°C for 7 min. The reaction was stopped by adding 50 μL of stop solution per well, and optical density was measured at 450 nm The positive clones were sequenced to obtain the amino acid sequences of 5 heavy chain antibody variable regions (VHH), with the clone numbers being HHC7, HHC11, HHC22, HHC53 and HHC78, respectively, in which the amino acid sequence of HHC78 was as follows:

QLQLVESGGGLVQPGGSLRLSCAGSGLFFASSDMSWFRQPPGKERELVAEITSVGNNIKY

ADSVEGRFTISRDNAKSTVYLQMNSLKPEDTAVYYCSARRRVRYWGQGTQVTVSS (SEQ

ID NO: 37)

Based on the amino acid sequence of the above HHC78, CDRs and FRs of the variable region of the antibody were determined according to the Kabat numbering rule, and the 3 CDR sequences of the antibody is shown in the following table:

Table 18

| SEQ ID NO | 22 | 23 | 24 |
|---|---|---|---|
| Amino acid sequence | SSDMS | EITSVGNNIKYADSVEG | RRRVRY |

## Example 16. Construction of the llama-derived anti-GPRC5D chimeric antibody and its transient transfection by transfection in eukaryotic cells

The gene fragments of interest, generated by linking the sequenced heavy chain antibody variable region of the present disclosure to the human IgG1 constant region, were cloned into the pTT5 expression vector to prepare a transfection-grade expression plasmid. The heavy chain antibody variable region can be linked to the human IgG1 constant region via a short peptide linker to form: heavy chain antibody variable region - short peptide linker - human IgG1 constant region. The sequence of the short peptide linker used in this example is GGGGS.

The sequence of the introduced human IgG1 constant region (SEQ ID NO: 38) was as follows:

DKTHTCPPCPAPELLGGPSVFLFPPKPKDTLMISRTPEVTCVVVDVSHEDPEVKFNW

YVDGVEVHNAKTKPREEQYNSTYRVVSVLTVLHQDWLNGKEYKCKVSNKALPAPIEKT

ISKAKGQPREPQVYTLPPSRDELTKNQVSLTCLVKGFYPSDIAVEWESNGQPENNYKTTP

PVLDSDGSFFLYSKLTVDKSRWQQGNVFSCSVMHEALHNHYTQKSLSLSPG

Expi293F™ cells (Thermo Fisher Scientific) were cultured in serum-free medium, seeded into shake flasks (Corning Inc.), and incubated on a shaker at 37°C, 8% $CO_2$. The cell density was adjusted, and the recombinant expression vector containing the gene fragment of interest was mixed with the PEI transfection reagent at an appropriate ratio and added to the shake flasks for cell culture. After 6 days of cell culture, the supernatant was collected and centrifugated by high-speed centrifugation to remove the cell debris, followed by affinity purification using a Protein A column. The column was washed with PBS until the A280 read fell to the baseline. The target protein was eluted with an acidic eluent of pH 3.0-pH 3.5 and neutralized with 1 M Tris-HCl, pH 8.0-9.0. After the eluted samples were appropriately concentrated, the buffer was exchanged into PBS and the samples were aliquoted for later use. The final purified chimeric antibody was subjected to SDS-PAGE, HPLC purity analysis and A280 concentration determination.

## Example 17. Binding of the llama-derived anti-GPRC5D chimeric antibody to GPRC5D-expressing cells

The culture medium for both the human GPRC5D-CHOK1 cells and the cynomolgus monkey GPRC5D-CHOK1 cells was F12K + 10% FBS + 400 μg/mL Hygromycin. The cells were cultured using T75 cell culture flasks, which were placed in a 37°C, 5% $CO_2$ incubator. Before use, the cells were washed twice with sterile DPBS, and digested with 0.25% trypsin-EDTA for about 5 minutes, and the digestion was terminated using a complete medium.

The resulting cells were centrifuged at 1000 rpm for 5 minutes at room temperature, the supernatant was discarded, and the cells were resuspended in 100 μL of 1% BSA (in PBS). The cells were counted, and the cell density was adjusted to 1E6/mL. The cells were plated into 96-well round bottom culture plates (corning 3799), centrifuged at 1500 rpm for 5 minutes at 4°C, and the supernatant was discarded. The cells were resuspended in 200 μL of 1% BSA (in PBS), centrifuged again at 1500 rpm for 5 minutes at 4°C. The supernatant was discarded, and the cells were kept at 4°C for later use. The antibody samples to be tested were diluted in 1% BSA (in PBS) with a starting concentration of 100 nM and then serially diluted 10-fold to obtain 7 concentrations. The cells were resuspended in the diluted antibodies at 100 μL/well and incubated at 4°C for 1 hour. The cells were centrifuged at 1500 rpm for 5 minutes at 4°C, and the supernatant was discarded. The cells were resuspended and washed with 160 $\mu$L of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at 1:400 according to the instructions. The cells were resuspended in the diluted secondary antibody at 100 $\mu$L/well and incubated at 4°C for 0.5 hour. The cells were centrifuged at 1500 rpm for 5 minutes at 4°C, and the supernatant was discarded. The cells were resuspended and washed with 200 $\mu$L of 1% BSA (in PBS), centrifuged at 1500 rpm for 5 minutes at 4°C, and the supernatant was discarded. The cells were resuspended in 100 $\mu$L of 1% BSA (in PBS), filtered through 300-mesh gauze, and the mean fluorescence intensity in the PE channels was measured by a flow cytometer.

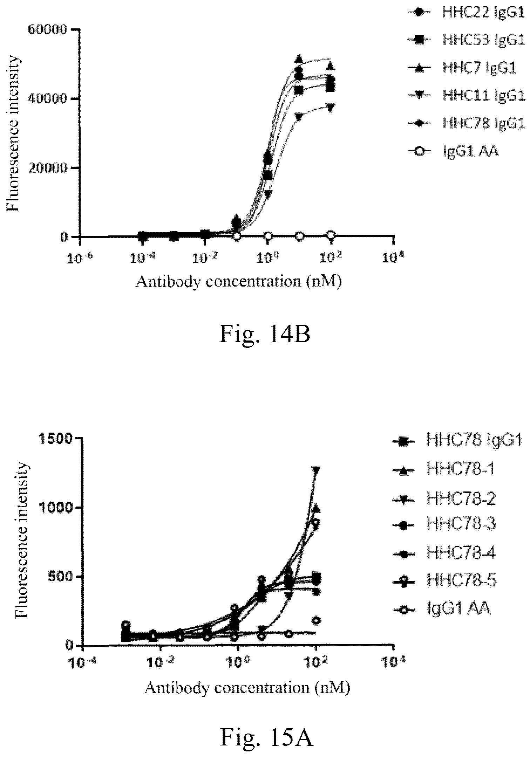

FCS files were exported from the flow cytometer. The mean fluorescence intensity (referred to as MFI below) of the PE channel of each sample was analyzed using flowjo software. The obtained mean fluorescence intensities were then imported into Graphpad for analysis of the half binding concentration (referred to as $EC_{50}$ below) of the antibodies to the cells and the top mean fluorescence intensity (Top MFI). The results are shown in Table 19 and FIG. 14. The screened anti-GPRC5D heavy chain antibody bound well to both the human GPRC5D and the cynomolgus monkey GPRC5D at the cellular level.

Table 19. Binding of the anti-GPRC5D chimeric antibodies to GPRC5D-expressing cells

| Clone numberings | Human GPRC5D-CHOK1 | | Cynomolgus monkey GPRC5D-CHOK1 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | No fitting | 36 | No fitting | 85 |
| HHC78 IgG1 | 0.79 | 47396 | 0.98 | 45829 |

## Example 18. Humanization design of the llama-derived anti-GPRC5D chimeric antibody

Germline gene sequences with high homology to the candidate heavy chain antibody were selected by sequence alignment as the VHH grafting framework templates. After grafting the CDR regions of the candidate antibody onto the selected framework for the human antibody variable region, individual amino acid back-mutations were introduced to obtain humanized antibodies H1, H2, H3, H4, and H5, in which the sequence of H4 is shown in Table 20:

Table 20. Amino acid sequence of the variable region of the humanized llama-derived anti-GPRC5D antibody

| | SEQ ID NO | Heavy chain variable region (VHH) |
|---|---|---|
| H4 | 10 | QVQLVESGGGVVQPGGSLRLSCAASGLFFASSDMSWFRQAPGKGRELV AEITSVGNNIKYADSVEGRFTISRDNSKNTVYLQMNSLRAEDTAVYYCS ARRRVRYWGQGTLVTVSS |

## Example 19. Preparation of the humanized llama-derived anti-GPRC5D antibody

The gene fragments of interest, generated by linking the variable region of the humanized antibody to the human IgG1 constant region, were cloned into the pTT5 expression vector as described in Example 16 to prepare a transfection-grade expression plasmid.

Expi293F™ cells (Thermo Fisher Scientific) were cultured in serum-free medium, seeded into shake flasks (Corning Inc.), and incubated on a shaker at 37°C, 8% $CO_2$. The cell density was adjusted, and the recombinant expression vector containing the gene fragment of interest was mixed with the PEI transfection reagent at an appropriate ratio and added to the shake flasks for cell culture. After 6 days of cell culture, the supernatant was collected and certrifugated by high-speed centrifugation to remove the cell debris, followed by affinity purification using a Protein A column. The column was washed with PBS until the A280 read fell to the baseline. The target protein was eluted with an acidic eluent of pH 3.0-pH 3.5 and neutralized with 1 M Tris-HCl, pH 8.0-9.0. After the eluted samples were appropriately concentrated, the buffer was exchanged into PBS and the samples were aliquoted for later use. The final purified chimeric antibody was subjected to SDS-PAGE, HPLC purity analysis and A280 concentration determination.

## Example 20. Binding of the humanized llama-derived anti-GPRC5D antibody to GPRC5D-expressing cells

The culture medium for NCI-H929 cells (ATCC, CRL-9068) was RPMI 1640 + 10% FBS + 0.05 mM mercaptoethanol, and the culture medium for cynomolgus monkey GPRC5D-CHOK1 cells was F12K + 10% FBS + 400 $\mu$g/mL

Hygromycin. The cells were cultured using T75 cell culture flasks, which were placed in a 37°C, 5% $CO_2$ incubator.

When NCI-H929 cells were needed, the cells were transferred directly to a 50 mL centrifuge tube using a pipette. When cynomolgus monkey GPRC5D-CHOK1 cells were needed, the cells were washed twice with sterile DPBS, digested with 0.25% trypsin-EDTA for about 5 minutes, and the digestion was terminated using a complete medium.

The resulting cells were centrifuged at 1000 rpm for 5 minutes at room temperature, the supernatant was discarded, and the cells were resuspended in 100 µL of 1% BSA (in PBS). The cells were counted, and the cell density was adjusted to 1E6/mL. The cells were plated into 96-well round bottom culture plates (corning 3799), centrifuged at 1500 rpm for 5 minutes at 4°C, and the supernatant was discarded. The cells were resuspended in 200 µL of 1% BSA (in PBS), centrifuged again at 1500 rpm for 5 minutes at 4°C. The supernatant was discarded, and the cells were kept at 4°C for later use. The antibody samples to be tested were diluted in 1% BSA (in PBS) with a starting concentration of 100 nM and then serially diluted 10-fold to obtain 7 concentrations. The cells were resuspended in the diluted antibodies at 100 µL/well and incubated at 4°C for 1 hour. The cells were centrifuged at 1500 rpm for 5 minutes at 4°C, and the supernatant was discarded. The cells were resuspended and washed with 160 µL of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at 1:400 according to the instructions. The cells were resuspended in the diluted secondary antibody at 100 µL/well and incubated at 4°C for 0.5 hour. The cells were centrifuged at 1500 rpm for 5 minutes at 4°C, and the supernatant was discarded. The cells were resuspended and washed with 200 µL of 1% BSA (in PBS), centrifuged at 1500 rpm for 5 minutes at 4°C, and the supernatant was discarded. The cells were resuspended in 100 µL of 1% BSA (in PBS), filtered through 300-mesh gauze, and the mean fluorescence intensity in the PE channels was measured by a flow cytometer.

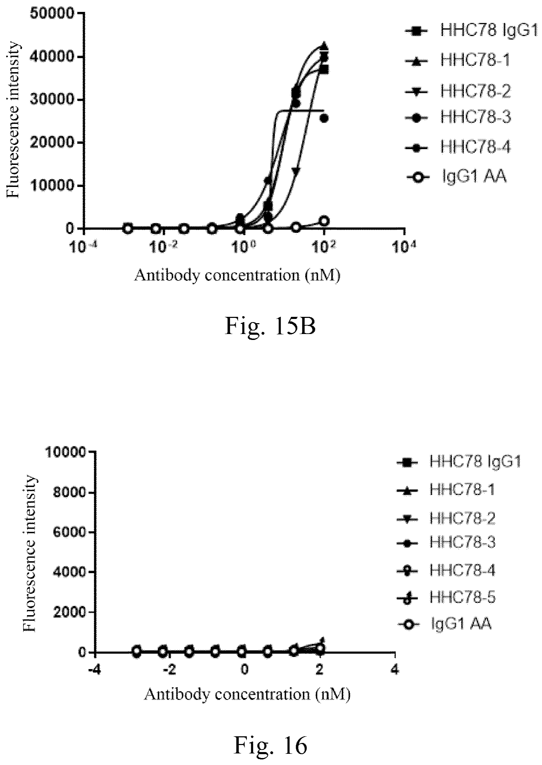

FCS files were exported from the flow cytometer. The mean fluorescence intensity (referred to as MFI below) of the PE channel of each sample was analyzed using flowjo software. The obtained mean fluorescence intensities were then imported into Graphpad for analysis of the half binding concentration (referred to as $EC_{50}$ below) of the antibodies to the cells and the top mean fluorescence intensity (Top MFI). The results are shown in Table 21 and FIG. 15. The binding of the humanized antibody of the clone HHC78 to cynomolgus monkey GPRC5D-CHOK1 cells was comparable to that of the parent antibody, while the binding to NCI-H929 tumor cells was maintained or attenuated compared to the parent antibody.

Table 21. Binding of the humanized anti-GPRC5D antibody to GPRC5D-expressing cells

| | NCI-H929 | | Cynomolgus monkey GPRC5D-CHOK1 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | No fitting | 178 | No fitting | 1840 |
| HHC78 IgG1 | 2.53 | 494 | 9.22 | 37000 |
| HHC78-H4 IgG1 | 1.18 | 386 | 8.68 | 39700 |

## Example 21. Binding of the humanized llama-derived anti-GPRC5D antibody to GPRCSA, a member of the same family

The culture medium for human GPRC5A-CHOK1 was F12K + 10% FBS + 400 µg/mL Hygromycin. The cells were cultured using T75 cell culture flasks, which were placed in a 37°C, 5% $CO_2$ incubator. Before use, the cells were washed twice with sterile DPBS, digested with 0.25% trypsin-EDTA for about 5 minutes, and the digestion was terminated with a complete medium.

The resulting cells were centrifuged at 1000 rpm for 5 minutes at room temperature, the supernatant was discarded, and the cells were resuspended in 100 µL of 1% BSA (in PBS). The cells were counted, and the cell density was adjusted to 1E6/mL. The cells were plated into 96-well round bottom culture plates (corning 3799), centrifuged at 1500 rpm for 5 minutes at 4°C, and the supernatant was discarded. The cells were resuspended in 200 µL of 1% BSA (in PBS), centrifuged again at 1500 rpm for 5 minutes at 4°C. The supernatant was discarded, and the cells were kept at 4°C for later use. The antibody samples to be tested were diluted in 1% BSA (in PBS) with a starting concentration of 100 nM and then serially diluted 10-fold to obtain 7 concentrations. The cells were resuspended in the diluted antibodies at 100 µL/well and incubated at 4°C for 1 hour. The cells were centrifuged at 1500 rpm for 5 minutes at 4°C, and the supernatant was discarded. The cells were resuspended and washed with 160 µL of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at 1:400 according to the instructions. The cells were resuspended in the diluted secondary antibody at 100 µL/well and incubated at 4°C for 0.5 hour. The cells were centrifuged at 1500 rpm for 5 minutes at 4°C, and the supernatant was discarded. The cells were resuspended and washed with 200 µL of 1% BSA (in PBS), centrifuged at 1500 rpm for 5 minutes at 4°C, and the supernatant was discarded. The cells were resuspended in 100 $\mu$L of 1% BSA (in PBS), filtered through 300-mesh gauze, and the mean fluorescence intensity in the PE channels was measured by a flow cytometer.

FCS files were exported from the flow cytometer. The mean fluorescence intensity (referred to as MFI below) of the PE channel of each sample was analyzed using flowjo software. The obtained mean fluorescence intensities were then imported into Graphpad for analysis of the half binding concentration (referred to as $EC_{50}$ below) of the antibodies to the cells and the top mean fluorescence intensity (Top MFI). The result showed that the antibodies tested did not exhibit non-specific binding to the same family member GPRC5A, as shown in FIG. 16.

**Example 22. Preparation of the anti-BCMA antibodies derived from mouse hybridomas**

Anti-BCMA monoclonal antibodies were generated by immunizing mice. Balb/c female mice, 6 weeks old (Charles River Corporation) were used in the experiment. Housing environment: SPF grade. After purchase, the mice were housed in the laboratory for 1 week, with the light/dark cycle of 12/12 hours, a temperature of 20-25°C; and a humidity of 40-60%. 25 $\mu$g of the immunogen, human BCMA ECD protein (SinoBiological, Cat. No.: 10620-H08H), was administered for each immunization on day 0, day 14, day 28, and day 42, and a booster immunization was performed 2 days before spleen cell fusion. Antibody titers in mouse serum were measured by ELISA during the immunization period. After the booster immunization, mice with high and plateaued serum antibody titers were selected for spleen cell fusion. Spleen lymphocytes were fused with Sp2/0 myeloma cells (ATCC® CRL-8287™) using an optimized electrofusion procedure to obtain hybridoma cells.

After 7-14 days of culture of the fused hybridoma cells, the culture medium supernatant was collected, and the hybridoma supernatant was screened for antibodies by FACS using CHO-K1 cells highly expressing human BCMA. The positive antibody strains were further screened using CHO-K1 cells highly expressing cynomolgus monkey BCMA and blank CHO-K1 cells to exclude antibody hybridomas with non-specific binding, thereby selecting hybridomas specifically binding to the human and the cynomolgus monkey BCMA. Hybridoma cells at logarithmic-phase were collected, and RNA was extracted using Trizol (Invitrogen, 15596-018) and reverse transcribed (PrimeScript™ Reverse Transcriptase, Takara #2680A). The cDNA obtained by reverse transcription was amplified by PCR using the mouse Ig-Primer Set (Novagen, TB326 Rev.B 0503) and sequenced to obtain the amino acid sequences of 11 monoclonal antibody variable regions of the present disclosure: 19CH-1, 19CH-2, 19CH-3, 19CH-4, 19CH-5, 19CH-7, 19CH-8, 19CH-9, 19CH-11, 19CH-13, and 19CH-16, in which the 19CH-16 is shown in Table 22.

Table 22. Amino acid sequence of the variable region of the anti-BCMA 19CH-16 monoclonal antibody derived from mouse hybridoma

| Clone numbering | | Heavy chain variable region (VH) | Light chain variable region (VL) |
|---|---|---|---|
| 19CH-16 | SEQ ID NO | 39 | 40 |
| | Amino acid sequence | QGQMQQSGAELVKPGTSVKLSCKT SGFTFRSSYISWLQQKPGQSLEWIA WIYAGSGNTDYNQKFTGKAQLTVD TSSSTAYMQFSSLTTEDSAIYYCAR HYDPPHYFDYWGQGTTLTVSS | QIVLTQSPAIMSASPGEKVTMTC SASSGVSHMHWYQQKSGTSPKR WIYDTSKLTSGVPTRFSGTGSGT SYSLSISSMEAEDAATYYCQQW SRNPWTFGGGTKLEIK |

Based on the above amino acid sequence, the CDRs and FRs of variable regions of the antibody were determined according to the Kabat numbering rule, and the 6 CDR sequences of 19CH-16 is shown in Table 23 below.

Table 23. CDR sequences of the anti-BCMA antibody derived from mouse hybridoma

| Clone numbering | | CDR1 | | CDR2 | | CDR3 | |
|---|---|---|---|---|---|---|---|
| | | SEQ ID NO | Amino acid sequence | SEQ ID NO | Amino acid sequence | SEQ ID NO | Amino acid sequence |
| 19CH-16 | Heavy chain | 16 | SSYIS | 41 | WIYAGSGNTDYNQKFTG | 18 | HYDPPHYFDY |
| | Light chain | 19 | SASSGVSHMH | 20 | DTSKLTS | 21 | QQWSRNPWT |

**Example 23. Construction of the anti-BCMA chimeric antibody derived from mouse hybridoma and its transient expression by transfection in eukaryotic cells**

The heavy chain variable region and light chain variable region of the sequenced monoclonal antibody of the present disclosure were respectively linked to the IgG1 (L234AL235A) heavy chain constant region and the $\kappa$ light chain constant region to generate the gene fragments of interest, which subsequently were cloned into the pTT5 expression vector to prepare a transfection-grade expression plasmid.

Expi293F™ cells (Thermo Fisher, A14527) were cultured in Expi293 Expression Medium (Thermo Fisher, A1435101), seeded into shake flasks, and incubated on a shaker at 37°C, 8% CO2. The cell density was adjusted, and the recombinant expression vector containing the gene fragment of interest was mixed with the PEI transfection reagent at an appropriate ratio and added to the shake flasks for cell culture. After 6 days of cell culture, the supernatant was collected and centrifugated by high-speed centrifugation to remove the cell debris, followed by affinity purification using a Mabselect Sure column. The column was washed with PBS until the A280 read fell to the baseline. The target protein was eluted with an acidic eluent of pH 3.0-pH 3.5 and neutralized with 1 M Tris-HCl, pH 8.0-9.0. After the eluted samples were appropriately concentrated, the buffer was exchanged into PBS and the samples were aliquoted for later use. The final purified chimeric antibody was subjected to SDS-PAGE, HPLC purity analysis and A280 concentration determination.

**Example 24. Binding of the anti-BCMA chimeric antibody derived from mouse hybridoma to BCMA-expressing cells**

The culture medium for both the human BCMA-CHOK1 cells and the cynomolgus monkey BCMA-CHOK1 cells was F12K + 10% FBS + 400 $\mu$g/mL Hygromycin. The cells were cultured using T75 cell culture flasks, which were placed in a 37°C, 5% $CO_2$ incubator. Before use, the cells were washed twice with sterile DPBS, digested with 0.25% trypsin-EDTA for about 5 minutes, and the digestion was terminated with a complete medium.

The resulting cells were centrifuged at 1000 rpm for 5 minutes at room temperature, the supernatant was discarded, and the cells were resuspended in 100 $\mu$L of 1% BSA (in PBS). The cells were counted, and the cell density was adjusted to 1E6/mL. The cells were plated into 96-well round bottom culture plates (corning 3799), centrifuged at 1500 rpm for 5 minutes at 4°C, and the supernatant was discarded. The cells were resuspended in 200 $\mu$L of 1% BSA (in PBS), centrifuged again at 1500 rpm for 5 minutes at 4°C. The supernatant was discarded, and the cells were kept at 4°C for later use. The antibody samples to be tested were diluted in 1% BSA (in PBS) with a starting concentration of 100 nM and then serially diluted 10-fold to 7 concentrations. The cells were resuspended in the diluted antibodies at 100 $\mu$L/well and incubated at 4°C for 1 hour. The cells were centrifuged at 1500 rpm for 5 minutes at 4°C, and the supernatant was discarded. The cells were resuspended and washed with 160 $\mu$L of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at 1:400 according to the instructions. The cells were resuspended in the diluted secondary antibody at 100 $\mu$L/well and incubated at 4°C for 0.5 hour. The cells were centrifuged at 1500 rpm for 5 minutes at 4°C, and the supernatant was discarded. The cells were resuspended and washed with 200 $\mu$L of 1% BSA (in PBS), centrifuged at 1500 rpm for 5 minutes at 4°C, and the supernatant was discarded. The cells were resuspended in 100 $\mu$L of 1% BSA (in PBS), filtered through 300-mesh gauze, and the mean fluorescence intensity in the PE channels was measured by a flow cytometer.

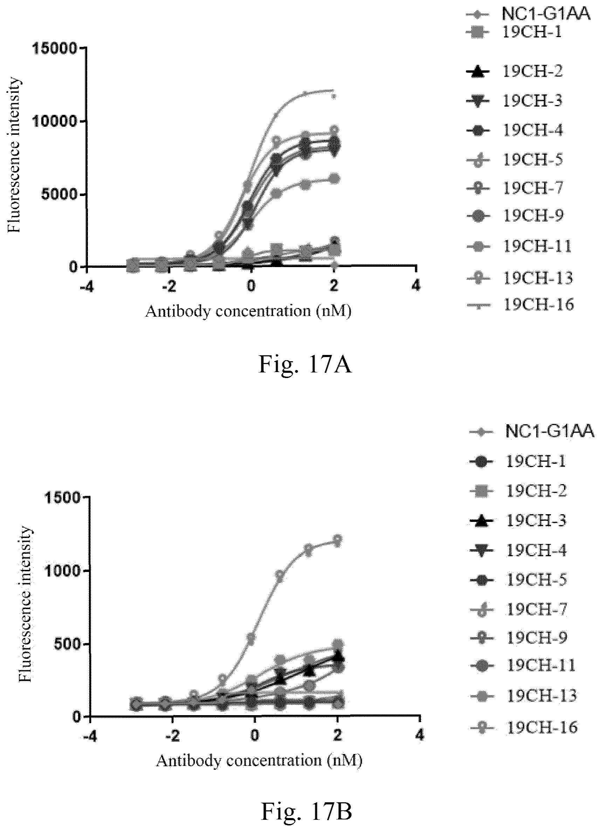

FCS files were exported from the flow cytometer. The mean fluorescence intensity (referred to as MFI below) of the PE channel of each sample was analyzed using flowjo software. The obtained mean fluorescence intensities were then imported into Graphpad for analysis of the half binding concentration (referred to as $EC_{50}$ below) of the antibodies to the cells and the top mean fluorescence intensity (Top MFI). The results are shown in Table 24 and FIG. 17. The screened 1 anti-BCMA chimeric antibody (19CH-16) bound well to both the human BCMA and the cynomolgus monkey BCMA at the cellular level.

Table 24. Binding of the anti-BCMA chimeric antibody derived from mouse hybridoma to BCMA-expressing cells

| Clone numberings | Human BCMA CHOK1 | | Cynomolgus monkey BCMA CHOK1 | |
|---|---|---|---|---|
| | EC$_{50}$ (nM) | Top mean fluorescence intensity (Top MFI) | EC$_{50}$ (nM) | Top mean fluorescence intensity (Top MFI) |
| IgG1 AA | N/A | 89.1 | N/A | 82.6 |
| 19CH-16 | 0.8852 | 11800 | 1.221 | 1194 |

## Example 25. Humanization design of the anti-BCMA antibody derived from mouse hybridoma

Based on the results of the expression purification test and the cell-level binding test, the antibody with clone numbering 19CH-16 was selected for humanization design.

Humanization of the murine anti-human BCMA monoclonal antibody was carried out as disclosed in a number of publications in the art. Briefly, the human constant domain was used to replace the parent (murine antibody) constant domain, and human germline antibody sequences were selected based on the homology between the murine and human antibodies. Based on the typical structure of the obtained murine antibody VH/VL CDRs, a human germline template with high homology was obtained by comparing the variable region sequences of the heavy and light chains with a human antibody germline database.

The CDR regions of the murine antibody were grafted onto the selected corresponding humanized template to replace the humanized variable region, and were recombined with the IgG constant region (preferably the heavy chain was IgG1 and the light chain was κ). Then, based on the three-dimensional structure of the murine antibody, reverse mutations were introduced at the embedded residues, residues directly interacting with the CDR regions, and residues significantly affecting the conformation of VL and VH. Antibodies composed of the following combinations of humanized light and heavy chain variable region sequences were designed: 19CH-16H1L1, 19CH-16H1L2, 19CH-16H1L3, 19CH-16H2L1, 19CH-16H2L2, 19CH-16H2L3, 19CH-16H3L1, 19CH-16H3L2, 19CH-16H3L3, 19CH-16H4L1, 19CH-16H4L2, and 19CH-16H4L3, in which the 19CH-16H2L2 is shown in Table 25.

Table 25. Amino acid sequences of variable regions of one humanized antibody derived from mouse hybridoma

| Clone number | | Heavy chain variable region (VH) | Light chain variable region (VL) |
|---|---|---|---|
| 19CH-16H2L2 | SEQ ID NO | 42 | 15 |
| | Amino acid se-quence | QVQLVQSGAEVKKPGASVKVSC KASGFTFRSSYISWVRQAPGQGL EWIAWIYAGSGNTDYNQKFTGR VTMTVDTSTSTVYMELSSLRSED TAVYYCARHYDPPHYFDYWGQG TLVTVSS | DIQLTQSPSSLSASVGDRVTIT CSASSGVSHMHWYQQKPGKA PKRWIYDTSKLTSGVPSRFSGS GSGTDFTLTISSLQPEDFATYY CQQWSRNPWTFGQGTKVEIK |

## Example 26. Preparation of the humanized anti-BCMA antibody derived from mouse hybridoma

The heavy chain variable region and light chain variable region of the humanized antibody were respectively linked to the IgG1 (L234AL235A) heavy chain constant region and the κ light chain constant region to generate the gene fragments of interest, which subsequently were cloned into the pTT5 expression vector to prepare a transfection-grade expression plasmid.

Expi293F™ cells (Thermo Fisher, A14527) were cultured in Expi293 Expression Medium (Thermo Fisher, A1435101), seeded into shake flasks, and incubated on a shaker at 37°C, 8% CO2. The cell density was adjusted, and the recombinant expression vector containing the gene fragment of interest was mixed with the PEI transfection reagent at an appropriate ratio and added to the cell culture shake flasks. After 6 days of cell culture, the supernatant was collected and centrifugated by high-speed centrifugation to remove the cell debris, followed by affinity purification using a Mabselect Sure column. The column was washed with PBS until the A280 read fell to the baseline. The target protein was eluted with an acidic eluent of pH 3.0-pH 3.5 and neutralized with 1 M Tris-HCl, pH 8.0-9.0. After the eluted samples were appropriately concentrated, the buffer was exchanged into PBS and the samples were aliquoted for later use. The final purified chimeric antibody was subjected to SDS-PAGE, HPLC purity analysis and A280 concentration determination.

## Example 27. Binding of the humanized anti-BCMA antibody derived from mouse hybridoma to BCMA-expressing cells

The culture medium for both the human BCMA-CHOK1 cells and the cynomolgus monkey BCMA-CHOK1 cells was F12K + 10% FBS + 400 μg/mL Hygromycin. Before use, the human BCMA-CHOK1 cells and the cynomolgus monkey BCMA-CHOK1 cells were washed twice with sterile DPBS, digested with 0.25% trypsin-EDTA for about 5 minutes, and the digestion was terminated with a complete medium.

The resulting cells were centrifuged at 1000 rpm for 5 minutes at room temperature, the supernatant was discarded, and the cells were resuspended in 100 μL of 1% BSA (in PBS). The cells were counted, and the cell density was adjusted to 1E6/mL. The cells were plated into 96-well round bottom culture plates (corning 3799), centrifuged at 1500 rpm for 5 minutes at 4°C, and the supernatant was discarded. The cells were resuspended in 200 μL of 1% BSA (in PBS), centrifuged again at 1500 rpm for 5 minutes at 4°C. The supernatant was discarded, and the cells were kept at 4°C for later use. The antibody samples to be tested were diluted in 1% BSA (in PBS) with a starting concentration of 100 nM and then serially diluted 10-fold to 7 concentrations. The cells were resuspended in the diluted antibodies at 100 μL/well and incubated at 4°C for 1 hour. The cells were centrifuged at 1500 rpm for 5 minutes at 4°C, and the supernatant was discarded. The cells were resuspended and washed with 160 μL of 1% BSA (in PBS), centrifuged at 1500 rpm for 5 minutes at 4°C, and the supernatant was discarded. The secondary antibody (goat anti-human IgG Fc PE) was diluted in 1% BSA (in PBS) at 1:400 according to the instructions. The cells were resuspended in the diluted secondary antibody at 100 μL/well and incubated at 4°C for 0.5 hour. The cells were centrifuged at 1500 rpm for 5 minutes at 4°C, and the supernatant was discarded. The cells were resuspended and washed with 200 μL of 1% BSA (in PBS), centrifuged at 1500 rpm for 5 minutes at 4°C, and the supernatant was discarded. The cells were resuspended in 100 μL of 1% BSA (in PBS), filtered through 300-mesh gauze, and the mean fluorescence intensity in the PE channels was measured by a flow cytometer.

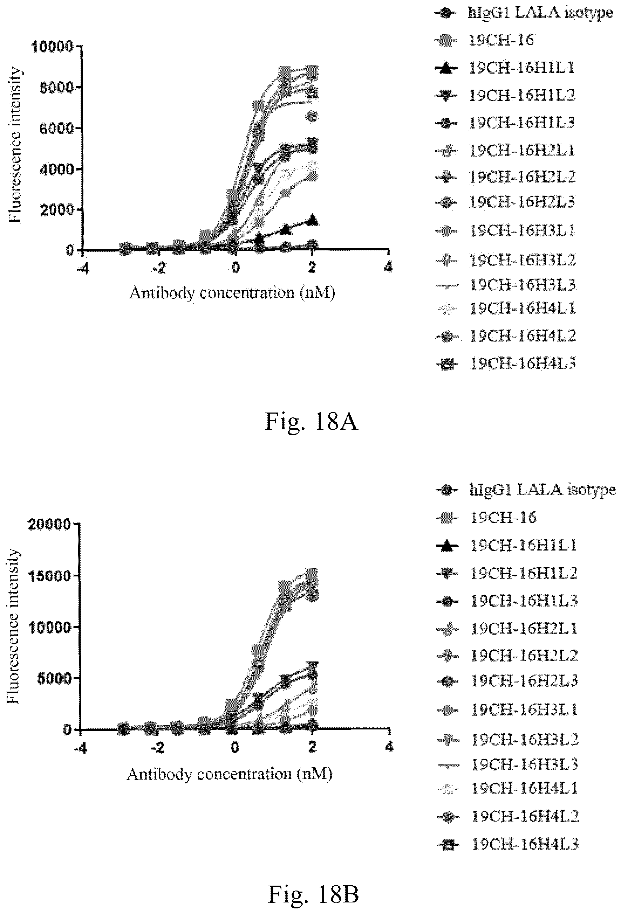

FCS files were exported from the flow cytometer. The mean fluorescence intensity (referred to as MFI below) of the PE channel of each sample was analyzed using flowjo software. The obtained mean fluorescence intensities were then imported into Graphpad for analysis of the half binding concentration (referred to as $EC_{50}$ below) of the antibodies to the cells and the top mean fluorescence intensity (Top MFI). The results are shown in Table 26 and FIG. 18. One humanized anti-BCMA antibody (19CH-16H2L2) screened and prepared in Example 26 bound well to both the human BCMA and the cynomolgus monkey BCMA at the cellular level.

Table 26. Binding of the humanized anti-BCMA antibody derived from mouse hybridoma to BCMA-expressing cells

| Clone numberings | Human BCMA CHOK1 | | Cynomolgus monkey BCMA CHOK1 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | N/A | 232 | N/A | 382 |
| 19CH-16H2L2 | 2.126 | 8632 | 4.821 | 14300 |

## Example 28. Preparation of variants of the humanized anti-BCMA antibodies derived from mouse hybridoma

Post-translational modification (PTM) analysis of the CDR regions of the above humanized anti-BCMA antibody 19CH-16H2L2 revealed that there was a deamidation site in the the heavy chain variable region. To eliminate the potential risk, a site-directed mutation at a single site was performed on the amino acid sequence of the 19CH-16H2, generating two variants, 19CH-16H2L2-NA and 19CH-16H2L2-QT. The amino acid sequences of the variable regions of the 19CH-16H2L2-NA variant are shown in Table 27.

Table 27. Amino acid sequences of the 19CH-16H2L2 humanized antibody variant

| Clone No. | | Heavy chain variable region (VH) | Light chain variable region (VL) |
|---|---|---|---|
| 19CH-16H2L2-NA | SEQ ID NO | 14 | 15 |
| | Amino acid sequence | QVQLVQSGAEVKKPGASVKVS CKASGFTFRSSYISWVRQAPGQ GLEWIAWIYAGSGNADYNQKF TGRVTMTVDTSTSTVYMELSS LRSEDTAVYYCARHYDPPHYF DYWGQGTLVTVSS | DIQLTQSPSSLSASVGDRVTITC SASSGVSHMHWYQQKPGKAP KRWIYDTSKLTSGVPSRFSGSG SGTDFTLTISSLQPEDFATYYCQ QWSRNPWTFGQGTKVEIK |

**EP 4 663 662 A1**

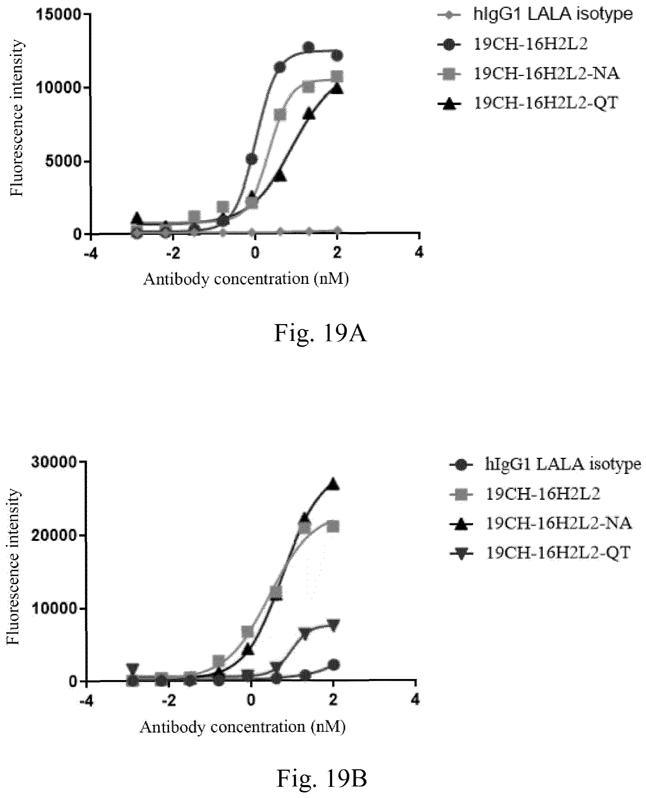

The above two variant proteins were prepared by transient transfection expression in Expi293 cells as described in Example 26. The affinity of the two variants was determined using the human BCMA-CHOK1 cells and the cynomolgus monkey BCMA-CHOK1 cells as described in Example 27. The results are shown in Table 28 and FIG. 19. 19CH-16H2L2-NA is capable of maintaining the binding affinity at a cellular level while eliminating the risk of post-translational modifications.

Table 28. Binding of the 19CH-16H2L2 humanized antibody variants to BCMA-expressing cells

| Clone numberings | Human BCMA CHOK1 | | Cynomolgus monkey BCMA CHOK1 | |
|---|---|---|---|---|
| | $EC_{50}$ (nM) | Top mean fluorescence intensity (Top MFI) | $EC_{50}$ (nM) | Top mean fluorescence intensity (Top MFI) |
| IgG1 AA | N/A | 232 | N/A | 382 |
| 19CH-16H2L2-NA | 2.154 | 10700 | 5.86 | 26900 |
| 19CH-16H2L2-QT | 7.761 | 9914 | 9.055 | 7555 |

The embodiments of the present disclosure described above are merely exemplary, and a person skilled in the art will recognize or be able to determine countless equivalents of specific compounds, materials, and procedures without the need for experimentation beyond routine procedures. All such equivalents are within the scope of this disclosure and are encompassed by the claims.

**Claims**

1.    A trispecific antigen-binding molecule, comprising the following polypeptides:

a first polypeptide comprising: (i) a heavy chain domain of an antigen-binding fragment Fab capable of specifically binding to a first antigen, (ii) a single chain antibody (scFv) domain capable of specifically binding to a second antigen, and (iii) a first Fc domain,
a second polypeptide comprising: a light chain domain of the antigen-binding fragment Fab capable of specifically binding to the first antigen, and,
a third polypeptide comprising: (i) a heavy chain single-domain antibody (VHH) domain capable of specifically binding to a third antigen, and (ii) a second Fc domain,
wherein the heavy chain domain of the antigen-binding fragment Fab of the first polypeptide forms a first binding site for the first antigen together with the light chain domain of the antigen-binding fragment Fab of the second polypeptide; the single chain antibody (scFv) domain forms a second binding site for the second antigen; the heavy chain single-domain antibody (VHH) domain forms a third binding site for the third antigen; and the first Fc domain and the second Fc domain associate with each other;
optionally, the trispecific antigen-binding molecule further comprises a fourth polypeptide, which comprises a light chain domain of the antigen-binding fragment Fab that specifically binds to the first antigen, wherein the light chain domain of the antigen-binding fragment Fab is the same as the light chain domain of the antigen-binding fragment Fab of the second polypeptide, and the third polypeptide further comprises a heavy chain domain of the antigen-binding fragment Fab that specifically binds to the first antigen, with its C-terminus linked to the N-terminus of the VHH domain, wherein the heavy chain domain of the antigen-binding fragment Fab of the third polypeptide is the same as the heavy chain domain of the antigen-binding fragment Fab of the first polypeptide, and the heavy chain domain of the antigen-binding fragment Fab of the third polypeptide forms a fourth binding site for the first antigen together with the light chain domain of the antigen-binding fragment Fab of the fourth polypeptide.

2.    The trispecific antigen-binding molecule of claim 1, wherein the single chain antibody (scFv) domain comprises a heavy chain variable region and a light chain variable region; preferably, the heavy chain variable region of the scFv domain is linked to the light chain variable region of the scFv domain via a first linker, wherein the C-terminus of the heavy chain variable region of the scFv domain is fused to the N-terminus of the first linker, and the C-terminus of the first linker is fused to the N-terminus of the light chain variable region of the scFv domain; more preferably, the first linker comprises the amino acid sequence $(G4S)_n$, wherein n is any integer from 1 to 10.

3.    The trispecific antigen-binding molecule of claim 1 or 2, wherein the first Fc domain comprises a first CH2 domain and a first CH3 domain of an immunoglobulin, with the C-terminus of the first CH2 domain fused to the N-terminus of the

44

first CH3 domain; the second Fc domain comprises a second CH2 domain and a second CH3 domain of an immunoglobulin, with the C-terminus of the second CH2 domain fused to the N-terminus of the second CH3 domain;

preferably, the first CH3 domain comprises a "knob" structure, and the second CH3 domain comprises a "hole" structure; more preferably, the "knob" structure comprises amino acid substitutions S354C and T366W, and the "hole" structure comprises amino acid substitutions Y349C, T366S, L368A, and Y407V;

preferably, the first and/or second Fc domains comprise amino acid substitutions of L234A, L235A, and/or G237A;

preferably, the second Fc domain comprises an amino acid substitution of H435R; preferably, the single chain antibody (scFv) domain is linked to the first Fc domain via a second linker, wherein the C-terminus of the single chain antibody (scFv) domain is fused to the N-terminus of the second linker, and the C-terminus of the second linker is fused to the N-terminus of the first Fc domain;

more preferably, the second linker comprises the amino acid sequence EPKSS;

preferably, the Fc domain is derived from IgG1.

4. The trispecific antigen-binding molecule of any one of claims 1-3, wherein the heavy chain domain of the antigen-binding fragment Fab comprises a heavy chain variable region and a CH1 domain of an immunoglobulin, with the C-terminus of the heavy chain variable region fused to the N-terminus of the CH1 domain; the light chain domain of the antigen-binding fragment Fab comprises a light chain variable region and a light chain constant region of an immunoglobulin, with the C-terminus of the light chain variable region fused to the N-terminus of the light chain constant region;

preferably, the heavy chain domain of the antigen-binding fragment Fab of the first polypeptide is linked to the scFv domain via a third linker, wherein the C-terminus of the heavy chain domain of the antigen-binding fragment Fab is fused to the N-terminus of the third linker, and the C-terminus of the third linker is fused to the N-terminus of the scFv domain;

optionally, the heavy chain domain of the antigen-binding fragment Fab of the third polypeptide is linked to the VHH domain via a fifth linker, wherein the C-terminus of the heavy chain domain of the antigen-binding fragment Fab is fused to the N-terminus of the fifth linker, and the C-terminus of the fifth linker is fused to the N-terminus of the VHH domain;

preferably, the third linker and/or the fifth linker comprises the amino acid sequence $(G_4S)_n$, wherein n is any integer from 1 to 10.

5. The trispecific antigen-binding molecule of any one of claims 1-4, wherein the C-terminus of the heavy chain single-domain antibody (VHH) domain is fused to the N-terminus of the second Fc domain, preferably, the C-terminus of the VHH domain is fused to the N-terminus of the second Fc domain via a fourth linker; more preferably, the fourth linker comprises the amino acid sequence EPKSS.

6. The trispecific antigen-binding molecule of any one of claims 1-5, wherein the first polypeptide comprises the following structure: the heavy chain domain of the Fab -the scFv domain - the first Fc domain, preferably, the first polypeptide comprises the following structure: the heavy chain variable region of the Fab -the Fab CH1 -the third linker - the heavy chain variable region of scFv - the first linker - the light chain variable region of scFv - the second linker - the first CH2 - the first CH3;

wherein the second polypeptide comprises the following structure: the light chain variable region of the Fab -the light chain constant region of the Fab;

and/or wherein the third polypeptide comprises the following structure: the VHH domain- the second Fc domain, preferably, the third polypeptide comprises the following structure: the VHH domain - the fourth linker - the second CH2 - the second CH3;

optionally, the fourth polypeptide comprises the following structure: the light chain variable region of the Fab -the light chain constant region of the Fab, and the third polypeptide comprises the following structure: the heavy chain domain of the Fab - the fifth linker - the VHH- the fourth linker- the second Fc domain, further preferably, the third polypeptide comprises the following structure: the heavy chain variable region of the Fab the Fab CH1 - the fifth linker - the VHH-the fourth linker - the second CH2 - the second CH3.

7. The trispecific antigen-binding molecule of any one of claims 1-6, wherein the second antigen is CD3, preferably CD3ε; preferably, the scFv domain comprises HCDR1 the sequence of which is as set forth in SEQ ID NO: 27, HCDR2 the sequence of which is as set forth in SEQ ID NO: 28, HCDR3 the sequence of which is as set forth in SEQ ID NO: 29, LCDR1 the sequence of which is as set forth in SEQ ID NO: 30, LCDR2 the sequence of which is as set forth in SEQ ID NO: 31, and LCDR3 the sequence of which is as set forth in SEQ ID NO: 32;

more preferably, the single chain antibody (scFv) domain comprises a heavy chain variable region the sequence of which is as set forth in SEQ ID NO: 25 and a light chain variable region the sequence of which is as set forth in SEQ ID NO: 26;
more preferably, the single chain antibody (scFv) domain comprises the amino acid sequence as set forth in SEQ ID NO: 13.

8. The trispecific antigen-binding molecule of any one of claims 1-7, wherein the first Fc domain comprises the amino acid sequence as set forth in SEQ ID NO: 33, and the second Fc domain comprises the amino acid sequence as set forth in SEQ ID NO: 34.

9. The trispecific antigen-binding molecule of any one of claims 1-8, wherein the first antigen is BCMA; preferably, the antigen-binding fragment Fab comprises HCDR1 the sequence of which is as set forth in SEQ ID NO: 16, HCDR2 the sequence of which is as set forth in SEQ ID NO: 17, and HCDR3 the sequence of which is as set forth in SEQ ID NO: 18, preferably, the antigen-binding fragment Fab comprises LCDR1 the sequence of which is as set forth in SEQ ID NO: 19, LCDR2 the sequence of which is as set forth in SEQ ID NO: 20, and LCDR3 the sequence of which is as set forth in SEQ ID NO: 21;

more preferably, the heavy chain domain of the antigen-binding fragment Fab comprises a heavy chain variable region the sequence of which is as set forth in SEQ ID NO: 14, the light chain domain of the antigen-binding fragment Fab comprises a light chain variable region the sequence of which is as set forth in SEQ ID NO: 15; more preferably, the heavy chain domain of the antigen-binding fragment Fab comprises the amino acid sequence as set forth in SEQ ID NO: 35; more preferably, the light chain domain of the antigen-binding fragment Fab comprises the amino acid sequence as set forth in SEQ ID NO: 7.

10. The trispecific antigen-binding molecule of any one of claims 1-9, wherein the third antigen is GPRC5D; preferably, the heavy chain single-domain antibody (VHH) domain, which is capable of specifically binding to the third antigen comprises HCDR1 the sequence of which is as set forth in SEQ ID NO: 22, HCDR2 the sequence of which is as set forth in SEQ ID NO: 23, and HCDR3 the sequence of which is as set forth in SEQ ID NO: 24; more preferably, the VHH domain comprises the sequence as set forth in SEQ ID NO: 10.

11. The trispecific antigen-binding molecule of any one of claims 1-10, wherein the first polypeptide of the trispecific antigen-binding molecule comprises the amino acid sequence as set forth in SEQ ID NO: 5, the second polypeptide comprises the amino acid sequence as set forth in SEQ ID NO: 7, and the third polypeptide comprises the amino acid sequence as set forth in SEQ ID NO: 6; or optionally, the first polypeptide of the trispecific antigen-binding molecule comprises the sequence as set forth in SEQ ID NO: 5, the second polypeptide and/or the fourth polypeptide comprises the sequence as set forth in SEQ ID NO: 7, and the third polypeptide comprises the sequence as set forth in SEQ ID NO: 8.

12. A trispecific antigen-binding molecule, being capable of binding to the epitopes which are the same as or overlap with the epitopes targeted by an antibody comprising the HCDR1 the sequence of which is as set forth in SEQ ID NO: 22, the HCDR2 the sequence of which is as set forth in SEQ ID NO: 23, and the HCDR3 the sequence of which is as set forth in SEQ ID NO: 24.

13. The trispecific antigen-binding molecule of claim 12, the epitopes to which the molecule is capable of binding are the same as or overlap with the epitopes targeted by an antibody comprising the amino acid sequence as set forth in SEQ ID NO: 10.

14. The trispecific antigen-binding molecule of claim 12 or 13, which is capable of binding to CD3 and BCMA, wherein the trispecific antigen-binding molecule is preferably capable of binding to CD3ε and BCMA.

15. A trispecific antigen-binding molecule, comprising:

(A) a first binding portion capable of specifically binding to GPRC5D3;
(B) a second binding portion; and
(C) a third binding portion, the first, second, and third binding portions specifically bind to antigens or epitopes different from each other;

the first binding portion comprises:
the HCDR1 the sequence of which is as set forth in SEQ ID NO: 22, the HCDR2 the sequence of which is as set forth in SEQ ID NO: 23, and the HCDR3 the sequence of which is as set forth in SEQ ID NO: 24.

16. The trispecific antigen-binding molecule of claim 15, wherein the first binding portion comprises:
the amino acid sequence as set forth in SEQ ID NO: 10.

17. The trispecific antigen-binding molecule of claim 15 or 16, wherein the second binding portion is capable of binding to CD3, preferably to CD3ε, and/or the third binding portion is capable of binding to BCMA.

18. A nucleic acid molecule, encoding the trispecific antigen-binding molecule of any one of the preceding claims.

19. An expression vector, comprising the nucleic acid molecule of claim 18.

20. A host cell, comprising the nucleic acid molecule of claim 18 or the expression vector of claim 19; preferably, the host cell is a prokaryotic or an eukaryotic cell; the prokaryotic cell is preferably *Escherichia coli*; the eukaryotic cell is preferably a mammalian cell or yeast; more preferably, the mammal cell is a CHO cell, an Expi293, or a HEK293 cell.

21. A method for preparing the trispecific antigen-binding molecule of any one of claims 1-17, comprising culturing the host cell of claim 20 under suitable conditions.

22. A pharmaceutical composition, comprising the trispecific antigen-binding molecule of any one of claims 1-17, the nucleic acid molecule of claim 18, the expression vector of claim 19, and/or the host cell of claim 20.

23. The pharmaceutical composition of claim 22, further comprising a pharmaceutically acceptable carrier.

24. The pharmaceutical composition of claim 22 or 23, further comprising one or more additional therapeutic agents.

25. Use of the trispecific antigen-binding molecule of any one of claims 1-17, the nucleic acid molecule of claim 18, the expression vector of claim 19, and/or the host cell of claim 20 in the preparation of a medicament for the treatment, alleviation, and/or prevention of a tumor.

26. The use of claim 25, wherein the tumor is a GPRC5D and/or BCMA-positive tumor.

27. The use of claim 25 or 26, wherein the tumor is selected from the group consisting of lymphomas, such as multiple myeloma, and metastatic cancers of the above tumors.

28. A method for inducing the death of a cell expressing GPRC5D and/or BCMA, comprising contacting the cell with the trispecific antigen-binding molecule of any one of claims 1-17, the nucleic acid molecule of claim 18, the expression vector of claim 19, the host cell of claim 20, and/or the pharmaceutical composition of any one of claims 22-24, preferably, the cell expressing GPRC5D and/or BCMA is a tumor cell.

29. The method of claim 28, wherein the tumor cell is selected from the group consisting of tumor cells of the following tumors: lymphomas, such as multiple myeloma, and metastatic cancers of the above tumors.

30. A method for treating a disease associated with the expression of GPRC5D and/or BCMA in a subject, comprising administering to the subject in need thereof the trispecific antigen-binding molecule of any one of claims 1-17, the nucleic acid molecule of claim 18, the expression vector of claim 19, the host cell of claim 20, and/or the pharmaceutical composition of any one of claims 22-24.

31. The method of claim 30, wherein the disease is a tumor;
preferably, lymphomas, such as multiple myeloma, and metastatic cancers of the above tumors.

32. The method of claim 30 or 31, further comprising administering an additional therapeutic agent to the subject.

33. An antigen-binding molecule or antigen-binding fragment thereof that specifically binds to BCMA, wherein the antigen-binding molecule or antigen-binding fragment thereof comprises HCDR sequences of the heavy chain variable region the sequence of which is as set forth in SEQ ID NO: 14, and/or LCDR sequences of the light chain variable region the sequence of which is as set forth in SEQ ID NO: 15; preferably, the antigen-binding molecule or antigen-binding fragment thereof that specifically binds to BCMA comprises a heavy chain variable region (VH) and a light chain variable region (VL), the heavy chain variable region comprises HCDR1 the sequence of which is as set forth in SEQ ID NO: 16, HCDR2 the sequence of which is as set forth in SEQ ID NO: 17, and HCDR3 the sequence of which is as set forth in SEQ ID NO: 18, and/or, the light chain variable region comprises an LCDR1 the sequence of which is as set forth in SEQ ID NO: 19, LCDR2 the sequence of which is as set forth in SEQ ID NO: 20, and LCDR3 the sequence of which is as set forth in SEQ ID NO: 21.

34. The antigen-binding molecule or antigen-binding fragment thereof that specifically binds to BCMA of claim 33, the antigen-binding molecule or antigen-binding fragment thereof specifically binds to BCMA comprises a heavy chain variable region as set forth in SEQ ID NO: 14, and/or a light chain variable region as set forth in SEQ ID NO: 15.

35. The antigen-binding molecule or antigen-binding fragment thereof that specifically binds to BCMA of claim 33 or 34, further wherein:

i) the antigen-binding molecule or antigen-binding fragment thereof that specifically binds to BCMA further comprises a heavy chain constant region and/or a light chain constant region; preferably, the heavy chain constant region comprises Fc; more preferably, Fc is derived from murine or human; even more preferably, the sequence of the Fc is either natural or modified; further preferably, the Fc domain of the IgG1 comprises the amino acid sequence as set forth in SEQ ID NO: 38;
ii) The antigen-binding molecule or antigen-binding fragment thereof that specifically binds to BCMA is a monoclonal antibody, a bispecific binding molecule, a multispecific binding molecule, a murine antibody, a humanized antibody, a chimeric antibody, a modified antibody, a fully human antibody, a full-length antibody, a heavy chain antibody, a nanobody, a Fab, an Fv, a scFv, a F(ab')2, a linear antibody, and/or a heavy chain single-domain antibody; and/or
iii) the antigen-binding molecule or antigen-binding fragment thereof that specifically binds to BCMA is of the IgG1, IgG2, IgG3, or IgG4 isotype.

36. The antigen-binding molecule or antigen-binding fragment thereof that specifically binds to BCMA of any one of claims 33-35, comprising the heavy chain amino acid sequence as set forth in SEQ ID NO: 9, and/or the light chain amino acid sequence as set forth in SEQ ID NO: 7.

37. A conjugate or a fusion protein, wherein the conjugate or the fusion protein is formed by coupling the antigen-binding molecule or antigen-binding fragment thereof that specifically binds to BCMA of any one of claims 33-36 to a capture label or a detection label; preferably, the detection label comprises a radionuclide, a luminescent substance, a colored substance, or an enzyme, or one of the fused portions of the fusion protein comprises the antigen-binding molecule or antigen-binding fragment thereof that specifically binds to BCMA of any one of claims 33-36.

38. An antibody-drug conjugate, wherein the antibody-drug conjugate is formed by coupling the antigen-binding molecule or antigen-binding fragment thereof that specifically binds to BCMA of any one of claims 33-36 to additionalbiologically active molecules; preferably, the additional biologically active molecule is a small molecule drug; preferably, the antigen-binding molecule or antigen-binding fragment thereof that specifically binds to BCMA is linked to the additional biologically active molecules via a linker.

39. A nucleic acid encoding the antigen-binding molecule or antigen-binding fragment thereof that specifically binds to BCMA of any one of claims 33-36, or a recombinant vector comprising the nucleic acid, or a host cell comprising the nucleic acid or the recombinant vector; preferably, the host cell is a prokaryotic cell (preferably *E. coli*), or an eukaryotic cell (preferably a mammalian cell or yeast; more preferably, the mammalian cell is a CHO cell or a HEK293 cell).

40. A method for preparing the antigen-binding molecule or antigen-binding fragment thereof that specifically binds to BCMA of any one of claims 33-36, comprising culturing the host cell of claim 53 under suitable conditions and purifying the expression product obtained from the cell.

41. Use of the antigen-binding molecule or antigen-binding fragment thereof that specifically binds to BCMA of any one of claims 33-36, the conjugate or the fusion protein of claim 37, the antibody-drug conjugate of claim 38, or the nucleic acid, the recombinant vector, or the host cell of claim 39 in the preparation of a medicament for the treatment or amelioration of tumors;

preferably, the medicament targets tumor cells with abnormal expression of BCMA; preferably, the tumor cell is selected from the group consisting of tumor cells of the following tumors: lymphomas, such as multiple myeloma, and metastatic cancers of the above tumors.

42. Use of the antigen-binding molecule or antigen-binding fragment thereof that specifically binds to BCMA of any one of claims 33-36, the conjugate or the fusion protein of claim 37, the antibody-drug conjugate of claim 38, or the nucleic acid, the recombinant vector, or the host cell of claim 39 in the preparation of a detection reagent or diagnostic reagent; preferably, the detection reagent is used for detecting the expression of BCMA; the diagnostic reagent is used for diagnosing tumors; preferably, the tumor cell is selected from the group consisting of tumor cells of the following tumors: lymphomas, such as multiple myeloma, and metastatic cancers of the above tumors.

43. A method for detecting the expression of BCMA in a sample, comprising:

(1) contacting the sample with the antigen-binding molecule or antigen-binding fragment thereof that specifically binds to BCMA of any one of claims 33-36;
(2) detecting the formation of a complex between the antigen-binding molecule or antigen-binding fragment thereof that specifically binds to BCMA and BCMA; optionally, the antigen-binding molecule or antigen-binding fragment thereof that specifically binds to BCMA is detectably labeled.

44. A pharmaceutical composition, comprising an effective amount of the antigen-binding molecule or antigen-binding fragment thereof that specifically binds to BCMA of any one of claims 33-36, the conjugate or the fusion protein of claim 37, the antibody-drug conjugate of claim 38, or the nucleic acid, the recombinant vector, or the host cell of claim 39; preferably, the pharmaceutical composition further comprises a pharmaceutically acceptable carrier; preferably, the pharmaceutical composition further comprises one or more additional therapeutic agents.

45. A chimeric antigen receptor (CAR) or a cell comprising the chimeric antigen receptor, comprising the antigen-binding molecule or antigen-binding fragment thereof that specifically binds to BCMA of any one of claims 33-36.

46. A method for inducing the death of a cell expressing BCMA, comprising contacting the cell with the pharmaceutical composition of claim 44, wherein the cell expressing BCMA is a tumor cell; preferably, the tumor cell is selected from the group consisting of tumor cells of the following tumors: lymphomas, such as multiple myeloma, and metastatic cancers of the above tumors.

47. A method for treating a disease associated with expression of BCMA in a subject, comprising administering to the subject in need thereof the pharmaceutical composition of claim 44;

preferably, the disease is a tumor; preferably, the tumor is selected from the group consisting of lymphomas such as multiple myeloma, and metastatic cancers of the above tumors; more preferably, the method further comprises administering an additional therapeutic agent to the subject.

Fig. 1A

Fig. 1B

Fig. 1C

Fig. 2A

Fig. 2B

Trispecific antibody 1                    Trispecific antibody 2

Anti-BCMA antibody
heavy chain variable region
Anti-BCMA antibody
light chain variable region

Anti-CD3 antibody
heavy chain variable region

Anti-CD3 antibody
light chain variable region

Anti-GPRC5D nanobody

Anti-BCMA full-length
antibody Fab fragment

Anti-CD3 scFv single
chain antibody

Fc domain with "knob"
and "hole" structure

Fig. 3

Fig. 4A

Fig. 4B

Fig. 4C

Fig. 4D

Fig. 4E

Fig. 4F

Fig. 4G

Fig. 4H

Fig. 5A

Fig. 5B

Fig. 5C

Fig. 6A

Fig. 6B

Fig. 7

Fig. 8A

Fig. 8B

Fig. 8C

Fig. 8D

Fig. 8E

Fig. 9

Fig. 10A

Fig. 10B

Fig. 11

Fig. 12

Fig. 13

Fig. 14A

Fig. 14B

Fig. 15A

Fig. 15B

Fig. 16

Fig. 17A

Fig. 17B

Fig. 18A

Fig. 18B

Fig. 19A

Fig. 19B

## INTERNATIONAL SEARCH REPORT

| International application No. |
| --- |
| **PCT/CN2024/076521** |

**A. CLASSIFICATION OF SUBJECT MATTER**

C07K16/30(2006.01)i; C07K16/28(2006.01)i; A61K39/395(2006.01)i; A61P35/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC:C07K,A61P,A61K

Documentation searched other than minimum documentation to the extent that such documents are included in the fields searched

Electronic data base consulted during the international search (name of data base and, where practicable, search terms used)

CJFD, CNTXT, ENTXTC, DWPI, VEN, CNKI, BING, NCBI, EBI, STN, 百度学术, Baidu Scholar: 申请人/发明人, applicant/ inventor, 多特异性, 三特异性, trispecific, 抗体, 抗原结合分子, antibod+, 先声药业, BCMA, B cell maturation antigen, CD3, GPRC5D, G protein coupled receptor C5 family subtype D, MBS314, SEQ ID NOs. 7-32, 多发性骨髓瘤, multiple myeloma

**C. DOCUMENTS CONSIDERED TO BE RELEVANT**

| Category* | Citation of document, with indication, where appropriate, of the relevant passages | Relevant to claim No. |
| --- | --- | --- |
| X | WO 2022174813 A1 (INNOVENT BIOLOGICS (SUZHOU) CO., LTD.) 25 August 2022 (2022-08-25) claims 1-2 and 20-25, and description, page 20, paragraph 4 | 1-6, 12-14, 18-32 |
| Y | WO 2022174813 A1 (INNOVENT BIOLOGICS (SUZHOU) CO., LTD.) 25 August 2022 (2022-08-25) claims 1-2 and 20-25, and description, page 20, paragraph 4 | 7, 18-32 |
| Y | US 2022411533 A1 (BOEHRINGER LNGELHEIM INTERNATIONAL GMBH) 29 December 2022 (2022-12-29) claim 1, and table 1 | 7, 18-32 |
| X | WO 2022175255 A2 (JANSSEN PHARMACEUTICA NV) 25 August 2022 (2022-08-25) claim 1, and description, paragraph 24 | 1-6, 12-14, 18-32 |
| Y | WO 2022175255 A2 (JANSSEN PHARMACEUTICA NV) 25 August 2022 (2022-08-25) claims 1-110, and figure 1A | 7, 14, 18-32 |

☑ Further documents are listed in the continuation of Box C. ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 April 2024** | **06 May 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/076521** |

### C.    DOCUMENTS CONSIDERED TO BE RELEVANT

| Category* | Citation of document, with indication, where appropriate, of the relevant passages | Relevant to claim No. |
| --- | --- | --- |
| A | 张瑞等 (ZHANG, Rui et al.). "一种三特异性抗体设计以及功能研究 (Structure Design and Function Research of the Trispecific Antibody)" <br> *药物生物技术 (Chinese Journal of Pharmaceutical Biotechnology)*, <br> Vol. 29, No. (3), 31 December 2022 (2022-12-31), 227-233 <br>     abstract, and figure 1 | 1-47 |
| A | 吴明 (WU, Ming). "靶向CD3-TROP2-PDL1的三特异性抗体对三阴性乳腺癌治疗功能的基础研究 (Non-official translation: Basic Study on the Therapeutic Function of Trispecific Antibodies Targeting CD3-TROP2-PDL1 in Triple-negative Breast Cancer)" <br> *中国优秀硕士学位论文全文数据库, 医药卫生科技辑 (China Master's Theses Full-text Database, Medicine and Health Sciences)*, <br> No. 2022, no. 2, 16 January 2022 (2022-01-16), E072-938 <br>     abstract, and figure 3.1 | 1-11, 18-32 |

Form PCT/ISA/210 (second sheet) (July 2022)

**INTERNATIONAL SEARCH REPORT**

| International application No. |
|---|
| **PCT/CN2024/076521** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/076521**

**Box No. II          Observations where certain claims were found unsearchable (Continuation of item 2 of first sheet)**

This international search report has not been established in respect of certain claims under Article 17(2)(a) for the following reasons:

1. ☑ Claims Nos.: **28-32, 46-47**
   because they relate to subject matter not required to be searched by this Authority, namely:

   Claims 28-32 and 46-47 relate to a method for treating diseases, which falls within the cases set out in PCT Rule 39.1(iv) for which a search is not required by the International Searching Authority. A search is conducted on the basis of amending the subject matter thereof to be the subject matter of a corresponding pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2024/076521** |

| Patent document cited in search report | | | Publication date (day/month/year) | Patent family member(s) | | | Publication date (day/month/year) |
| --- | --- | --- | --- | --- | --- | --- | --- |
| WO | 2022174813 | A1 | 25 August 2022 | JP | 2024510098 | A | 06 March 2024 |
| | | | | CA | 3211407 | A1 | 25 August 2022 |
| | | | | TW | 202237661 | A | 01 October 2022 |
| | | | | TWI | 830151 | B | 21 January 2024 |
| | | | | AU | 2022223152 | A1 | 31 August 2023 |
| | | | | EP | 4296286 | A1 | 27 December 2023 |
| US | 2022411533 | A1 | 29 December 2022 | AR | 126161 | A1 | 27 September 2023 |
| | | | | TW | 202317625 | A | 01 May 2023 |
| | | | | WO | 2022263507 | A1 | 22 December 2022 |
| WO | 2022175255 | A2 | 25 August 2022 | IL | 305144 | A | 01 October 2023 |
| | | | | AR | 124882 | A1 | 17 May 2023 |
| | | | | JP | 2024507180 | A | 16 February 2024 |
| | | | | TW | 202302636 | A | 16 January 2023 |
| | | | | CR | 20230398 | A | 15 November 2023 |
| | | | | AU | 2022221884 | A1 | 05 October 2023 |
| | | | | CO | 2023011907 | A2 | 18 September 2023 |
| | | | | KR | 20230146578 | A | 19 October 2023 |
| | | | | CA | 3211163 | A1 | 25 August 2022 |
| | | | | EP | 4294528 | A2 | 27 December 2023 |
| | | | | US | 2022267438 | A1 | 25 August 2022 |
| | | | | UY | 39634 | A | 31 August 2022 |
| | | | | WO | 2022175255 | A3 | 29 September 2022 |
| | | | | DOP | 2023000158 | A | 29 February 2024 |
| | | | | BR | 112023016121 | A2 | 28 November 2023 |
| | | | | ECSP | 23069904 | A | 31 October 2023 |

Form PCT/ISA/210 (patent family annex) (July 2022)

**REFERENCES CITED IN THE DESCRIPTION**

**Patent documents cited in the description**

- CN 2023101003684
- WO 2018017786 A2
- WO 2019220368 A1
- WO 2022175255 A2
- WO 2022174813 A1

**Non-patent literature cited in the description**

- GenBank, BC069341
- NCBI, NP_061124.1
- UniProtKB/Swiss-Prot, Q9NZD1
- **BRAUNER-OSBORNE, H et al.** *Biochim.Biophys.Acta*, 2001, vol. 1518, 237-248
- UniProt, Q02223